(19) Europäisches Patentamt
European Patent Office
Office européen des brevets

(11) EP 4 397 369 A2

(12) EUROPEAN PATENT APPLICATION

(43) Date of publication:
**10.07.2024 Bulletin 2024/28**

(21) Application number: **24160966.8**

(22) Date of filing: **26.06.2019**

(51) International Patent Classification (IPC):
***A61P 21/00*** *(2006.01)*

(52) Cooperative Patent Classification (CPC):
**A61K 31/10; A61K 45/06; A61P 21/00;** A61K 9/00

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2018 US 201862689996 P**

(62) Document number(s) of the earlier application(s) in accordance with Art. 76 EPC:
**19824575.5 / 3 813 806**

(71) Applicant: **Ribonova Inc.**
**Wynnewood, Pennsylvania 19096 (US)**

(72) Inventors:
- **WEBB, Nigel L.**
  **Wynnewood, Pennsylvania 19096 (US)**
- **YUEN, Eric C.**
  **Wynnewood, Pennsylvania 19096 (US)**
- **FORD-HUTCHINSON, Anthony W.**
  **Wynnewood, Pennsylvania 19096 (US)**
- **FALK, Marni J.**
  **Philadelphia, Pennsylvania 19104 (US)**
- **ZOLKIPLI-CUNNINGHAM, Zarazuela**
  **Philadelphia, Pennsylvania 19104 (US)**
- **MCCORMACK, Shana E.**
  **Philadelphia, Pennsylvania 19104 (US)**

(74) Representative: **D Young & Co LLP**
**3 Noble Street**
**London EC2V 7BQ (GB)**

Remarks:
•This application was filed on 01.03.2024 as a divisional application to the application mentioned under INID code 62.
•Claims filed after the date of filing of the application (Rule 68(4) EPC)/after the date of receipt of the divisional application (Rule 68(4) EPC).

# (54) METHODS OF TREATING MITOCHONDRIAL DYSFUNCTION

(57) This disclosure is directed to methods of treating or preventing mitochondrial dysfunction or mitochondrial disease in a subject comprising administration of probucol, or a pharmaceutically acceptable salt thereof. This disclosure is also directed to methods of diagnosing genetic mitochondrial disease in a subject prior to and in association with said treatment or prevention. This disclosure is also directed to methods of assessing and managing a subject with mitochondrial dysfunction or mitochondrial disease using a composite measurement.



Processed by Luminess, 75001 PARIS (FR)

## Description

TECHNICAL FIELD

Disclosed herein are methods of treating or preventing mitochondrial dysfunction or mitochondrial disease in a subject comprising administration of probucol, or a pharmaceutically acceptable salt thereof. Also disclosed herein are methods of diagnosing genetic mitochondrial disease in a subject prior to and in association with said treatment or prevention. Also disclosed herein are methods of managing patients with mitochondrial dysfunction or mitochondrial disease and assessing their response to therapeutic intervention through the use of a composite measurement that combines the results from two or more sub-instruments. Also disclosed herein are methods of computing said composite measurement. Further disclosed herein are integrated methods for diagnosing and treating human subjects with mitochondrial dysfunction or mitochondrial disease and assessing their response using composite measurements.

BACKGROUND

Primary mitochondrial disease comprises a heterogeneous group of genetic conditions that impair the ability to generate cellular energy. Kremer et al., Mitochondrial Disease Genetics in Diagnosis and Management of Mitochondrial Disorders; Mancuso and Klopstock (Eds); Springer 2019; pp 41-62. The disease affects at least 1 in 4,300 individuals with a highly variable, but often progressive array of multi-systemic manifestations. Gorman et al., Ann. Neurol. 2015; 77:753-9. The more severe manifestations can lead to significant morbidity and mortality. Two studies of affected children found mortality rates of 35% and 36%, respectively, during their childhood years. Scaglia et al., Pediatrics. 2004; 114:925-31. Verity et al., Dev. Med. Child Neurol. 2010; 52:434-40. In a longitudinal study of all non-military hospitalizations in California from 2007 to 2011, 9.9% of all participants (9% of adults, 10% of children) with at least one hospitalization for mitochondrial disease died in-hospital during five years of follow-up. McCormack et al., Mol. Genet. Metab. 2017; 121(2):119-126. There are no proven treatment options for the underlying disease process. Pfeffer et al., Nat. Rev. Neurol. 2013; 9:474-81.

Pre-clinical studies have shown that probucol may have potential as a therapeutic agent to investigate in individuals with primary mitochondrial disease, but the compound has not previously been employed for the treatment of mitochondrial disease. Zhang et al., Mol. Genet. Metab. 2010; 99(3): 309-18. Falk et al., EMBO Mol. Med. 2011; 3(7):410-27. Peng et al., Hum. Mol. Genet. 2015; 24(17):4829-47. Byrnes et al., Neurochem. Int. 2018; 117:23-34. Probucol has known safety and efficacy profiles for the treatment of hyperlipidemia, but its safety and efficacy have not been established in patients with mitochondrial disease. Yamashita et al., Curr. Opin. Lipidol. 2015; 26(4):304-16. There is currently a significant unmet medical need to treat patients with mitochondrial disease. Gorman et al., Nat. Rev. Dis. Prim. 2016; 2:16080. The disclosed methods are directed to this and other important needs.

Most of the reported clinical studies of probucol in humans employ the oral administration of tablet formulations for lipid management and related cardiovascular applications. Yamashita et al., Curr. Opin. Lipidol. 2015; 26(4):304-16. The use in humans of a liquid formulation of probucol for lipid reduction has been reported. International Application No. WO9210996. Probucol has also been employed to coat stents to prevent restenosis. Kim et al. Catheterization and Cardiovascular Interventions 2002; 57(4):424-8.

Mitochondrial disease typically impacts multiple organs. For example, gastrointestinal (GI) involvement and nutritional deficit are common. GI involvement has been reported in 29% (Debray et al., Pediatrics. 2007; 119(4):722-33) to 48% (Skladal et al., Clin. Pediatr. 2003; 42(8):703-10) of pediatric patients with mitochondrial disease. Major GI symptoms can include persistent vomiting, failure to thrive, and dysphagia. Swallowing difficulties frequently inhibit sufficient oral food intake and may result in aspiration or chest infection. Kisler et al., Dev. Med. Child. Neurol. 2010; 52(5):422-33. 48% of adult patients with mitochondrial disease experience problems with swallowing. Read et al., Int. J. Lan. Commun. Disord. 2012; 47:106-11.

Enteral tube feeding is indicated in some patients with such neurologic symptoms. Braegger et al., J Pediatr. Gastroenterol. Nutr. 2010 Jul; 51(1):110-22. Nasogastric tube feeding is feasible for up to several months, and gastrostomy is the appropriate option if feeding problems persist. Research shows that gastrostomy benefits pediatric patients with neurodevelopmental diseases in terms of clinical progress and quality of life. Kisler et al., Dev. Med. Child. Neurol. 2010; 52(5):422-33. In one study of pediatric mitochondrial disease patients, the majority of patients had GI symptoms requiring enteral tube feeding, with oropharyngeal dysphagia being the most common indication, apparent in 81% of patients. Choi and Lee, Scientific Reports 2017; 7: 16909.

The serious clinical consequences of mitochondrial disease frequently inhibit or prevent the administration of oral tablets. As a result, the disclosed liquid formulation of probucol will have significant medical utility and will increase the number of patients with mitochondrial disease who can be treated with probucol, by administration through nasogastric, gastrostomy or jejunostomy tubes. Furthermore, the liquid formulation of probucol enables the more accurate and convenient dosing of probucol to pediatric patients including neonates, who present a wide range of body weights that

present challenges for both dose calculation and methods of administration.

The eye is one of the most frequently affected organs in mitochondrial disease, with devastating effects that may variably involve the extraocular eye muscles, levator muscle, lens, retina, or optic nerve. Haas et al., Pediatrics. 2007; 120(6):1326-33. Many primary mitochondrial diseases have ophthalmologic involvement. The four most common neuro-ophthalmic abnormalities seen in mitochondrial disorders are (i) bilateral optic neuropathy, as in Leber's Hereditary Optic Neuropathy (LHON); Fraser et al., Surv. Ophthalmol. 2010; 55(4):299-334; (ii) ophthalmoplegia and ptosis, as in Chronic Progressive External Ophthalmoplegia (CPEO), including Kearns-Sayre Syndrome (KSS); Gronlund et al., Br. J. Ophthalmol. 2010; 94(1):121-127; (iii) pigmentary retinopathy, as in Mitochondrial Encephalopathy, Lactic Acidosis and Stroke like episodes (MELAS), Myoclonic Epilepsy with Ragged Red Fibers (MERRF), Neurogenic weakness, Ataxia, and Retinitis Pigmentosa (NARP), Leigh Syndrome, CPEO and KSS; Gronlund et al., Br. J. Ophthalmol. 2010; 94(1):121-127; and (iv) retro-chiasmal visual loss, as in MELAS. CPEO, a complex disorder that impairs extraocular muscle mobility in association with ptosis, is the most common ocular manifestation of mitochondrial myopathies; Schoser and Pongratz, Strabismus. 2006; 14(2):107-13. LHON is characterized by acute and painless central vision loss of both eyes in a sequential fashion over a period of days to months.

Certain ophthalmologic diseases that have not traditionally been considered to have obvious mitochondrial origins are increasingly recognized to result in part from impaired mitochondrial function, increased oxidative stress, and increased apoptosis. Schrier and Falk, Curr. Opin. Ophthalmol. 2011; 22(5): 325-331. As a high energy demand organ, the eye is particularly susceptible to the consequences of mitochondrial damage. Jarrett et al., Ophthalmic Res. 2010; 44(3):179-90. Ophthalmic disorders that involve mitochondrial dysfunction include diabetic retinopathy and Age-related Macular Degeneration (AMD). Mohammad et al., Lab. Invest. 2010;90(9):1365-1372). Kenney et al., Invest. Ophthalmol. Vis. Sci. 2010; 51(8):4289-4297.

Local topical administration of probucol for ophthalmic conditions provides certain advantages over systemic administration. Eye drops can result in greater delivered local dosages directly to the eye, and can alleviate the challenges of systemic administration with regard to the transport of probucol across the blood-brain barrier. Probucol is relatively water-insoluble and is known to be absorbed preferentially by high fat tissue, a property that can enhance uptake by clinically relevant components of the eye.

Probucol is relatively insoluble in water. Hendler et al. described water-soluble phosphate esters of probucol, in order to generate different types of formulations. International Application No. WO9924400. The claims address using this water-soluble formulation to treat oxidative damage. Oxidative stress is caused by many physiologic conditions, including certain mitochondrial diseases, but this work does not describe the use of water-soluble phosphate esters of probucol to treat any underlying mitochondrial disease.

Yoshitaka proposed a novel antidiabetic treatment and prophylactic medicine which contains probucol or a salt or solvate thereof. Japanese Patent Application No. JP2000319441A. This invention addresses the use of probucol to prevent diabetes by protecting pancreatic beta-cells from oxidative stress. While some patients with mitochondrial diseases experience diabetes, this condition is only one of numerous manifestations of mitochondrial dysfunction. In this work, probucol was proposed to target pancreatic beta-cells alone, and thus is not proposed as a multi-organ or broad spectrum therapeutic agent. Probucol is presented as a preventative agent against diabetes and not as a treatment for underlying mitochondrial disease or other symptoms thereof.

Methods and combinations of compounds were described for modulating the release of cytochrome c from mitochondria to inhibit cell death, as a means to prevent and treat disorders for which the patient is otherwise free of indications for treatment (i.e. excluding patients with *inter alia* symptomatic mitochondrial disease). International Application No. WO2007114948. One of the numerous compounds claimed to inhibit cytochrome c release in the treatment of cell death-associated disorders was probucol. Some cell-death-associated disorders cause mitochondrial dysfunction, but, of the 40 classifications of mitochondrial disease (www.umdf.org/types), only four types are classified as having mitochondrial cytochrome c oxidase deficiency: benign infantile mitochondrial type, French-Canadian type, infantile mitochondrial myopathy type, and Leigh syndrome. Therefore, neither cell-death nor cytochrome c oxidase deficiency defines underlying mitochondrial disease.

A macromolecular structure was described that comprised two branched peptide chains capable of forming water-soluble micelles to deliver therapeutic agents to mitochondria. International Application No. WO2019055988.

Compositions and methods have been described for increasing the cellular respiration of melanized catecholamine neurons, and methods were described for alleviating symptoms or stopping appearance and/or progression of symptoms of Parkinson's disease and related conditions, characterized by nigrostriatal degeneration. U.S. Patent Application No. 20020198231.

(1-phenyl-2-heteroaryl) ethyl-guanidine compounds and salts thereof were described as inhibitors of mitochondrial F1F0 ATP hydrolase activity. U.S. Patent 6,916,813.

A method had been described that uses polyamines to treat degenerative diseases caused by acquired mitochondrial damage and inherited mitochondrial defects. U.S. Patent Application No. 20030013772.

Probucol been described as one of many potential antioxidants to be used in conjunction with mitochondrial

activators, including pyrroloquinoline quinone and coenzyme Q, for the prophylaxis or treatment of disease caused by mitochondrial dysfunction. U.S. Patent Application No. 20070259908.

SUMMARY

The invention relates to a method of treating a human subject with a disease caused by mitochondrial dysfunction comprising administering an amount of probucol effective to maintain or improve mitochondrial function. In some embodiments, the diagnosis of said mitochondrial dysfunction is based in whole or part on the presence of at least one mutation or variant in the subject's DNA. The invention also relates to a method of treating a human subject with a disease caused by mitochondrial dysfunction comprising: (a) obtaining a biological sample from the human subject, (b) detecting the presence in DNA from said sample of at least one mutation or variant associated with mitochondrial dysfunction, and (c) administering to the human subject with said DNA mutation or variant associated with mitochondrial dysfunction probucol or a pharmaceutically acceptable salt thereof in an amount effective to maintain or improve mitochondrial function.

In some embodiments the human subject has a mitochondrial disease selected from the group consisting of: autosomal dominant optic atrophy (ADOA); beta-oxidation defects; carnitine deficiency; carnitine-acyl-carnitine deficiency; chronic progressive external ophthalmoplegia syndrome (CPEO); co-enzyme Q10 deficiency; complex I deficiency (NADH dehydrogenase deficiency); complex II deficiency (succinate dehydrogenase deficiency); complex III deficiency (ubiquinone-cytochrome c oxidoreductase deficiency); complex IV deficiency (cytochrome c oxidase deficiency or COX deficiency'); complex V deficiency (ATP synthase deficiency); multiple respiratory chain complex deficiency; carnitine palmitoyltransferase (CPT) I deficiency; CPT II deficiency; diabetes mellitus and deafness (DAD); creatine deficiency syndrome; Friedreich's ataxia (FA); Kearns-Sayre syndrome (KSS); lactic acidosis; Leber's hereditary optic neuropathy (LHON); Leigh syndrome (Leigh disease); lethal infantile cardiomyopathy (LIC or Barth Syndrome); leukodystrophy; leukoencephalopathy with brain stem and spinal cord involvement and lactate elevation (LBSL); long-chain acyl-CoA dehydrogenase deficiency (LCAD); long-chain 3-hydroxyacyl-CoA dehydrogenase (LCHAD); Luft disease; medium-chain acyl-CoA dehydrogenase deficiency (MCAD); mitochondrial cytopathy; mitochondrial DNA depletion; mitochondrial DNA deletion(s); mitochondrial encephalopathy; mitochondrial myopathy (MM); mitochondrial myopathy, encephalomyopathy, lactic acidosis, stroke-like symptoms (MELAS); mitochondrial enoyl CoA reductase protein associated neurodegeneration (MEPAN); mitochondrial neurogastrointestinal encephalomyopathy (MNGIE); mitochondrial recessive ataxia syndrome (MIRAS); multiple acyl-CoA dehydrogenase deficiency (MAD or glutaric aciduria type II); myoclonic epilepsy with ragged red fibers (MERRF); neuropathy, ataxia, and retinitis pigmentosa (NARP); Pearson syndrome; POLG mutations; progressive infantile poliodystrophy (Alper's disease); ptosis; pyruvate carboxylase deficiency (PCD); pyruvate dehydrogenase complex deficiency (PDCD or PDH); short-chain acyl-CoA dehydrogenase deficiency (SCAD); short chain 3-hydroxyacyl-CoA dehydrogenase deficiency (SCHAD); and very long-chain acyl-CoA dehydrogenase deficiency (VLCAD).

In some embodiments, the at least one mutation or variant is in a mitochondrial DNA encoded gene selected from the group consisting of MT-ATP6, MT-ATP8, MT-CO1, MT-CO2, MT-CO3, MT-CYB, MT-ND1, MT-ND2, MT-ND3, MT-ND4, MT-ND4L, MT-ND5, MT-ND6, MT-RNR1, MT-RNR2, MT-TA, MT-TC, MT-TE, MT-TF, MT-TH, MT-TI, MT-TK, MT-TL1, MT-TL2, MT-TM, MT-TN, MT-TQ, MT-TS1, MT-TS2, MT-TV and MT-TW.

In some embodiments, the at least one mutation or variant is in a nuclear DNA encoded gene selected from the group consisting of AARS2, ABCC8, ACAD9, ACADM, ACADS, ACADVL, ACAT1, ACO2, ADCK3, ADRB2, ADRB3, AFG3L2, AGK, AGRP, AIFM1, AK2, AKT2, ALDH2, AMT, APOPT1, ATP5A1, ATP5E, ATP5F1A, ATP5F1D, ATP5F1E, ATPAF2, AUH, BCS1L, BOLA3, C10orf2, C12orf65, C19orf12, C1QBP, CAPN10, CARS2, CARTPT, CDH23, CDKAL1, CHCHD10, CHKB, CISD2, CLRN1, COA5, COA7, COQ2, COQ4, COQ6, COQ7, COQ8A, COQ9, COX10, COX14, COX15, COX20, COX6B1, COX8A, CPS1, CPT1A, CPT2, CYC1, DARS2, DFNB31, DGUOK, DLAT, DNA2, DNAJC19, DNM1L, EARS2, ECHS1, ELAC2, ENPP1, ETFA, ETFB, ETFDH, ETHE1, FARS2, FASTKD2, FBXL4, FOXRED1, FXN, GATM, GCGR, GCK, GCSH, GFER, GFM1, GHRL, GJB2, GJB3, GJB6, GLDC, GPD2, GPR98, GTPBP3, HADH, HADHA, HADHB, HARS2, HMGA1, HMGCS2, HNF1A, HNF1B, HNF4A, HSD17B10, HSPD1, IBA57, IDH2, IGF2BP2, IL6, INSR, IRS1, IRS2, ISCA1, ISCA2, ISCU, KANK1, KCNJ11, LARS2, LEPR, LIAS, LIPC, LRPPRC, LYRM4, LYRM7, MAPKSIP1, MARS2, MC4R, MFF, MFN2, MGME1, MIPEP, MPC1, MPV17, MRAP2, MRPL3, MRPL44, MRPS16, MRPS2, MRPS22, MRPS34, MRPS7, MSTO1, MTFMT, MTO1, MTPAP, MYO7A, NARS2, NDUFA1, NDUFA10, NDUFA11, NDUFA12, NDUFA13, NDUFA2, NDUFA6, NDUFA9, NDUFAF1, NDUFAF2, NDUFAF3, NDUFAF4, NDUFAF5, NDUFAF6, NDUFB11, NDUFB3, NDUFB8, NDUFB9, NDUFS1, NDUFS2, NDUFS3, NDUFS4, NDUFS6, NDUFS7, NDUFS8, NDUFV1, NDUFV2, NEUROD1, NF2, NFU1, NR0B2, NUBPL, NUP62, OPA1, OPA3, PAX4, PC, PCDH15, PCK2, PDHA1, PDHB, PDP1, PDSS1, PDSS2, PDX1, PET100, PMPCB, PNPLA8, PNPT1, POLG, POLG2, POMC, PPARG, PPARGC1B, PPP1R3A, PUS1, PYY, RARS2, RMND1, RNASEH1, RRM2B, SCO1, SCO2, SDC3, SDHA, SDHAF1, SDHD, SERAC1, SFXN4, SIM1, SLC22A5, SLC25A1, SLC25A12, SLC25A13, SLC25A15, SLC25A19, SLC25A20, SLC25A22, SLC25A26, SLC25A3, SLC25A4, SLC2A2, SLC2A4, SLC30A8, SPG7, SUCLA2, SUCLG1,

SURF1, TACO1, TARS2, TAZ, TDF7L2, TFAM, TIMM8A, TIMMDC1, TK2, TMEM126B, TMEM70, TOP3A, TRIT1, TRMT10C, TRMT5, TRMU, TRNT1, TSFM, TTC19, TUFM, TWNK, TXN2, TYMP, UCP1, UCP3, UQCC2, UQCC3, UQCRB, UQCRC2, UQCRQ, USH1C, USH1G, USH2A, VARS2, WARS2, WFS1 and YARS2.

In some embodiments, the at least one mutation or variant is in a nuclear DNA encoded gene selected from the group consisting of ABCB7, ACADSB, AKAP10, ALAS2, ALDH4A1, ALDH6A1, AMACR, APTX, ARMS2, BAX, BCAT2, BCKDHA, BCKDHB, BCL2, C12orf62, C20orf7, CSorf38, COX4I2, CRAT, CYB5R3, CYCS, CYP11A1, CYP11B1, CYP11B2, CYP24A1, CYP27A1, CYP27B1, D2HGDH, DBT, DECR1, DHODH, DIABLO, DLD, DMGDH, FH, GDAP1, GK, GLRX5, GLUD1, HCCS, HIBCH, HK1, HLCS, HMGCL, HOGA1, HTRA2, IDH3B, IVD, KARS, KIF1B, L2HGDH, LRRK2, MAOA, MCCC1, MCCC2, MCEE, ME2, MLYCD, MMAA, MMAB, MMADHC, MUT, NAGS, NGLY1, OAT, OGDH, OTC, OXCT1, PANK2, PARK2, PARK7, PCCA, PCCB, PDHX, PINK1, PNKD, PPOX, PYCR1, REEP1, RMRP, SACS, SARDH, SARS2, SDHAF2, SDHB, SDHC, SOD2, SPG20, STAR, TMEM126, UCP2, USMG5, WWOX and XPNPEP3.

In some embodiments, the disease is Myoclonus Epilepsy with Ragged-Red Fibers (MERRF) and the DNA mutation or variant is in one or more genes selected from the group consisting of MT-ND5, MT-TF, MT-TH, MT-TS1, MT-TS2, MT-TL1 and MT-TK.

In some embodiments, the disease is mitochondrial encephalopathy, lactic acidosis, and stroke-like episodes syndrome (MELAS) and the DNA mutation or variant is in one or more genes selected from the group consisting of MT-ND1, MT-ND4, MT-ND5, MT-ND6, MT-TC, MT-TF, MT-TH, MT-TQ, MT-TS1, MT-TS2, MT-TL1 and MT-TK. In some embodiments, the disease is non-syndromic sensorineural hearing loss and the DNA mutation or variant is in one or more genes selected from the group consisting of MT-RNR1 and MT-RNR2.

In some embodiments, the disease is Leigh syndrome and the DNA mutation or variant is in one or more genes selected from the group consisting of MT-ATP6, MT-TK, MT-TV, MT-TW, ATP5A1, ATP5E, BCS1L, COX10, COX15, CYC1,FOXRED1, LRPPRC, LYRM7, NDUFA10, NDUFA12, NDUFA2, NDUFA9, NDUFAF2, NDUFAF6, NDUFS3, NDUFS4, NDUFS7, NDUFS8, POLG, SCO1, SCO2, SDHA, SDHC, SDHC, SDHAF1, SDHAF2, SURF1, TMEM70, ATPAF2, UQCRB, UQCRQ, UQCRC2, UQCC2, UQCC3 and USMG5.

In some embodiments, the disease is leukoencephalopathy and the DNA mutation or variant is in one or more genes selected from the group consisting of DARS2, SDHA, SDHB, SDHC, SDHD, SDHAF1 and SDHAF2.

In some embodiments, the disease is Alpers-Huttenlocher syndrome and the DNA mutation or variant is in the POLG gene.

In some embodiments, the disease is mitochondrial neurogastrointestinal encephalopathy (MNGIE) syndrome and the DNA mutation or variant is in one or more genes selected from the group consisting of TK2, DGUOK, SUCLG1, SUCLA2, ABAT, TYMP, RRM2B, MT-TK, POLG, SLC25A4, AGK and MPV17.

In some embodiments, the disease is liver failure and the DNA mutation or variant is in the TFAM gene.

In some embodiments, the disease is a neurologic disease or liver disease and the DNA mutation or variant is in one or more genes selected from the group consisting of DARS2, RARS2, EARS2, MARS2, FARS2, YARS2, SARS2, AARS2 and HARS2.

In some embodiments, the disease is primary mitochondrial disease and the DNA mutation or variant is in one or more genes selected from the group consisting of MRPS and MRPL.

In some embodiments, the disease is a respiratory chain deficiency, neurologic disease, vision loss or liver disease and the DNA mutation or variant is in one or more genes selected from the group consisting of TACO1, GFM1 and C12ORF65.

In some embodiments, the disease is peripheral neuropathy or optic neuropathy and the DNA mutation or variant is in one or more genes selected from the group consisting of OPA1, MFN1, MFN2, DNM1L and MIEF2.

In some embodiments, the disease is a mitochondrial complex deficiency and the DNA mutation or variant is in one or more genes selected from the group consisting of MT-ATP6, MT-CO1, MT-CO2, MT-CO3, MT-ND1, MT-ND2, MT-ND3, MT-ND4, MT-TL1, MT-TN, MT-TK, MT-TV, MT-TW, APOPT1, ATPAF2, ATP5A1, ATP5E, ATP5F1A, ATP5F1D, ATP5F1E, BCS1L, COA5, COA7, COX6B1, COX8A,COX10, COX14, COX15, COX20,CYC1, FASTKD2, FOXRED1, MTFMT, NDUFA1, NDUFA2, NDUFA6, NDUFA9, NDUFA10, NDUFA11, NDUFA12, NDUFA13, NDUFAF1, NDUFAF2, NDUFAF3, NDUFAF4, NDUFAF5, NDUFAF6, NDUFB3, NDUFB8, NDUFB9, NDUFB11, NDUFS1, NDUFS2, NDUFS3, NDUFS4, NDUFS6, NDUFS7, NDUFS8, NDUFV1, NDUFV2, NUBPL, PET100, SCO1, SDHA, SDHAF1, SDHD, SURF1, TACO1, TIMMDC1, TMEM70, TMEM126B, LYRM4, TTC19, UQCC2, UQCC3, UQCRB, UQCRC2 and UQCRQ.

In some embodiments, the disease is a co-enzyme Q10 deficiency and the DNA mutation or variant is in one or more genes selected from the group consisting of ADCK3, COQ2, COQ4, COQ6, COQ7, COQ8A, COQ9, PDSS1 and PDSS2.

In some embodiments, the disease is chronic progressive external ophthalmoplegia (CPEO) and the DNA mutation or variant is in one or more genes selected from the group consisting of MT-TA, MT-TL2, MT-TM, MT-TW, C10orf2, DGUOK, DNA2, POLG, RNASEH1, RRM2B, SLC25A4, TK2, TOP3A and TWNK.

In some embodiments, the disease is a carnitine deficiency and the DNA mutation or variant is in one or more genes selected from the group consisting of CPT1A, CPT2, SLC22A5 and SLC22A20.

In some embodiments, the disease is Friedreich's ataxia and the DNA mutation or variant is in the FXN gene.

In some embodiments, the disease is Kearns-Sayre syndrome and the DNA mutation or variant is in the MT-TL1 gene.

In some embodiments, the disease is lethal infantile cardiomyopathy (LIC) or Barth syndrome and the DNA mutation or variant is in the TAZ gene.

In some embodiments, the disease is Leber's hereditary optic neuropathy (LHON) and the mutation or variant is in one or more genes selected from the group consisting of MT-ND1, MT-ND2, MT-ND3, MT-ND4, MT-ND5, MT-ND6, MT-ND4L, MT-CYB, MT-CO1, MT-CO2, MT-CO3, MT-ATP6, and MT-ATP8.

In some embodiments, the mitochondrial dysfunction is assessed, monitored, or diagnosed by a method comprising: (i) employing a panel of two or more sub-instruments to measure one or more clinical symptoms of mitochondrial dysfunction or mitochondrial disease in a subject, (ii) combining the measurements obtained from said two or more sub-instruments into a single composite measurement, and (iii) assessing the overall severity of, or change in, the mitochondrial dysfunction or mitochondrial disease in the subject by comparing the composite measurement to a reference value or another composite measurement in the same subject.

In some embodiments, the (i) one or more composite measurements are employed to measure the clinical effect on the subject of a diagnostic, therapeutic or other type of medical intervention; (ii) for each of the sub-instruments in said panel, the subject is classified as: (a) a sub-instrument responder or sub-instrument non-responder, (b) a member of a clinical category, or (c) a member of a metric range, based on the change in said one or more clinical symptoms as measured using said sub-instrument; and (iii) the measurements obtained from the sub-instruments in the panel are combined into a single composite measurement, by either: (a) separately assessing the change in each measurement obtained from the panel sub-instruments prior to combining each measurement into a single composite measurement, or (b) combining measurements obtained from the panel sub-instruments at a first time point and generating a single composite measurement for said first time point and then comparing the single composite measurement for said first time point to a single composite measurement generated from the same panel sub-instruments for a second time point.

In some embodiments, the panel sub-instruments comprise one or more of Motor Function Measure, Six Minute Walk Test, Two Minute Walk Test, Modified Fatigue Impact Scale, Friedreich's Ataxia Rating Scale, Patient-Reported Outcomes Measurement Information System Gastrointestinal Symptom Scales, 30-Second Chair Stand Test, Muscle Strength by Myometry Test, Newcastle Mitochondrial Disease Adult Scale, Newcastle Mitochondrial Disease Pediatric Scale, 36-Item Short Form Survey, Clinical Global Impression, Patient's Global Impression, Patient-Reported Initial MitoPC Symptoms, Patient-Reported MitoPC Symptom Changes, Columbia Suicide Severity Rating Scale, Neuropathy Impairment Score, Migraine Disability Assessment Test, Quick Inventory of Depressive Symptomatology- Self-Report, Montreal Cognitive Assessment, NY Heart Association Functional Classification, Diabetes Health Profile, Ocular Motility Assessments, Marginal Reflex Distance, Ocular Motility & Fixation in 8 Gaze Directions, Degree of Ocular Saccades, Visual Function Test, Logarithm of Minimum Angle of Resolution, Pelli-Robson Score, Humphrey Visual Field mean deviation, or a combination thereof.

In some embodiments, the panel sub-instruments comprise Motor Function Measure, Six Minute Walk Test, Modified Fatigue Impact Scale, Friedreich's Ataxia Rating Scale, and the Patient-Reported Outcomes Measurement Information System Gastrointestinal Symptom Scales. In some embodiments, the panel sub-instruments comprises five sub-instruments including, but not limited to, Motor Function Measure, Six Minute Walk Test, Modified Fatigue Impact Scale, Friedreich's Ataxia Rating Scale, and the Patient-Reported Outcomes Measurement Information System Gastrointestinal Symptom Scales.

In some embodiments, the one or more clinical symptoms are selected from the group consisting of fatigue, muscle weakness, neuromuscular dysfunction, exercise intolerance, imbalance, dysautonomia, gastrointestinal dysfunction, vision loss, eye muscle dysfunction, retinal dysfunction, optic nerve dysfunction, ptosis, headache, dehydration, peripheral neuropathy, numbness, epilepsy, seizures, insomnia, mood disorder, depression, diabetes mellitus, obesity, kidney dysfunction, hyperlipidemia, liver disease, sleep apnea, autism spectrum behavior, delayed developmental milestones, arrhythmia, heart muscle dysfunction, cardiac disease, stroke, speech disorder, tinnitus, hearing impairment, learning disability, cognitive impairment, dementia, or a combination thereof.

In some embodiments, the probucol, or a pharmaceutically acceptable salt thereof, is administered at a dose of about 1 mg, about 10 mg, about 100 mg, about 250 mg, about 500 mg, about 1 g, about 2 g, about 5 g, or about 10 g. In some embodiments, the probucol, or a pharmaceutically acceptable salt thereof, is administered at a dose of about 1 mg/day, about 10 mg/day, about 100 mg/day, about 250 mg/day, about 500 mg/day, about 1 g/day, about 2 g/day, about 5 g/day, or about 10 g/day.

In some embodiments, the probucol is administered as a liquid formulation. In some embodiments, the liquid formulation is administered by a route selected from the group consisting of oral, enteral, gastrostomy tube, jejunostomy tube, orogastric tube, nasogastric tube, parenteral, intravenous, subcutaneous, intramuscular, intraperitoneal, intracisternal, intraarticular, intracerebral, intraparenchymal, intracerebro-ventricular, nasal, vaginal, sublingual, intraocular, intravitreal, rectal, topical, transdermal and inhalation. In some embodiments, the probucol, or a pharmaceutically acceptable salt thereof, is administered in the form of a tablet.

In some embodiments, the probucol, or a pharmaceutically acceptable salt thereof, is administered on a continuous dosing schedule. In some embodiments, the probucol, or a pharmaceutically acceptable salt thereof, is administered twice per day, once per day, once every two days, once every three days, once every four days, once every five days, once every six days, once a week, once every two weeks or once per month. In some embodiments, the probucol, or a pharmaceutically acceptable salt thereof, is administered initially in a loading dose that is higher than a subsequent dose.

In some embodiments, probucol is administered with one or more additional pharmaceutical agents. In some embodiments, the one or more additional pharmaceutical agents are selected from niacin, cycloheximide, bezafibrate, vatiquinone, carnitine, coenzyme Q10, alpha-tocopherolquinone, coenzyme Q10 analogs, cytochrome C, dichloroacetate, 5-[(e)-2-(4-hydroxyphenyl)-ethenyl] benzene-1,3 diol, flavin mononucleotide, elamipretide, idebenone, latrepirdine, levocarnitine, 2',3',5'-tri-O-acetyluridine, olesoxime, omaveloxolone, thiamin diphosphate, ubiquinone, vitamin C, vitamin D, vitamin E, thiamine, riboflavin, magnesium, calcium, phosphate, membrane phospholipid, unsaturated fatty acid, creatine, pyruvate, $\alpha$-lipoic acid, NADH, PPAR delta modulator, PPAR delta agonist, cysteamine bitartrate, nicotinic acid, nicotinamide, nicotinamide riboside, acipimox, resveratrol, glucose, L-carnitine, glutathione, redox-modulating agent, dichloroacetate, curcumin, schisandrin, triheptanoin, viral or non-viral vector gene therapy targeting mitochondrial disease mutations, or a combination thereof.

## BRIEF DESCRIPTION OF THE DRAWINGS

The summary, as well as the following detailed description, is further understood when read in conjunction with the appended drawings. For the purpose of illustrating the disclosed methods, there are shown, in the drawings, exemplary embodiments of the methods; however, the methods are not limited to the specific embodiments disclosed. In the drawings:

**FIG. 1** shows a flow diagram for a clinical study according to an embodiment of the disclosure.

## DETAILED DESCRIPTION

The disclosed methods may be understood more readily by reference to the following detailed description taken in connection with the accompanying figures, which form a part of this disclosure. It is to be understood that the disclosed methods are not limited to the specific methods described and/or shown herein, and that the terminology used herein is for the purpose of describing particular embodiments by way of example only and is not intended to be limiting of the claimed methods.

Unless specifically stated otherwise, any description as to a possible mechanism or mode of action or reason for improvement is meant to be illustrative only, and the disclosed methods are not to be constrained by the correctness or incorrectness of any such suggested mechanism or mode of action or reason for improvement.

It is to be appreciated that certain features of the disclosed methods which are, for clarity, described herein in the context of separate embodiments, may also be provided in combination in a single embodiment. Conversely, various features of the disclosed methods that are, for brevity, described in the context of a single embodiment, may also be provided separately or in any subcombination.

The term "about" when used in reference to numerical ranges, cutoffs, or specific values is used to indicate that the recited values may vary by up to as much as 10% from the listed value. As many of the numerical values used herein are experimentally determined, it should be understood by those skilled in the art that such determinations can, and often times will, vary among different experiments. The values used herein should not be considered unduly limiting by virtue of this inherent variation. Thus, the term "about" is used to encompass variations of $\pm$ 10% or less, variations of $\pm$ 5% or less, variations of $\pm$ 1% or less, variations of $\pm$ 0.5% or less, or variations of $\pm$ 0.1% or less from the specified value.

Where a range of numerical values is recited or established herein, the range includes the endpoints thereof and all the individual integers and fractions within the range, and also includes each of the narrower ranges therein formed by all the various possible combinations of those endpoints and internal integers and fractions to form subgroups of the larger group of values within the stated range to the same extent as if each of those narrower ranges was explicitly recited. For example, the range of "1 mg to about 10,000 mg" is inclusive of the endpoints, 1 mg and 10,000 mg, and all the intermediate values. Where a range of numerical values is stated herein as being greater than a stated value, the range is nevertheless finite and is bounded on its upper end by a value that is operable within the context of the invention as described herein. Where a range of numerical values is stated herein as being less than a stated value, the range is nevertheless bounded on its lower end by a non-zero value. It is not intended that the scope of the invention

be limited to the specific values recited when defining a range. All ranges are inclusive and combinable.

As used herein, the singular forms "a," "an," and "the" include the plural.

Various terms relating to aspects of the description are used throughout the specification and claims. Such terms are to be given their ordinary meaning in the art unless otherwise indicated. Other specifically defined terms are to be construed in a manner consistent with the definitions provided herein.

The term "comprising" is intended to include examples encompassed by the terms "consisting essentially of" and "consisting of"; similarly, the term "consisting essentially of" is intended to include examples encompassed by the term "consisting of."

The disclosure is directed to methods of treating or preventing mitochondrial dysfunction or mitochondrial disease in a subject, comprising administering to the subject probucol, or a pharmaceutically acceptable salt thereof, wherein the administration results in an improvement or delayed onset of one or more clinical symptoms selected from fatigue, muscle weakness, neuromuscular dysfunction, exercise intolerance, balance problems, dysautonomia, gastrointestinal problems, vision problems, eye muscle problems, retinal problems, optic nerve problems, ptosis, headache, dehydration, peripheral neuropathy, numbness, epilepsy, seizures, sleep problems, mood disorder, depression, diabetes mellitus, weight problems, kidney dysfunction, hyperlipidemia, liver disease, sleep apnea, autism spectrum behavior, behavioral problems, delayed developmental milestones, heart rhythm problems, heart muscle problems, cardiac disease, stroke, speech problems, tinnitus, hearing impairment, intellectual disability, learning disability, cognitive impairment, dementia, or a combination thereof.

Probucol is a small molecule pharmaceutical compound. Probucol was first approved by the FDA in 1977 as a lipid-lowering drug under the brand name LORELCO® and was voluntarily withdrawn from the U.S. market by the manufacturer in 1995. Under various brand names, probucol has also been prescribed in several Asian countries, including Japan, China, and South Korea, with dosages and indications similar to those previously approved in the United States. Probucol is a white crystalline powder with a melting point of 126 to 127° C. Its IUPAC name is 4,4'-[propane-2,2-diylbis(thio)]bis(2,6-di-tert-butylphenol) and it has been assigned CAS No. 23288-49-5. Probucol was first synthesized in the 1960s and its structure was unambiguously established using standard techniques (NMR, MS, IR). The molecular formula of probucol is $C_{31}H_{48}O_2S_2$ and its chemical structure is shown below:

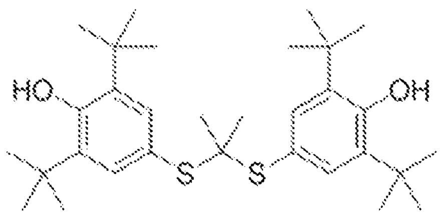

Probucol

.

"Pharmaceutically acceptable salt" refers to a salt of probucol. In particular, such salts are non-toxic, and may be inorganic or organic acid addition salts or base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. In certain aspects, the pharmaceutically acceptable salt of probucol is an organic amine salt. Preferably, the organic amine salt is a meglumine salt.

"Treat," "treatment," and like terms refer to therapeutic treatment that reduces the severity and/or frequency of symptoms, eliminates symptoms and/or the underlying cause of the symptoms, reduces the frequency or likelihood of symptoms and/or their underlying cause, improves or remediates damage caused, directly or indirectly, by the mitochondrial dysfunction or mitochondrial disease, delays the onset of mitochondrial dysfunction or mitochondrial disease,

or prevents the onset of mitochondrial dysfunction or mitochondrial disease. Subjects to be treated include those that have mitochondrial dysfunction or mitochondrial disease as well as those prone to develop mitochondrial dysfunction or mitochondrial disease. In certain embodiments, subjects with or prone to develop mitochondrial dysfunction or mitochondrial disease are clinically asymptomatic. Other treatment methods within the scope of the disclosure are those that prevent the onset of symptoms in subjects in which mitochondrial dysfunction or mitochondrial disease is to be prevented. Those subjects in which mitochondrial dysfunction or mitochondrial disease is to be prevented can be ascertained using methods described herein. Still other treatment methods within the scope of the disclosure are those that prevent decompensation in the face of stressors or those that add resiliency in the face of stressors, as measured using acceptable clinical methods known to one of skill in the art.

As used herein, "subject" includes mammals, in particular, domesticated animals such as dogs, cats, horses, pigs, sheep, cattle and the like. In preferred embodiments, "subject" includes humans. The terms "human," "patient," and "subject" are used interchangeably herein.

In certain embodiments, the mitochondrial dysfunction is respiratory chain dysfunction. Respiratory chain dysfunction, sometimes referred to as respiratory chain deficiency, represents a heterogeneous and highly morbid group of energy deficiency disorders involving aberrations in the cellular oxidative phosphorylation processes.

In certain embodiments, the mitochondrial dysfunction is associated with inherited mitochondrial disease. In other embodiments, the mitochondrial dysfunction is an acquired mitochondrial disease. In still other embodiments, the mitochondrial dysfunction is a primary mitochondrial disease. In other embodiments, the mitochondrial dysfunction is a secondary mitochondrial disease.

In certain embodiments, the mitochondrial dysfunction is associated with one or more genetic mutations, sometimes referred to as genetically-confirmed mitochondrial disease. Genetically-confirmed mitochondrial disease represents a class of disorders associated with a wide range of clinical and pathological features. Gorman et al., Nat. Rev. Dis. Prim. 2016; 2:16080. Symptoms are highly varied, and individuals can present at any age, from before birth into older adulthood. Parikh et al., Genet. Med. 2015; 17:689-701.

In embodiments of the disclosure the subject has a mitochondrial disease selected from autosomal dominant optic atrophy (or 'ADOA'); beta-oxidation defects; carnitine deficiency; carnitine-acyl-carnitine deficiency; chronic progressive external ophthalmoplegia syndrome (or 'CPEO'); co-enzyme Q10 deficiency; complex I deficiency (or 'NADH dehydrogenase deficiency'); complex II deficiency (or 'succinate dehydrogenase deficiency'); complex III deficiency (or 'ubiquinone-cytochrome c oxidoreductase deficiency'); complex IV deficiency (or 'cytochrome c oxidase deficiency' or 'COX deficiency'); complex V deficiency (or 'ATP synthase deficiency'); multiple respiratory chain complex deficiency; CPT I deficiency; CPT II deficiency; diabetes mellitus and deafness (or 'DAD'); creatine deficiency syndrome; Friedreich's ataxia (or 'FA'); Kearns-Sayre syndrome (or' KSS'); lactic acidosis; Leber's hereditary optic neuropathy (or 'LHON'); Leigh syndrome (or 'Leigh disease'); lethal infantile cardiomyopathy (or 'LIC' or 'Barth Syndrome'); leukodystrophy; leukoencephalopathy with brain stem and spinal cord involvement and lactate elevation (or 'LBSL'); long-chain acyl-CoA dehydrogenase deficiency (or 'LCAD'); long-chain 3-hydroxyacyl-CoA dehydrogenase (or 'LCHAD'); Luft disease; medium-chain acyl-CoA dehydrogenase deficiency (or 'MCAD'); mitochondrial cytopathy; mitochondrial DNA depletion; mitochondrial DNA deletion(s); mitochondrial encephalopathy; mitochondrial myopathy (or 'MM'); mitochondrial myopathy, encephalomyopathy, lactic acidosis, stroke-like symptoms (or 'MELAS'); mitochondrial neurogastrointestinal encephalomyopathy (or 'MNGIE'); mitochondrial recessive ataxia syndrome (or 'MIRAS'); multiple acyl-CoA dehydrogenase deficiency (or 'MAD' or 'glutaric aciduria type II'); myoclonic epilepsy with ragged red fibers (or 'MERRF'); neuropathy, ataxia, and retinitis pigmentosa (or 'NARP'); Pearson syndrome; POLG2 mutations; progressive infantile poliodystrophy (or 'Alper's disease'); ptosis; pyruvate carboxylase deficiency (or 'PCD'); pyruvate dehydrogenase complex deficiency (or 'PDCD' or 'PDH'); short-chain acyl-CoA dehydrogenase deficiency (or SCAD'); short chain 3-hydroxyacyl-CoA dehydrogenase deficiency (or 'SCHADD'); very long-chain acyl-CoA dehydrogenase deficiency (or 'VLCAD'); or a combination thereof.

In other embodiments of the disclosure the subject has been diagnosed with a mitochondrial disease selected from autosomal dominant optic atrophy (or 'ADOA'); beta-oxidation defects; carnitine deficiency; carnitine-acyl-carnitine deficiency; chronic progressive external ophthalmoplegia syndrome (or 'CPEO'); co-enzyme Q10 deficiency; complex I deficiency (or 'NADH dehydrogenase deficiency'); complex II deficiency (or 'succinate dehydrogenase deficiency'); complex III deficiency (or 'ubiquinone-cytochrome c oxidoreductase deficiency'); complex IV deficiency (or 'cytochrome c oxidase deficiency' or 'COX deficiency'); complex V deficiency (or 'ATP synthase deficiency'); multiple respiratory chain complex deficiency; CPT I deficiency; CPT II deficiency; diabetes mellitus and deafness (or 'DAD'); creatine deficiency syndrome; Friedreich's ataxia (or 'FA'); Kearns-Sayre syndrome (or' KSS'); lactic acidosis; Leber's hereditary optic neuropathy (or 'LHON'); Leigh syndrome (or 'Leigh disease'); lethal infantile cardiomyopathy (or 'LIC' or 'Barth Syndrome'); leukodystrophy; leukoencephalopathy with brain stem and spinal cord involvement and lactate elevation (or 'LBSL'); long-chain acyl-CoA dehydrogenase deficiency (or 'LCAD'); long-chain 3-hydroxyacyl-CoA dehydrogenase (or 'LCHAD'); Luft disease; medium-chain acyl-CoA dehydrogenase deficiency (or 'MCAD'); mitochondrial cytopathy; mitochondrial DNA depletion; mitochondrial DNA deletion(s); mitochondrial encephalopathy; mitochondrial myopathy

(or ’MM’); mitochondrial myopathy, encephalomyopathy, lactic acidosis, stroke-like symptoms (or ’MELAS’); mitochondrial neurogastrointestinal encephalomyopathy (or ’MNGIE’); mitochondrial recessive ataxia syndrome (or ’MIRAS’); multiple acyl-CoA dehydrogenase deficiency (or ’MAD’ or ’glutaric aciduria type II’); myoclonic epilepsy with ragged red fibers (or ’MERRF’); neuropathy, ataxia, and retinitis pigmentosa (or ’NARP’); Pearson syndrome; POLG2 mutations; progressive infantile poliodystrophy (or ’Alper’s disease’); ptosis; pyruvate carboxylase deficiency (or ’PCD’); pyruvate dehydrogenase complex deficiency (or ’PDCD’ or ’PDH’); short-chain acyl-CoA dehydrogenase deficiency (or SCAD’); short chain 3-hydroxyacyl-CoA dehydrogenase deficiency (or ’SCHADD’); very long-chain acyl-CoA dehydrogenase deficiency (or ’VLCAD’); or a combination thereof.

Probucol, or a pharmaceutically acceptable salt thereof, is administered in any suitable manner. Suitable routes of administration include, but are not limited to, oral, enteral (e.g., gastrostomy tube, jejunostomy tube, orogastric tube or nasogastric tube), parenteral (e.g., intravenous, subcutaneous, intramuscular), intraperitoneal, intracisternal, intraarticular, intracerebral (intraparenchymal and intracerebroventricular), nasal, vaginal, sublingual, intraocular, rectal, topical, transdermal and inhalation. In certain embodiments, administration is oral or enteral.

In certain embodiments, the improvement or delayed onset of one or more clinical symptoms is clinically significant. By "clinically significant," it is meant to include one or more improvements that one of ordinary skill in the art would consider (i) statistically significant, or (ii) practically important in that it has an observable positive effect on daily life. For example, clinically significant improvements can be improvements of pre-selected symptoms, as compared to pre-defined severity thresholds. In some embodiments, a clinically significant improvement of select symptoms includes the absence of statistically significant deterioration in any of other symptoms. In other embodiments, a clinically significant improvement includes an improvement in a quality of life parameter.

In particular embodiments, administration of probucol, or pharmaceutically acceptable salts thereof, results in a clinically significant improvement of a composite measurement that is computed using measurements from two or more of said clinical symptoms. In certain embodiments, the composite measurement is computed using a method comprising: (i) employing two or more sub-instruments to measure changes in one or more of said clinical symptoms over time, (ii) for each of said two or more sub-instruments, classifying the subject as (a) a sub-instrument responder or a sub-instrument non-responder, (b) a member of a clinical category, or (c) a member of a metric range, based on the change in said one or more clinical symptoms as measured using said sub-instrument, and (iii) employing an algorithm to combine the measurements obtained from said two or more sub-instruments into a single composite measurement, wherein said algorithm comprises (i) separately assessing the change in each measurement obtained from said two or more sub-instruments prior to combining each measurement into a single composite measurement, or (ii) combining measurements obtained from said two or more sub-instruments at a first time point and generating a single composite measurement for said first time point and then comparing the single composite measurement for said first time point to a single composite measurement generated from the same two or more sub-instruments at a second time point. Representative computations are detailed in the Examples provided herein.

By way of example, classifying the subject as a member of a clinical category may mean classifying the subject as having a normal or abnormal measurement.

By way of further example, classifying the subject as a member of a metric range may mean classifying the subject as falling within or outside a pre-defined numeric range.

In some aspects, said two or more measurement instruments are selected from Motor Function Measure, Six Minute Walk Test, Two Minute Walk Test, Modified Fatigue Impact Scale, Friedreich’s Ataxia Rating Scale, Patient-Reported Outcomes Measurement Information System Gastrointestinal Symptom Scales, 30-Second Chair Stand Test, Muscle Strength by Myometry Test, Newcastle Mitochondrial Disease Adult Scale, Newcastle Mitochondrial Disease Pediatric Scale, 36-Item Short Form Survey, Clinical Global Impression, Patient’s Global Impression, Patient-Reported Initial MitoPC Symptoms, Patient-Reported MitoPC Symptom Changes, Columbia Suicide Severity Rating Scale, Neuropathy Impairment Score, Migraine Disability Assessment, Quick Inventory of Depressive Symptomatology- Self-Report, Montreal Cognitive Assessment, NY Heart Association Functional Classification, Diabetes Health Profile, Ocular Motility Assessments, Marginal Reflex Distance, Ocular Motility & Fixation in 8 Gaze Directions, Degree of Ocular Saccades, Visual Function Tests, Logarithm of Minimum Angle of Resolution, Pelli-Robson Score, Humphrey Visual Field mean deviation, or a combination thereof. In preferred embodiments, said two or more measurement instruments are selected from Six Minute Walk Test, Motor Function Measure, Modified Fatigue Impact Scale, Friedreich’s Ataxia Rating Scale and Patient-Reported Outcomes Measurement Information System Gastrointestinal Symptom Scales. In more preferred embodiments, said two or more measurement instruments are the Six Minute Walk Test, Motor Function Measure, Modified Fatigue Impact Scale, Friedreich’s Ataxia Rating Scale and Patient-Reported Outcomes Measurement Information System Gastrointestinal Symptom Scales. Most of these measurement instruments, including how to conduct, measure, and evaluate them, are known in the art. Karaa et al., J. Inherit. Metab. Dis. 2017; 40(3):403-14.

In one aspect, the measurement instrument is a Motor Function Measure. In another aspect, the measurement instrument is a Six Minute Walk Test. In a further aspect, the measurement instrument is a Two Minute Walk Test. In one aspect, the measurement instrument is a Modified Fatigue Impact Scale. In another aspect, the measurement

instrument is a Friedreich's Ataxia Rating Scale. In a further aspect, the measurement instrument is a Patient-Reported Outcomes Measurement Information System Gastrointestinal Scale(s). In one aspect, the measurement instrument is a 30-Second Chair Stand Test. In another aspect, the measurement instrument is a Muscle Strength by Myometry Test. In another aspect, the measurement instrument is a Newcastle Mitochondrial Disease Adult Scale. In a further aspect, the measurement instrument is a Newcastle Mitochondrial Disease Pediatric Scale. In one aspect, the measurement instrument is a 36-Item Short Form Survey. In another aspect, the measurement instrument is a Clinical Global Impression. In another aspect, the measurement instrument is a Patient's Global Impression. In another aspect, the measurement instrument is a Patient-Reported Initial MitoPC Symptoms. In a further aspect, the measurement instrument is a Patient-Reported MitoPC Symptom Changes. In a further aspect, the measurement instrument is a Columbia Suicide Severity Rating Scale. In one aspect, the measurement instrument is a Neuropathy Impairment Score. In another aspect, the measurement instrument is a Migraine Disability Assessment. In a further aspect, the measurement instrument is a Quick Inventory of Depressive Symptomatology - Self-Report. In one aspect, the measurement instrument is a Montreal Cognitive Assessment. In another aspect, the measurement instrument is a NY Heart Association Functional Classification. In a further aspect, the measurement instrument is a Diabetes Health Profile. In one aspect, the measurement instrument is an Ocular Motility Assessment(s). In another aspect, the measurement instrument is a Marginal Reflex Distance. In a further aspect, the measurement instrument is an Ocular Motility & Fixation in 8 Gaze Directions. In one aspect, the measurement instrument is a Degree of Ocular Saccades. In another aspect, the measurement instrument is a Visual Function Test. In another aspect, the measurement instrument is a Logarithm of Minimum Angle of Resolution. In a further aspect, the measurement instrument is a Pelli-Robson Score. In one aspect, the measurement instrument is a Humphrey Visual Field mean deviation. In another aspect, the measurement is a Patient-Reported Initial MitoPC Symptoms. In a further aspect, the measurement is a Patient-Reported MitoPC Symptom Changes.

In certain embodiments, the methods disclosed herein further comprise administering one or more additional pharmaceutical agents. As used herein, "pharmaceutical agents" means approved or approvable by a regulatory agency of the United States Federal or a state government, or a corresponding agency in countries other than the United States, or that is listed in the U.S. Pharmacopoeia or other generally recognized pharmacopoeia for use in animals, and, more particularly, in humans.

In aspects of the disclosure, the one or more additional pharmaceutical agents are selected from niacin, cycloheximide, bezafibrate, vatiquinone, carnitine, coenzyme Q10, alpha-tocopherolquinone, coenzyme Q10 analogs, cytochrome C, dichloroacetate, 5-[(e)-2-(4-hydroxyphenyl)-ethenyl] benzene-1,3 diol, flavin mononucleotide, elamipretide, idebenone, latrepirdine, levocarnitine, 2',3',5'-tri-O-acetyluridine, olesoxime, omaveloxolone, thiamin diphosphate, ubiquinone, vitamin C, vitamin D, vitamin E, thiamine, riboflavin, magnesium, calcium, phosphate, membrane phospholipid, unsaturated fatty acid, creatine, pyruvate, $\alpha$-lipoic acid, NADH, PPAR$\delta$ modulator, nicotinic acid, nicotinamide, nicotinamide riboside, acipimox, resveratrol, glucose, L-carnitine, glutathione, redox-modulating agent, dichloroacetate, curcumin, schisandrin, triheptanoin, viral or non-viral vector gene therapy targeting mitochondrial disease mutations, or a combination thereof. Polyak et al., Mol. Genet. Metab. 2018; 123(4):449-462. Peng et al., Hum. Mol. Genet. 2015; 24(17):4829-4847. Zhang et al., PLoS One 2013; 8(7):e69282. McCormack et al., Mitochondrion 2015; 22:45-59.

In certain embodiments, the subject is diagnosed as having a genetic mitochondrial disease prior to the administration. In some aspects, the diagnosis of mitochondrial disease my involve cellular or tissue biopsy-based biochemical investigations or massively parallel DNA or RNA sequencing in blood and/or tissue.

In some aspects, the diagnosis is achieved by: (a) sequencing the subject's nucleic acid, or a portion thereof; (b) comparing the subject's nucleic acid sequence or a portion thereof to a sequence in a database comprising sequences containing pathologic nucleic acid mutations; and (c) determining whether the subject's nucleic acid sequence has at least one pathologic nucleic acid mutation in said database. In preferred embodiments, the database comprising sequences containing pathologic nucleic acid mutations refers to the Mitochondrial Disease Sequence Data Resource (MSeqDR), a community-wide Internet resource to curate mitochondrial disease genomic data. Falk et al., Mol. Genet. Metab. 2015; 114(3):388-396. As of 2019, the MSeqDR manages 267 mitochondrial diseases, 1,607 genes associated with mitochondrial biology or disease, and 4,486 pathogenic variants in those genes. Shen et al., Hum. Mutat. 2016; 37(6):540-548. MseqDR web site: mseqdr.org/mb.php?url=index.php. Accessed on June 7, 2019. In certain embodiments, the nucleic acid is DNA. In certain embodiments, the nucleic acid is RNA. In certain embodiments, the nature, abundance and location of chemical modifications to component nucleotides in the nucleic acid are employed for diagnostic purposes.

In some embodiments, the method of the present disclosure may comprise selecting and/or isolating a genetic mutation or variation of interest, and quantifying the amount of each locus present (for example for determining copy number) and/or the relative amounts of different locus variants (for example two alleles of a given DNA sequence).

In another aspect, the methods of the present disclosure may comprise contacting one or more probes to the nucleic acid sample, hybridizing the probes to the nucleic acid region of interest, and/or amplifying the probes. Moreover, the immobilization of the probe to a substrate (e.g. array) may be performed simultaneously or before contacting the

probes to the nucleic acid sample, hybridizing the probes to the nucleic acid region of interest, amplifying and/or detecting probe hybridization. In this alternative embodiment, the nucleic acid mutation or variation can be detected without sequencing.

In some embodiments, a genetic mutation or variation may have a reference sequence associated with it. "Reference sequence" as used herein denotes a sequence to which a locus of interest in a nucleic acid is being compared. In certain embodiments, a reference sequence is considered a "wild type" sequence for a locus of interest. A nucleic acid that contains a locus of interest having a sequence that varies from a reference sequence for the locus of interest is sometimes referred to as polymorphic or mutant or genetic variation. A nucleic acid that contains a locus of interest having a sequence that does not vary from a reference sequence for the locus of interest is sometimes referred to as wild type or non-genetic variation. In certain embodiments, a locus of interest may have more than one distinct reference sequence associated with it (e.g., where a locus of interest is known to have a polymorphism that is to be considered a normal or wild type).

In additional embodiments, the region of interest described herein may include "consensus genetic variant sequence" which refers to the nucleic acid or protein sequence, the nucleic or amino acids of which are known to occur with high frequency in a population of individuals who carry the gene which codes for a mitochondrial dysfunction. Moreover, the region of interest described herein may include "consensus normal gene sequence" which refers to a nucleic acid sequence, the nucleic acid of which is known to occur at the respective positions with high frequency in a population of individuals who carry the gene which codes for a protein not functioning normally, or which itself does not function normally. In further embodiments, the control region that is not the region of interest or the reference sequence described herein may include "consensus normal sequence" which refers to the nucleic acid or protein sequence, the nucleic or amino acids of which are known to occur with high frequency in a population of individuals who carry the gene which codes for a normally functioning protein, or in which the nucleic acid itself has normal function.

As of 2016, there were nearly 300 distinct genes in which mutations are known to cause mitochondrial disease (Gorman et al., Nat. Rev. Dis. Prim. 2016; 2:16080. Koopman et al., N. Engl. J. Med. 2012; 366:1132-41), with pathogenic etiologies arising in both the nuclear DNA genome and the mitochondrial DNA (mtDNA) genome. McCormick et al., Neurotherapeutics 2013; 10:251-61. In certain embodiments, the at least one pathologic mutation is a substitution, deletion, or insertion.

In certain embodiments, the at least one pathologic nucleic acid mutation is a mitochondrial DNA (mtDNA) mutation or a combination of mtDNA mutations. Exemplary mtDNA-encoded genes associated with mitochondrial disease when mutated *include MT-ND1, MT-ND2, MT-ND3, MT-ND4, MT-ND4L, MT-ND5, MT-ND6, MT-CYB, MT-CO1, MT-CO2, MT-CO3, MT-RNR1, MT-RNR2, MT-TA, MT-TC, MT-TE, MT-TF, MT-TH, MT-TI, MT-TK, MT-TL1, MT-TL2, MT-TM, MT-TN, MT-TO, MT-TS1, MT-TS2, MT-TV, MT-TW, MT-ATP6,* and *MT-ATP8,* and combinations thereof. In certain embodiments, the mtDNA mutation is in a gene for a mitochondrial transfer RNA (mt-tRNA) or in a gene for a mitochondrial ribosomal RNA (mt-rRNA).

In certain embodiments, the at least one pathologic nucleic acid mutation is a nuclear DNA (nDNA) mutation or a combination of nDNA mutations. Exemplary nDNA-encoded genes associated with mitochondrial disease when mutated include, but are not limited to, *AARS2, ABCB7, ABCC8, ACAD8, ACAD9, ACADM, ACADS, ACADSB, ACADVL, ACAT1, ACO2, ADCK3, ADRB2, ADRB3, AFG3L2, AGK, AGRP, AIFM1, AK2, AKAP10, AKT2, ALAS2, ALDH2, ALDH4A1, ALDH6A1, AMACR, AMT, APOPT1, APTX, ARMS2, ATPSA1, ATP5E, ATP5F1A, APT5F1D, ATP5F1E, ATPAF2, AUH, BAX, BCAT2, BCKDHA, BCKDHB, BCL2, BCS1L, BOLA3, C8orf38, C10orf2, C12orf62, C12orf65, Cl9orf12, C20orf7, C1QBP, CAPN10, CARS2, CARTPT, CDH23, CDKAL1, CHCHD10, CHKB, CISD2, CLRN1,COA5, COA7, COA10, COQ2, COQ4, COQ6, COQ7, COQ8A, COQ9, COX10, COX14, COX15, COX20, COX412, COX681, COX8A, CPS1, CPT1A, CPT2, CRAT, CYB5R3, CYC1, CYC5, CYCS, CYP11A1, CYP1181, CYP1182, CYP24A1, CYP27A1, CYP2781, D2HGDH, DARS2, DBT, DECR1, DFNB31, DGUOK, DHODH, DIABLO, DLD, DLAT, DMGDH, DNA2, DNAJC19, DNM1L, EARS2, ECHS1, ELAC2, ENPP1, ETFA, ETFB, ETFDH, ETHE1, FARS2, FASTKD2, FBXL4, FOXRED1, FH, FXN, GATM, GCDH, GCGR, GCK, GCSH, GDAP1, GFER, GFM1, GHRL, GJ82, GJB3, GJ86, GK, GLDC, GLRX5, GLUD1, GPD2, GPR98, GTPBP3, HADH, HADHA, HADHB, HARS2, HCCS, HIBCH, HK1, HLCS, HMGA1, HMGCS2, HMGCL, HNF1A, HNF1B, HNF4A, HOGA1, HSD17810, HSPD1, HTRA1, HTRA2, IBA57, IDH2, IDH3B, IGF2BP2, IL6, INSR, IRS1, IRS2, ISCA1, ISCA2, ISCU, IVD, KANK1, KARS, KCNJ11, KIF18, L2HGDH, LARS2, LEPR, LIAS, LIPC, LRPPRC, LRRK2, LYRM4, LYRM7, MAOA, MAPK8IP1, MARS2, MC4R, MCCC1, MCCC2, MCEE, ME2, MFF, MFN2, MGME1, MIPEP, MLYCD, MMAA, MMAB, MMADHC, MPC1, MPV17, MRAP2, MRPL3, MRPL44, MRPS2, MRPS7, MRPS16, MRPS22, MRPS34, MSTO1, MTFMT, MTO1, MTPAP, MUT, MYO7A, NAGS, NARS2, NDUFA1, NDUFA2, NDUFA6, NDUFA9, NDUFA10, NDUFA11, NDUFA12, NDUFA13, NDUFAF1, NDUFAF2, NDUFAF3, NDUFAF4, NDUFAF5, NDUFAF6, NDUFB3, NDUFB8, NDUFB9, NDUFS1, NDUFS2, NDUFS3, NDUFS4, NDUFS5, NDUFS6, NDUFS7, NDUFS8, NDUFV1, NDUFV2, NEUROD1, NF2, NFU1, NGLV1, NROB2, NUBPL, NUP62, OAT, OGDH, OPA1, OPA3, OTC, OXCT1, PANK2, PARK2, PARK7, PAX4, PC, PCCA, PCCB, PCDH15, PCK2, PDHA1, PDHB, PDHX, PDP1, PDSS1, PDSS2, PDX1, PET100, PINK1, PNKD, PNPT1, POLG, POLG2, POMC, PPARG, PPARGC1B, PPOX, PPP1R3A, PUS1, PYCR1, PYY, RARS2, REEP1, RMRP, RMND1, RNASEH1, RRM2B, SACS,*

*SARDH, SARS2, SCO1, SCO2, SCO3, SDHA, SDHAF1, SDHAF2, SDHB, SDHC, SDHD, SERAC1, SFXN4, SIM1, SLC2A2, SLC2A4, SLC22A5, SLC25A1, SLC25A3, SLC25A4, SLC25A12, SLC25A13, SLC25A15, SLC25A19, SLC25A20, SLC25A22, SLC25A26, SLC25A38, SLC30A8, SOD2, SPG7, SPG20, STAR, SUCLA2, SUCLG1, SURF1, TACO1, TARS2, TAZ, TDF7L2, TFAM, TIMM8A, TIMMDC1, TK2, TMEM70, TMEM126, TMEM126B, TOP3A, TRIT1, TRMT10C, TRMT5, TRMU, TRNT1, TSFM, TTC19, TUFM, TWNK, UCP1, UCP2, UCP3, UNG, UQCC2, UQCC3, UQCRB, UQCRC2, UQCRQ, USMG5, USH1C, USH1G, USG2A, VARS2, WARS2, WF51, WWOX, XPNPEP3, YARS2,* and combinations thereof.

In some aspects, the nucleic acid mutation or variation is in a gene selected from any of the genes recited in Tables 8 or 9 or any combination thereof. Table 8 is a classification of genes associated with mitochondrial diseases based on information from from Kremer et al., Mitochondrial Disease Genetics in Diagnosis and Management of Mitochondrial Disorders; Mancuso and Klopstock (Eds); Springer 2019; pp 41-62. Table 9 is based on informaiton from the Mitochondrial Disease Sequence Data Resource (MSeqDR.org). Shen et al., MSeqDR: a centralized knowledge repository and bioinformatics web resource to facilitate genomic investigations in mitochondrial disease, Hum. Mutat. 2016; 37(6):540-548. Details about the nucleic acid mutation or variation can be found at www.omim.org which is the Online Mendelian Inheritance in Man (OMIM) database. OMIM is a comprehensive, authoritative compendium of human genes and genetic phenotypes. Information on each genetic mutation or variation can be found in this database based on the OMIM identifier provided in Table 9. The information for each nucleic acid mutation or variation recited in Table 9 from the OMIM database is herein incorporated by reference in its entirety.

In some aspects, the method may detect from 1 to 100, from 1 to 50, from 1 to 25, from 2 to 10, or from 5 to 10 nucleic acid mutations or variations; 2, 3, 4, 5, 6, 7, 8, 9, 10 or more nucleic acid mutations or variations; and 100, 50, 30, 20, 10 or less nucleic acid mutations or variations from any of Tables 8 or 9. In another aspect, the method according to some embodiments may detect at least two nucleic acid mutations or variations from Tables 8 or 9. In another aspect, the method according to some embodiments may detect at least three nucleic acid mutations or variations from Tables 8 or 9. In another aspect, the method may according to some embodiments detect at least four nucleic acid mutations or variations from Tables 8 or 9. In another aspect, the method according to some embodiments may detect at least five nucleic acid mutations or variations from Tables 8 or 9. In another aspect, the method according to some embodiments may detect at least six nucleic acid mutations or variations from Tables 8 or 9. In another aspect, the method according to some embodiments may detect at least seven nucleic acid mutations or variations from Tables 8 or 9. In another aspect, the method according to some embodiments may detect at least eight nucleic acid mutations or variations from Tables 8 or 9. In another aspect, the method according to some embodiments may detect at least nine nucleic acid mutations or variations from Tables 8 or 9. In another aspect, the method according to some embodiments may detect at least ten nucleic acid mutations or variations from Tables 8 or 9.

In some aspects, the pathologic nucleic acid mutation is a combination of at least one mtDNA mutation and at least one nDNA mutation, and/or are dependent on mtDNA haplogroup background and/or environmental exposure.

In certain aspects of the disclosure, the mitochondrial dysfunction is associated with neurodegenerative disorders such as Parkinson's disease, amyotrophic lateral sclerosis, multiple sclerosis, or Huntington's disease. In some aspects of the disclosure, the mitochondrial dysfunction is associated with cognition, psychiatric, and/or mood impairment. In further aspects of the disclosure, the mitochondrial dysfunction is associated with Alzheimer's disease. In still further aspects of the disclosure, the mitochondrial dysfunction is associated with cardiac dysfunction.

In certain embodiments, the subject is 18 years or older. That is, in some embodiments, the subject is an adult. For example, the subject can be 18 years or older, 19 years or older, 20 years or older, 21 years or older, 22 years or older, 23 years or older, 24 years or older, 25 years or older, 26 years or older, 27 years or older, 28 years or older, 29 years or older, 30 years or older, 31 years or older, 32 years or older, 33 years or older, 34 years or older, 35 years or older, 36 years or older, 37 years or older, 38 years or older, 39 years or older, 40 years or older, 41 years or older, 42 years or older, 43 years or older, 44 years or older, 45 years or older, 46 years or older, 47 years or older, 48 years or older, 49 years or older, 50 years or older, 51 years or older, 52 years or older, 53 years or older, 54 years or older, 55 years or older, 56 years or older, 57 years or older, 58 years or older, 59 years or older, 60 years or older, 61 years or older, 62 years or older, 63 years or older, or 64 years or older. In other aspects, the subject is a geriatric adult, for example, the subject is 65 years or older or 70 years or older. For example, the geriatric subject can be 65 years or older, 66 years or older, 67 years or older, 68 years or older, 69 years or older, 70 years or older, 71 years or older, 72 years or older, 73 years or older, 74 years or older, 75 years or older, or 80 years or older.

In some aspects, the subject is a child, that is, the subject is less than 18 years old. For example, the subject can be less than 18 years, 17 years or younger, 16 years or younger, 15 years or younger, 14 years or younger, 13 years or younger, 12 years or younger, 11 years or younger, 10 years or younger, 9 years or younger, 8 years or younger, 7 years or younger, 6 years or younger, 5 years or younger, 4 years or younger, 3 years or younger, 2 years or younger, or 1 year or younger. In some aspects, the subject is a fetus, newborn, or infant. In some aspects, the subject is an adolescent. In certain embodiments, the subject is between about 3 weeks to about 52 weeks of age. In preferred embodiments, the subject is 18 years or older, 17 years or younger, 12 years or younger, 65 years or younger, 75 years

or younger, or between about 3 weeks to about 52 weeks of age.

In aspects of the disclosure, probucol, or a pharmaceutically acceptable salt thereof, is administered at a dose of about 1 mg to about 10,000 mg. In other embodiments, probucol, or pharmaceutically acceptable salts thereof, is administered at a dose of about 1 mg to about 5,000 mg. In other embodiments, probucol, or a pharmaceutically acceptable salt thereof, is administered at a dose of about 1 mg/day to about 1,000 mg/day. In other embodiments, probucol, or a pharmaceutically acceptable salt thereof, is administered at a dose of about 1 mg to about 500 mg. In other embodiments, probucol, or a pharmaceutically acceptable salt thereof, is administered at a dose of about 1 mg to about 100 mg. In other embodiments, probucol, or a pharmaceutically acceptable salt thereof, is administered at a dose of about 1 mg to about 50 mg. In aspects of the disclosure, probucol, or a pharmaceutically acceptable salt thereof, is administered at a dose of about 10 mg to about 10,000 mg. In other embodiments, probucol, or a pharmaceutically acceptable salt thereof, is administered at a dose of about 10 mg to about 5,000 mg. In other embodiments, probucol, or a pharmaceutically acceptable salt thereof, is administered at a dose of about 10 mg to about 1,000 mg. In other embodiments, probucol, or a pharmaceutically acceptable salt thereof, is administered at a dose of about 10 mg to about 500 mg. In other embodiments, probucol, or a pharmaceutically acceptable salt thereof, is administered at a dose of about 10 mg to about 100 mg. In other embodiments, probucol, or a pharmaceutically acceptable salt thereof, is administered at a dose of about 10 mg to about 50 mg. In aspects of the disclosure, probucol, or a pharmaceutically acceptable salt thereof, is administered at a dose of about 50 mg to about 10,000 mg. In other embodiments, probucol, or a pharmaceutically acceptable salt thereof, is administered at a dose of about 50 mg to about 5,000 mg. In other embodiments, probucol, or a pharmaceutically acceptable salt thereof, is administered at a dose of about 50 mg to about 1,000 mg. In other embodiments, probucol, or a pharmaceutically acceptable salt thereof, is administered at a dose of about 50 mg to about 500 mg. In other embodiments, probucol, or a pharmaceutically acceptable salt thereof, is administered at a dose of about 50 mg to about 1,000 mg. In aspects of the disclosure, probucol, or a pharmaceutically acceptable salt thereof, is administered at a dose of about 100 mg to about 10,000 mg. In other embodiments, probucol, or a pharmaceutically acceptable salt thereof, is administered at a dose of about 100 mg to about 5,000 mg. In other embodiments, probucol, or a pharmaceutically acceptable salt thereof, is administered at a dose of about 100 mg to about 1,000 mg. In other embodiments, probucol, or a pharmaceutically acceptable salt thereof, is administered at a dose of about 100 mg to about 500 mg. In aspects of the disclosure, probucol, or a pharmaceutically acceptable salt thereof, is administered at a dose of about 500 mg to about 10,000 mg. In other embodiments, probucol, or a pharmaceutically acceptable salt thereof, is administered at a dose of about 500 mg to about 5,000 mg. In other embodiments, probucol, or a pharmaceutically acceptable salt thereof, is administered at a dose of about 500 mg to about 1,000 mg. In aspects of the disclosure, probucol, or a pharmaceutically acceptable salt thereof, is administered at a dose of about 1,000 mg to about 10,000 mg. In other embodiments, probucol, or a pharmaceutically acceptable salt thereof, is administered at a dose of about 1,000 mg to about 5,000 mg. In aspects of the disclosure, probucol, or a pharmaceutically acceptable salt thereof, is administered at a dose of about 5,000 mg to about 10,000 mg. For example, probucol, or a pharmaceutically acceptable salt thereof, is administered at a dose of about 1 mg, about 2 mg, about 3 mg, about 4 mg, about 5 mg, about 6 mg, about 7 mg, about 8 mg, about 9 mg, about 10 mg, about 20 mg, about 30 mg, about 40 mg, about 50 mg, about 60 mg, about 70 mg, about 80 mg, about 90 mg, about 100 mg, about 150 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1,000 mg, about 1,500 mg, about 2,000 mg, about 2,500 mg, about 3,000 mg, about 3,500 mg, about 4,000 mg, about 4,500 mg, about 5,000 mg, about 5,500 mg, about 6,000 mg, about 6,500 mg, about 7,000 mg, about 7,500 mg, about 8,000 mg, about 8,500 mg, about 9,000 mg, about 9,500 mg or about 10,000 mg. In preferred aspects, probucol, or a pharmaceutically acceptable salt thereof, is administered at a dose of about 1 mg, about 10 mg, about 100 mg, about 250 mg, about 500 mg, about 1,000 mg, about 5,000 mg or about 10,000 mg.

In some embodiments, probucol, or a pharmaceutically acceptable salt thereof, is administered on a continuous dosing schedule. As used herein "continuous dosing schedule" refers to continuous drug administration at a prescribed frequency (e.g. daily) without intermission. The continuous dosing schedule is distinguished from intermittent administration. By way of example, the continuous dosing schedule may be daily or less frequently than daily. For example, in some embodiments, probucol, or a pharmaceutically acceptable salt thereof, may be administered at a dosing interval of once per day, once every 2 days, once every 3 days, once every 4 days, once every 5 days, once every 6 days, once a week, once every 10 days, once every two weeks, once every three weeks, or once a month, without intermission. In other preferred embodiments, probucol, or a pharmaceutically acceptable salt thereof, may be administered at a dosing interval of twice per day, twice per week, twice per month, three times per day, three times per week, three times per month, four times per day, four times per week or four times per month, without intermission. In more preferred embodiments, probucol, or a pharmaceutically acceptable salt thereof, is administered twice per day, once per day, once every two days, once every three days, once every four days, once every five days, once every six days, once a week, once every two weeks or once per month.

By way of example, as used herein, once weekly dosing means that probucol, or a pharmaceutically acceptable

salt thereof, is administered once a week, i.e. one time during a seven day period, preferably on the same day of each week. A non-limiting example of administration of a dose of 1,000 mg administered at a dosing interval of once per week would entail administered a single unit dose of 1,000 mg every Sunday. In preferred embodiments, the unit dose is not administered on the same or consecutive days, but a dose of twice per week can include a dosing regimen in which unit doses are administered on the same or consecutive days within a weekly period or different weekly periods.

In some embodiments, probucol, or a pharmaceutically acceptable salt thereof, is administered at a dose of about 1 mg/day to about 10,000 mg/day. In other embodiments, probucol, or a pharmaceutically acceptable salt thereof, is administered at a dose of about 1 mg/day to about 5,000 mg/day. In other embodiments, probucol, or a pharmaceutically acceptable salt thereof, is administered at a dose of about 1 mg/day to about 1,000 mg/day. In other embodiments, probucol, or a pharmaceutically acceptable salt thereof, is administered at a dose of about 1 mg/day to about 500 mg/day. In other embodiments, probucol, or a pharmaceutically acceptable salt thereof, is administered at a dose of about 1 mg/day to about 100 mg/day. In other embodiments, probucol, or a pharmaceutically acceptable salt thereof, is administered at a dose of about 1 mg/day to about 50 mg/day. In aspects of the disclosure, probucol, or a pharmaceutically acceptable salt thereof, is administered at a dose of about 10 mg/day to about 10,000 mg/day. In other embodiments, probucol, or a pharmaceutically acceptable salt thereof, is administered at a dose of about 10 mg/day to about 5,000 mg/day. In other embodiments, probucol, or a pharmaceutically acceptable salt thereof, is administered at a dose of about 10 mg/day to about 1,000 mg/day. In other embodiments, probucol, or a pharmaceutically acceptable salt thereof, is administered at a dose of about 10 mg/day to about 500 mg/day. In other embodiments, probucol, or a pharmaceutically acceptable salt thereof, is administered at a dose of about 10 mg/day to about 100 mg/day. In other embodiments, probucol, or a pharmaceutically acceptable salt thereof, is administered at a dose of about 10 mg/day to about 50 mg/day. In aspects of the disclosure, probucol, or a pharmaceutically acceptable salt thereof, is administered at a dose of about 50 mg/day to about 10,000 mg/day. In other embodiments, probucol, or a pharmaceutically acceptable salt thereof, is administered at a dose of about 50 mg/day to about 5,000 mg/day. In other embodiments, probucol, or a pharmaceutically acceptable salt thereof, is administered at a dose of about 50 mg/day to about 1,000 mg/day. In other embodiments, probucol, or a pharmaceutically acceptable salt thereof, is administered at a dose of about 50 mg/day to about 500 mg/day. In other embodiments, probucol, or a pharmaceutically acceptable salt thereof, is administered at a dose of about 50 mg/day to about 1,000 mg/day. In aspects of the disclosure, probucol, or a pharmaceutically acceptable salt thereof, is administered at a dose of about 100 mg/day to about 10,000 mg/day. In other embodiments, probucol, or a pharmaceutically acceptable salt thereof, is administered at a dose of about 100 mg/day to about 5,000 mg/day. In other embodiments, probucol, or a pharmaceutically acceptable salt thereof, is administered at a dose of about 100 mg/day to about 1,000 mg/day. In other embodiments, probucol, or a pharmaceutically acceptable salt thereof, is administered at a dose of about 100 mg/day to about 500 mg/day. In aspects of the disclosure, probucol, or a pharmaceutically acceptable salt thereof, is administered at a dose of about 500 mg/day to about 10,000 mg/day. In other embodiments, probucol, or a pharmaceutically acceptable salt thereof, is administered at a dose of about 500 mg/day to about 5,000 mg/day. In other embodiments, probucol, or a pharmaceutically acceptable salt thereof, is administered at a dose of about 500 mg/day to about 1,000 mg/day. In aspects of the disclosure, probucol, or a pharmaceutically acceptable salt thereof, is administered at a dose of about 1,000 mg/day to about 10,000 mg/day. In other embodiments, probucol, or a pharmaceutically acceptable salt thereof, is administered at a dose of about 1,000 mg/day to about 5,000 mg/day. In aspects of the disclosure, probucol, or a pharmaceutically acceptable salt thereof, is administered at a dose of about 5,000 mg/day to about 10,000 mg/day.

In some embodiments, probucol, or a pharmaceutically acceptable salt thereof, may be administered as a single unit dose or as multiple unit doses. By way of example, administration of a dose of 1,000 mg can entail administration of a single unit dose of 1,000 mg, two unit doses of 500 mg or four unit doses of 250 mg and so on such that the sum of all single unit doses is equal to the recited dose. By way of further example, administration of a dose of 1,000 mg/day can entail administration of a single unit dose of 1,000 mg each day, two unit doses of 500 mg or four unit doses of 250 mg and so on such that the sum of all single unit doses each day is equal to the recited dose.

In other embodiments, probucol, or a pharmaceutically acceptable salt thereof, is administered on an intermittent dosing schedule. As used herein, "intermittent dosing schedule" refers to non-continuous drug administration at a prescribed frequency (e.g. daily) with intermission. For example, in some embodiments, probucol, or a pharmaceutically acceptable salt thereof, may be administered daily for four weeks, followed by two weeks off, followed again by daily administration for four weeks.

In certain embodiments, probucol, or a pharmaceutically acceptable salt thereof, is administered initially in a loading dose that is higher than a subsequent dose. In other embodiments, probucol, or a pharmaceutically acceptable salt thereof, is administered initially in a loading dose that is lower than a subsequent dose.

All formulations are in dosages suitable for administration to a human. The pharmaceutical formulations described herein include, but are not limited to, solid formulations (e.g., tablets, capsules, and suppositories), semisolid formulations, and liquid formulations (e.g., suspensions and solutions). By way of example, the pharmaceutical formulations contemplated herein include aqueous liquid dispersions, self-emulsifying dispersions, solid solutions, liposomal

dispersion, aerosols, solid dosage forms, powders, immediate release formulations, controlled release formulations, fast melt formulations, tablets, capsules, pills, patches, delayed release formulations, extended release formulations, pulsatile release formulations, multi-particulate formulations, and mixed immediate and controlled release formulations. In certain preferred embodiments, the pharmaceutical formulation is a tablet. In certain preferred embodiments, the pharmaceutical formulation is a liquid.

In certain aspects of the disclosure, probucol, or a pharmaceutically acceptable salt thereof, is administered in combination with food. In some embodiments, the food is a high fat food. Examples of high fat foods include oils, meat, whole-milk dairy, butter, eggs, nuts, seeds, and avocados.

In some aspects, the methods disclosed herein may result in a treatment related adverse event (AE) as established by the Common Terminology Criteria for Adverse Events (CTCAE), published by the U.S. Department of Health and Human Services. As used herein, an AE is any untoward medical occurrence in a subject who has received an intervention (drug, biologic, or other intervention). The occurrence does not necessarily have to have a causal relationship with the study treatment. An AE can therefore be any unfavorable or unintended incident (including an abnormal laboratory finding, for example), symptom, or disease temporally associated with the use of a medicinal product, whether or not considered related to the medicinal product. Grade numbers refers to the severity of the AE, where grade 1 is least severe and grade 5 is death related to AE.

In certain aspects, the methods disclosed herein may not result in a grade 4 or grade 5 adverse event. In other aspects, the treatments described herein may result in no more than a grade 1 adverse event. In further aspects, the treatments described herein may result in no more than a grade 2 adverse event. In still further aspects, the treatments described herein may result in no more than a grade 3 adverse event.

The disclosure is further directed to methods of diagnosing mitochondrial dysfunction or genetic mitochondrial disease in a subject, comprising (a) sequencing the subject's nucleic acid or a portion thereof; (b) comparing the subject's nucleic acid sequence or a portion thereof to one or more sequences in a database comprising reference population and pathologic nucleic acid mutations; and (c) determining whether the subject's nucleic acid sequence has at least one pathologic mutation in the database. In certain embodiments, the pathologic mutation is predicted. In certain embodiments, the pathologic mutation is confirmed.

Aspects of the methods of diagnosis disclosed herein may further comprise administering to the subject from, for example, about 1 mg to about 10,000 mg of probucol, or a pharmaceutically acceptable salt thereof, or any intermediate dose, as described herein, using any of the dosing protocols described herein.

In certain embodiments, the administration results in an improvement of one or more clinical symptoms selected from fatigue, muscle weakness, neuromuscular dysfunction, exercise intolerance, balance problems, dysautonomia, gastrointestinal problems, vision problems, eye muscle problems, retinal problems, optic nerve problems, ptosis, headache, dehydration, peripheral neuropathy, numbness, epilepsy, seizures, sleep problems, mood disorder, depression, diabetes mellitus, weight problems, kidney dysfunction, hyperlipidemia, liver disease, sleep apnea, autism spectrum behavior, behavioral problems, delayed developmental milestones, heart rhythm problems, heart muscle problems, cardiac disease, stroke, speech problems, tinnitus, hearing impairment, intellectual disability, learning disability, cognitive impairment, dementia, or a combination thereof.

In certain embodiments, the administration results in an improvement of fatigue. In certain embodiments, the administration results in an improvement of muscle weakness. In certain embodiments, the administration results in an improvement of neuromuscular dysfunction. In certain embodiments, the administration results in an improvement of exercise intolerance. In certain embodiments, the administration results in an improvement of a balance problem. In certain embodiments, the administration results in an improvement of dysautonomia. In certain embodiments, the administration results in an improvement of a gastrointestinal problem. In certain embodiments, the administration results in an improvement of a vision problem. In certain embodiments, the administration results in an improvement of an eye muscle problem. In certain embodiments, the administration results in an improvement of a retinal problem. In certain embodiments, the administration results in an improvement of an optic nerve problem. In certain embodiments, the administration results in an improvement of ptosis. In certain embodiments, the administration results in an improvement of headache. In certain embodiments, the administration results in an improvement of dehydration. In certain embodiments, the administration results in an improvement of peripheral neuropathy. In certain embodiments, the administration results in an improvement of numbness. In certain embodiments, the administration results in an improvement of epilepsy. In certain embodiments, the administration results in an improvement of seizures. In certain embodiments, the administration results in an improvement of a sleep problem. In certain embodiments, the administration results in an improvement of a mood disorder. In certain embodiments, the administration results in an improvement of a psychiatric disorder. In certain embodiments, the administration results in an improvement of depression. In certain embodiments, the administration results in an improvement of diabetes mellitus. In certain embodiments, the administration results in an improvement of a weight problem. In certain embodiments, the administration results in an improvement of kidney dysfunction. In certain embodiments, the administration results in an improvement of hyperlipidemia. In certain embodiments, the administration results in an improvement of liver disease. In certain embodiments, the administration results

in an improvement of sleep apnea. In certain embodiments, the administration results in an improvement of autism spectrum behavior. In certain embodiments, the administration results in an improvement of a behavioral problem. In certain embodiments, the administration results in an improvement of a delayed developmental milestone. In certain embodiments, the administration results in an improvement of a heart rhythm problem. In certain embodiments, the administration results in an improvement of a heart muscle problem. In certain embodiments, the administration results in an improvement of cardiac disease. In certain embodiments, the administration results in an improvement of stroke. In certain embodiments, the administration results in an improvement of a speech problem. In certain embodiments, the administration results in an improvement of tinnitus. In certain embodiments, the administration results in an improvement of a hearing impairment. In certain embodiments, the administration results in an improvement of an intellectual disability. In certain embodiments, the administration results in an improvement of a learning disability. In certain embodiments, the administration results in an improvement of a cognitive impairment. In certain embodiments, the administration results in an improvement of dementia.

Also disclosed herein are methods of managing patients with mitochondrial dysfunction or mitochondrial disease and/or assessing the response of said patients to therapeutic intervention through the use of a composite endpoint that measures clinically significant improvements, comprising (i) a panel of multiple sub-instruments that measure relevant and/or prevalent symptoms affecting patients with mitochondrial disease; (ii) a baseline assessment of a patient using two or more of said sub-instruments; (iii) a subsequent assessment of a patient using the same sub-instruments; and (iv) an algorithm to compare the result of the subsequent assessment to the baseline assessment.

Also disclosed herein are methods of computing a composite measurement, wherein said methods comprise: (i) employing two or more sub-instruments to measure changes in one or more of said clinical symptoms over time, (ii) for each of said two or more sub-instruments, classifying the subject as (a) a responder or non-responder, (b) a member of a clinical category, or (c) a member of a metric range, based on the change in said one or more clinical symptoms as measured using said sub-instrument, and (iii) employing an algorithm to combine the measurements obtained from said two or more sub-instruments into a single composite measurement, wherein said algorithm comprises (a) separately assessing the change in each measurement obtained from said two or more sub-instruments prior to combining each measurement into a single composite measurement, or (b) combining measurements obtained from said two or more sub-instruments at a single time point and generating a single composite measurement for the single time point and then comparing the single composite measurement for the single time point to a single composite measurement for a different single time point.

In certain embodiments, said two or more measurement sub-instruments are selected from Motor Function Measure, Six Minute Walk Test, Two Minute Walk Test, Modified Fatigue Impact Scale, Friedreich's Ataxia Rating Scale, Patient-Reported Outcomes Measurement Information System Gastrointestinal Symptom Scales, 30-Second Chair Stand Test, Muscle Strength by Myometry Test, Newcastle Mitochondrial Disease Adult Scale, Newcastle Mitochondrial Disease Pediatric Scale, 36-Item Short Form Survey, Clinical Global Impression, Patient's Global Impression, Columbia Suicide Severity Rating Scale, Neuropathy Impairment Score, Migraine Disability Assessment Test, Quick Inventory of Depressive Symptomatology-Self-Report, Montreal Cognitive Assessment, NY Heart Association Functional Classification, Diabetes Health Profile, Ocular Motility Assessments, Marginal Reflex Distance, Ocular Motility & Fixation in 8 Gaze Directions, Degree of Ocular Saccades, Visual Function Test, Logarithm of Minimum Angle of Resolution, Pelli-Robson Score, Humphrey Visual Field mean deviation, Patient-Reported MitoPC Symptom Changes, or a combination thereof.

In certain embodiments, said two or more measurement sub-instruments are validated outcome measures for mitochondrial dysfunction or mitochondrial disease.

## EXAMPLES

The following is provided to further describe some of the embodiments disclosed herein. It is intended to illustrate, not to limit, the disclosed embodiments.

### Example 1. A Phase II Randomized, Double-Blind, Placebo-Controlled, Two-Period Crossover Study to Assess the Safety and Efficacy of Probucol in Adults with Genetically-Confirmed Mitochondrial Disease

A non-limiting example of a clinical trial evaluating the efficacy of probucol, or pharmaceutically acceptable salts thereof, in subjects with genetically-confirmed mitochondrial disease, is described below.

### A. Study Objectives

*Primary Objectives*

The primary objectives of this study are to evaluate, among other things, (i) the efficacy of probucol, or pharmaceutically acceptable salts thereof, as compared to placebo, by measuring the proportion of subjects that are responders over a 16-week treatment period when assessed by the Mitochondrial Disease Personalized Composite (MitoPC) endpoint, which is based on the most prevalent symptoms in patients with mitochondrial disease, and (ii) the safety of probucol, or pharmaceutically acceptable salts thereof, using adverse event (AE) rates, laboratory values, and electrocardiogram results. The selection of symptoms for inclusion in the MitoPC endpoint were based on a survey of mitochondrial disease symptoms in adult and pediatric mitochondrial disease patients. Zolkipli-Cunningham et al., PLoS One 2018; 13(5):e0197513.

*Secondary Objectives*

The key secondary objective of this study is to evaluate the efficacy of probucol, or pharmaceutically acceptable salts thereof, as compared to placebo over a 16-week treatment period as measured by the Six Minute Walk Test (6MWT). Other secondary objectives include evaluating the efficacy of probucol, or pharmaceutically acceptable salts thereof, compared to placebo over a 16-week treatment period. The following evaluations will be conducted for all subjects:

- Two Minute Walk Test (2MWT)
- Motor Function Measure - 32 items (MFM-32)
- Modified Fatigue Impact Scale (MFIS)
- Friedreich's Ataxia Rating Scale (FARS)
- Patient-Reported Outcome Measurement Information System - Gastrointestinal (PROMIS-GI)
- 30-Second Chair Stand Test (30CST)
- Muscle Strength by Myometry in 4 Groups (MSM-4)
- Newcastle Mitochondrial Disease Adult Scale (NMDAS)
- Quick Inventory of Depressive Symptomatology- Self Report (QIDS-SR)
- 36-Item Short Form Survey Physical Component Summary (SF-36 PCS)
- Clinical Global Impression (CGI)
- Patient's Global Impression (PGI)
- Patient-Reported Initial MitoPC Symptoms (PRIMS)
- Patient-Reported MitoPC Symptom Changes (PRMSC)
- Columbia Suicide Severity Rating Scale (CSSRS)

The following evaluations will be performed for all subjects for whom the result from the assessment in the first study visit (Visit 1) meets or exceeds the threshold value for qualification:

- Neuropathy Impairment Score - Lower Limb (NIS-LL)
- Migraine Disability Assessment Test (MIDAS)
- Montreal Cognitive Assessment (MoCA)
- New York Heart Association Functional Classification (NYHAFC)
- Diabetes Health Profile - 18 items (DHP-18)
- Ocular Motility Assessments (OMA) comprising:

    - Marginal Reflex Distance (MRD1) - a measure of ptosis;
    - Ocular Motility & Fixation in 8 Gaze Directions (OMF8) - an assessment of ocular motility and fixation
    - Degree of Ocular Saccades (DOS) - an assessment of the latency, precision and conjugacy of saccadic eye movements.

*Exploratory Objectives*

Exploratory objectives of this study include evaluating the safety and efficacy of probucol, or pharmaceutically acceptable salts thereof, compared to placebo over a 16-week treatment period as measured by:

- Laboratory biomarker measures (in both blood and urine)
- Days of school and/or work missed

- Days of hospitalization
- Suicidality assessment (Columbia Suicide Severity Rating Scale, CSSRS)
- Event-free survival
- Change in average velocity between first- and sixth-minute 6MWT periods
- Visual Function Test (VFT) comprising:
    - Logarithm of Minimum Angle of Resolution (LogMAR)
    - Pelli-Robson Score (PRS)
    - Visual Field Mean Deviation (VFMD).

**B. Eligibility**

*Inclusion Criteria (study open to all sexes, aged 18 to 75 years):*

- Subjects must be able to provide written informed consent for participation; and in the opinion of the investigator, be willing and able to fulfill the requirements of the study;
- Subjects with a definite primary pathogenic mitochondrial DNA mutation or a nuclear DNA defect that is associated with reduced function of the mitochondrial respiratory chain;
- Subjects with at least moderate or severe symptomatology of mitochondrial disease, as defined by a minimum score of 6 on the NMDAS;
- Subjects who meet or exceed the threshold score for at least one MitoPC sub-instrument;
- Female subjects with only negative pregnancy test results at the time of enrollment and throughout the entire study period; and
- Sexually active subjects must practice acceptable methods of contraception during the entire study period.

*Exclusion Criteria:*

- Subjects with any known hypersensitivity or adverse reaction to probucol;
- Subjects with any potential for QTc prolongation:
    - Subjects with a QTc interval greater than 450 msec;
    - Subjects with known mutations in genes that cause Long QT Syndrome (LQTS);
    - Subjects with known mutations in genes that cause hypertrophic cardiomyopathy sufficient to categorize the subject in class III or IV of the New York Heart Association Functional Classification (NYHAFC);
    - Subjects on concomitant medications that are reported by The Arizona Center for Education and Research on Therapeutics (AZCERT) to prolong the QT interval AND are clearly associated with a known risk of Torsades de Pointes (TdP), even when taken as recommended;
    - Subjects with risk factors for TdP, including clinically significant hypokalemia or bradycardia;
    - Subjects with severe ventricular arrhythmias (e.g., frequent episodes of multifocal ventricular extrasystole);
    - Subjects with atrial fibrillation (including paroxysmal atrial fibrillation);
    - Subjects with NYHAFC Class III or IV;
    - Subjects with congestive heart failure or unstable angina;
- Subjects who have an active fungal infection, acute blood, lung, or bladder infection, clinically significant hepatic or renal dysfunction, and/or viral infection (including human immunodeficiency virus or hepatitis virus B or C);
- Subjects who have clinically significant laboratory abnormalities, including but not limited to:
    - Elevated results greater than 2.5 times the upper limit of normal for gamma-glutamyl transferase;
    - Elevated results greater than 3 times the upper limit of normal for serum creatine; or
    - Estimated glomerular filtration rate (eGFR) less than 60 mL/min/1.73 $m^2$.
- Subjects with any medical condition or abnormal laboratory result that, in the opinion of the investigator, will interfere with the safe completion of the study;
- Subjects who are using any investigational products within one month prior to randomization;

- Subjects who are actively breastfeeding at the time of screening;
- Subjects who have known or suspected active alcohol and/or substance abuse; and
- Subjects who have a history of or current suicidal ideation, behavior, and/or attempts.

**C. Study Design**

*Overall Design*

The study is a randomized, double-blind, placebo-controlled, 2-period crossover study of probucol, or pharmaceutically acceptable salts thereof. The study will enroll subjects with genetically-confirmed mitochondrial disease who meet specific inclusion and exclusion criteria. The study design is depicted in FIG. 1.

Each participant will be in the study for approximately 61 to 65 weeks, which includes the following three phases: (1) Pre-treatment (up to 8 weeks); (2) Double-blind treatments (48 weeks); and (3) Post-treatment follow-up (9 weeks).

Active participation will last for approximately 15 months and will include up to 13 on-site visits to the clinical study center. If there are sudden safety concerns, additional on-site visits may be required.

Prior to enrollment in the study, potential subjects will complete a pre-screening assessment to determine potential study eligibility. The subject's screening visit (Visit 1) will be scheduled no later than 90 days after this pre-screening assessment. Up to 44 eligible subjects will be enrolled and randomized, with the goal of 30 evaluable subjects completing the clinical trial.

*Pre-Treatment (Screening) Phase*

Subjects who meet the eligibility criteria will be screened at Visit 1 and complete the pre-treatment phase. Subjects will complete a standardized intake of their active clinical symptoms. The investigator will discuss the disease manifestations of each subject so that they have a full understanding of the severity of their disease and relevance of their specific symptoms. Each subject will then: (1) identify the presence or absence of five specific symptoms on the MitoPC menu by completing the five MitoPC sub-instruments for all five specific symptoms, regardless of whether or not such symptoms are present; (2) indicate whether or not each symptom has a personal impact and whether or not the subject seeks its improvement (by completing the PRIMS assessment); and (3) rank the symptoms in order of importance to the subject and degree of negative impact on their life (by completing the PRIMS assessment).

In addition, subjects will also complete all assessments and tests for primary and secondary endpoints. A complete list of study assessments, symptoms assessed, threshold values, Minimal Clinically Important Difference (MCID or M) values, and corresponding objectives or endpoints is provided in Table 1.

**Table 1. Study Assessments**

| Instr. # | Test # | Code | Assessment | Symptom assessed | Threshold[1] | | MCID | For end point[2] |
|---|---|---|---|---|---|---|---|---|
| **A All subjects** | | | ***Compulsory in visits V1, V2, V5, V6, V7, V8, V11, V12 & V13 (regardless of whether or not V1 threshold is met).*** | | | | | |
| **1** | 1 | **6/2MWT[3]** | Six and Two Minute Walk Test | Exercise Intolerance | These thresholds are used only for *MitoPC* endpoint analysis[4] | Algorithm[5] | 40 meters[6] | 1° & 2° |
| **2** | 2 | **MFM-32'** | Motor FunctionMeasure - 32 items | Muscle weakness | | ≥ 89 points | 5 points | 1° & 2° |
| **3** | 3 | **MFIS[3]** | Modified Fatigue Impact Scale | Fatigue | | > 32 points | 10 points | 1° & 2° |
| **4** | 4 | **FARS[3]** | Friedreich's Ataxia Rating Scale | Balance difficulties | | ≥ 29 points | 5 points | 1° & 2° |
| **5** | 5 | **PROMIS-GI[3]** | Patient-Reported Outcomes Measurement Information System - Gastrointestinal Scales | Gastrointestinal symptoms | | ≥ 55 points | 6 points | 1° & 2° |
| **6** | 6 | **30CST** | 30-second Chair Stand Test | Lower extremity strength | | | 26 stands | 2° |
| **7** | 7 | **MSM-4** | Muscle Strength by Myometry - 4 groups | Muscle weakness | | | 19.5% | 2° |
| **8** | 8 | **NMDAS** | Newcastle Mitochondrial Disease Adult Scale | Mitochondrial disease | | | 7 points | 2° |
| **9** | 9 | **QIDS-SR** | Quick Inventory of Depressive Symptomatology - Self-Report | Depression symptoms | | | 5 points | 2° |
| **10** | 10 | **SF-36 PCS** | 36-Item Short Form Survey PCS | Quality of life | | | 7 points | 2° |
| **11** | 11 | **CGI** | Clinical Global Impression | Illness severity | | | 1 point | 2° |
| **12** | 12 | **PGI** | Patient's Global Impression | Illness severity | | | 1 point | 2° |
| **13** | 13 | **CSSRS** | Columbia Suicide Severity Rating Scale | Suicidality | | | | Safety |
| **B. All** | **subjects** | **during screening and baseline visits** | | ***Compulsory in visits V1, V2 and V8.*** | | | | |
| **14** | 14 | **PRIMS** | Patient-Reported Initial MitoPC Symptoms | MitoPC symptoms | | | | |

(continued)

| C. All | subjects | during selected subsequent visits | | Compulsory in visits V5, V6, V7, V11, V12 & V13. | | | |
|---|---|---|---|---|---|---|---|
| **15** | 15 | **PRMSC** | Patient-Reported MitoPC Symptom Changes | MitoPC symptom changes | | 1 pt/ symptom | 2° |
| **D. Qualified subjects only** | | ***Compulsory in visit V1. Contingent (upon meeting V1 threshold) in visits V2, V5, V6, V7, V8, V11, V12 & V13.*** | | | | | |
| **16** | 16 | **NIS-LL** | Neuropathy Impairment Score - Lower Limbs | Peripheral neuropathy | ≥ 2 | 3 points | 2° |
| **17** | 17 | **MIDAS** | Migraine Disability Assessment Test | Headache-related disability | > 10 | 5 points | 2° |
| **18** | 18 | **MoCA** | Montreal Cognitive Assessment | Cognitive symptoms | ≤ 23 | 7 points | 2° |
| **19** | 19 | **NYHAFC** | NYHeart Association Functional Classification | Heart failure | ≥ II | 1 class | 2° |
| **20** | 20 | **DHP-18** | Diabetes Health Profile - 18 Items | Impact of diabetes | ≥ 19 | 4 points | 2° |
| **21** | | **OMA**[7] | Ocular Motility Assessments | | | | |
| | 21 | **MRD1** | (Marginal Reflex Distance) | Ptosis | ≤ 4 mm | 2 mm | 2° |
| | 22 | **OMF8** | (Ocular Motility & Fixation in 8 Gaze Directions) | Eye muscle dysfunction | ≤ -1 | 1 point | 2° |
| | 23 | **DOS** | (Degree of Ocular Saccades) | Ocular myopathy | ≥ 25% | 1 grade | 2° |
| **22** | | **VFT**[8] | Visual Function Test | | | | |

(continued)

| D. Qualified subjects only | | | *Compulsory in visit V1. Contingent (upon meeting V1 threshold) in visits V2, V5, V6, V7, V8, V11, V12 & V13.* | | | | |
|---|---|---|---|---|---|---|---|
| | 24 | **LogMAR** | (Logarithm of Minimum Angle of Resolution) | Visual acuity | > 0.18 | 0.1 | Exp. |
| | 25 | **PRS** | (Pelli-Robson Score) | Contrast sensitivity | < 1.5 | 0.15 | Exp. |
| | 26 | **VFMD** | (Humphrey visual field mean deviation) | Visual field | ≤ -4.20 | 2.00 | Exp. |

1 Threshold values are used to qualify the subject in V1 for (i) MitoPC statistical analysis (Instr. #s 1-5), and (ii) contingent testing (Instr. #s 13-19).
2 1° = primary, 2° = secondary, Exp. = exploratory. The primary endpoint is the MitoPC composite of up to 5 assessments.
3 These MitoPC sub-instruments are administered to all subjects regardless of whether they meet the V1 threshold.
4 Meeting one of these V1 thresholds determines inclusion of the assessment in the MitoPC statistical analysis.
5 6MWT threshold is determined by gender, age, height and weight.
6 The stated MCID is for the Six Minute Walk Test.
7 The subject meets the OMA threshold in V1 if s/he meets or exceeds any of the sub-assessent thresholds (one or more of MRD 1, OMF8, DOS).
8 The subject meets the VFT threshold in V1 if s/he meets or exceeds any of the sub-assessent thresholds (one or more of LogMAR, PRS, VFMD).

During subsequent on-site visits, all MitoPC sub-instruments and most secondary assessments and tests will be administered. Some symptom-specific secondary tests will not be administered during subsequent visits if the subject did not report or demonstrate these symptoms during their standardized intake.

All screening procedures will be completed and reviewed to confirm eligibility and overall subject safety for the study. At least 30 days prior to any treatment period, subjects must be on a stable standard-of-care treatment regimen that is intended to remain unchanged throughout the entire duration of the study.

*Double-Blind Treatment Phase*

At Visit 2, subjects will be randomly assigned to one of two different treatment sequences in a 1:1 ratio:

(1) Treatment Sequence A (Probucol/Placebo): 500 mg probucol, or a pharmaceutically acceptable salt thereof, taken twice daily with food for 16 weeks during Treatment Period 1, followed by a 16-week washout period, and then placebo taken twice daily for 16 weeks during Treatment Period 2; and

(2) Treatment Sequence B (Placebo/Probucol): Placebo taken twice daily with food for 16 weeks during Treatment Period 1, followed by a 16-week washout period, and then 500 mg probucol, or a pharmaceutically acceptable salt thereof, taken twice daily for 16 weeks during Treatment Period 2.

During the treatment phase (Treatment Period 1, washout, Treatment Period 2), subjects will receive safety and efficacy evaluations approximately every 8 weeks. After completion of the double-blind treatment phase, subjects will begin the post-treatment phase, which includes on-site and/or telephone safety evaluations. A complete schedule of study activities is summarized in table 2.

**Table 2: Study Activities[1]**

| **Study Period** | **Sere en** | **Treatment Period 1** | | | | **Washout** | | **Treatment Period 2** | | | | **Follow up** | |
|---|---|---|---|---|---|---|---|---|---|---|---|---|---|
| **Visit** | **V1[2]** | **V2** | **V3** | **V4** | **V5** | **V6** | **V7** | **V8** | **V9** | **V10** | **V11** | **V12** | **V13** |
| **Study week** | **1** | **5** | **6** | **7** | **13** | **21** | **29** | **37** | **38** | **39** | **45** | **53** | **61** |
| **Duration** (days) | **2** | **2** | **1** | **1** | **1** | **1** | **1** | **2** | **1** | **1** | **1** | **1** | **1** |
| **Location[3]** | **C** | **C** | **H** | **H** | **C** | **C** | **C** | **C** | **H** | **H** | **C** | **C** | **C** |
| Informed consent | A | | | | | | | | | | | | |
| Medical history | A | | | | | | | | | | | | |
| Concomitant medication review | A | A | | | A | A | A | A | | | A | A | A |
| Physical examination | A | A | | | A | A | A | A | | | A | A | A |
| Vital signs | A | A | | | A | A | A | A | | | A | A | A |
| Height | A | | | | | | | | | | | | |
| Weight | A | A | | | A | A | A | A | | | A | A | A |
| Randomization | | A | | | | | | | | | | | |
| Study medication dispensed | | A | | | | | | A | | | | | |
| Assessments (all subjects) | A | A | | | A | A | A | A | | | A | A | A |
| Assessments (qualified subjects)[4] | A | Q | | | Q | Q | Q | Q | | | Q | Q | Q |
| Initial symptom assessment | A | A | | | | | | A | | | | | |
| Patient's change assessment | | | | | A | A | A | | | | A | A | A |
| Laboratory tests (all subjects)[5] | A | A | | | A | A | A | A | | | A | A | A |
| Pregnancy test (serum) | W | | | | | | | | | | | | |
| Pregnancy test (urine) | | W | | | W | W | W | W | | | W | W | W |

(continued)

| Study Period | Sere en | Treatment Period 1 | | | | Washout | | Treatment Period 2 | | | | Follow up | |
|---|---|---|---|---|---|---|---|---|---|---|---|---|---|
| PK blood draws[6] | | A | A | A | A | A | A | A | A | A | A | A | A |
| MSD blood draws[7] | | A | | | | A | | A | | | | A | |
| ECG[8] | A | A | A | A | A | A | A | A | A | A | A | A | A |
| Adverse event reviews | A | A | | | A | A | A | A | | | A | A | A |
| Complete case report forms | A | A | A | A | A | A | A | A | A | A | A | A | A |

[1]Subject codes: A = All subjects; Q = Qualified subjects (V1 assessment meets or exceeds threshold); W = women of childbearing potential.

[2]Visit V1 must be no more than 8 weeks before Visit V2.

[3] Location codes: C = CHOP; H = CHOP (the preferred location) or the Subject's Home (if approved by the PI).

[4] Administered to all subjects in V1; administered to qualified subjects in Visits V2, V5, V6, V7, V8, V11, V12 & V13.

[5] Laboratory tests: hemoglobin A1c, comprehensive metabolic panel (glucose, calcium, albumin, total protein, sodium, potassium, CO2, chloride, BUN, creatinine, ALP, ALT, AST and bilirubin), serum lipid panel (total cholesterol, HDL-C, LDL-C, triglycerides, VLDL-C, Non-HDL-C and cholesterol/HDL ratio), thyroid panel (TSH, Total T4, free T4 and reverse T3), CBC with differential (hemoglobin, hematocrit, RBC, WBC, WBC differential, platelet count and reticulocyte count), plasma amino acids (quantitative), plasma acylcarnitine profile, creatine kinase (total), serum pyruvate/lactate analysis, urinalysis and urine organic acids (quantitative).

[6] One PK draw per visit, except V2 & V8 (five draws per two-day visit: one draw on day 1; four draws on day 2).

[7] MSD blood draws refer to the collection of blood for immunoassay analysis in the P-S6RP and AMPK-$\alpha$1 Panels.

[8] One ECG per visit, except V2 and V8 (two ECGs per two-day visit: one on day 1; one on day 2).

The total amount of blood to be drawn from each subject throughout the entire study period is approximately 750 mL.

Due to the potential of probucol to prolong corrected QT interval (QTc), electrocardiograms (ECGs) will be conducted in triplicate at baseline and at each study visit throughout the three study phases. Serum and plasma drug levels will be collected at the same approximate time of ECGs to correlate with QTc measurements.

*Stopping Study Participation*

Each subject will be monitored for QTc prolongation during their entire participation and, if needed, they will be safely removed from the study if prolongation of QTc is more than 60 msec compared to baseline or an absolute QTc greater than 500 msec. Re-challenge with the study drug may be considered only if the QTc interval returns to within 10 msec of baseline and absolute QTc of less than or equal to 450 msec.

In addition, subjects will be closely monitored for any cardiovascular conditions or abnormalities, and if required, removed from the study and referred to a cardiologist for consult.

All adverse events (AEs), regardless of severity or grade, will be closely monitored and referred for clinical care if needed.

Any serious adverse event (SAE) or AE with severity of Grade 3 or worse according to Common Terminology Criteria for Adverse Events (CTCAE) classification will be immediately reviewed by the investigator and, if treatment is continued, must have the benefit-risk for continued treatment assessed and documented by the investigator.

*Post-Treatment Phase*

Subjects who complete the double-blind treatment phase will proceed with the post-treatment phase, which includes a minimum of two safety evaluations on-site and/or by telephone.

**D. Endpoints**

*Primary Endpoint*

The primary efficacy endpoint will be evaluated by comparing the proportion of "MitoPC Responders" in the

drug and placebo treatment arms for each 16-week treatment period of the crossover study.

The five MitoPC sub-instruments, and the associated symptoms assessed, are summarized in Table 3.

**Table 3: MitoPC**

| MitoPC: symptoms assessed and sub-instruments | | |
|---|---|---|
| **Symptom** | **Sub-Instrument** | **Code** |
| Exercise intolerance | 6-Minute Walk Test | 6MWT |
| Muscle weakness | Motor Function Measure (32 items) | MFM-32 |
| Fatigue | Modified Fatigue Impact Scale | MFIS |
| Balance problems | Friedreich's Ataxia Rating Scale | FARS |
| Gastrointestinal | Patient-Reported Outcomes Measurement Information | PROMIS-GI |
| symptoms | System - Gastrointestinal Symptom Scales | |

All MitoPC sub-instruments will be administered to all subjects at each visit, but only "qualified sub-instruments" (where the sub-instrument result in screening Visit 1 meets or exceeds the pre-defined threshold value) will be included in the MitoPC primary endpoint analysis.

Each subject will be classified as a "Sub-instrument Responder" or "Sub-instrument Non-responder" for each "qualified sub-instrument" during each treatment period, by comparing the measured change to an established Minimum Clinically Important Difference (MCID or M) for that sub-instrument (Table 4).

**Table 4: MitoPC Sub-Instrument Responder**

| (M = MCID) | **Change from treatment period baseline** | | |
|---|---|---|---|
| **Change** | ≤ -M | ≥ -M and < +M | ≥ +M |
| **Assessment** | Worse | Neutral | Better |
| **Definition** | Non-Responder | | Responder |

A "Sub-instrument Responder" is defined as subject who achieves an improvement from treatment period baseline that equals or exceeds the MCID for that sub-instrument (i.e., an assessment of "Better").

If a subject does not achieve an MCID improvement for that sub-instrument (i.e., an assessment of "Neutral or Worse"), then that subject is considered a "Sub-instrument Non-responder".

The subject will then be categorized as a "MitoPC Responder" or a "MitoPC Non-Responder." A "MitoPC Responder" is a subject who is a "Sub-instrument Responder" for at least one of the up to five "qualified sub-instruments" in that subject's personalized MitoPC, in the absence of a clinically meaningful deterioration in any of the other "qualified sub-instruments" (Table 5).

A "MitoPC Responder" is a subject who is not a "Sub-instrument Responder" for at least one of the up to five "qualified sub-instruments" in that subject's personalized MitoPC, or experiences a clinically meaningful deterioration in any of the other "qualified sub-instruments" (Table 5).

**Table 5: MitoPC Responder Definitions**

| MitoPC Sub-Instruments | | | | |
|---|---|---|---|---|
| **#** | **Symptom** | **Sub-Instrument** | **Assessment** | **Definition** |
| 1 | Exercise intolerance | Six-Minute Walk Test | Better | 6MWT Responder |
| | | | Neutral or Worse | 6MWT Non-Responder |
| 2 | Muscle weakness | Motor Function Measure | Better | MFM-32 Responder |
| | | | Neutral or Worse | MFM-32 Non-Responder |

(continued)

| MitoPC Sub-Instruments | | | | |
|---|---|---|---|---|
| # | Symptom | Sub-Instrument | Assessment | Definition |
| 3 | Fatigue | Modified Fatigue Impact Scale | Better | MFIS Responder |
| | | | Neutral or Worse | MFIS Non-Responder |
| 4 | Balance problems | Friedreich's Ataxia Rating Scale | Better | FARS Responder |
| | | | Neutral or Worse | FARS Non-Responder |
| 5 | GI problems | Patient-Reported Outcomes Measurement Information System - Gastrointestinal Symptoms | Better | PROMIS-GI Responder |
| | | | Neutral or Worse | PROMIS-GI Non-Responder |
| **MitoPC Responder** | | Responder on *any* of the "qualified sub-instruments", with no assessment of Worse for any of the "qualified sub-instruments" | | |
| **MitoPC Non-Responder** | | Non-Responder on *all* of the "qualified sub-instruments", or assessment of Worse for *any* of the "qualified sub-instruments" | | |

The primary efficacy endpoint will be evaluated by comparing the proportion of "MitoPC Responders" in the drug and placebo treatment arms for each 16-week treatment period of the crossover study.

MitoPC Sub-Instrument Threshold Values. For each sub-instrument, the threshold value is defined as the score that corresponds to a "mild" disease or symptom severity rating in literature, or the clinical equivalent thereof. These values were determined by a literature review and evaluation by a committee of mitochondrial disease clinicians and experts. Brennan et al., Am. J. Gastroenterol. 2014; 109:1804-1814. Flachenecker et al., Multiple Sclerosis 2002; 8:523-526. Subramony et al., Neurology 2005; 64:1261-1262. Berard et al., Neuromuscular Disorders 2005; 15:463-470. Enright and Scherrill, Am. J. Respir. Crit. Core Med. 1998; 158:1384-1387.

These threshold values are employed for patient quaificaton and statistical analysis of the MitoPC endpoint, and are not necessarily considered in the clinical administration of the subsequent sub-instrument tests.

Scores or values below threshold indicate values that are typical of asymptomatic individuals and the general population. The derived threshold values for each sub-instrument are stated in Table 6.

**Table 6: MitoPC Sub-Instrument Threshold Values**

| | Sub-Instrument | Threshold Value | | |
|---|---|---|---|---|
| 1 | 6MWT | Less than | (algorithm) | Meters |
| 2 | MFM-32 | Greater than or equal to | 89 | Points |
| 3 | MFIS | Greater than or equal to | 39 | Points |
| 4 | FARS | Greater than or equal to | 29 | Points |
| 5 | PROMIS-GI | Greater than or equal to | 55 | T-score Points |

*Key and Other Secondary Endpoints*

The key secondary endpoint for this study is the change in the 6MWT score from treatment period baseline. The other secondary endpoints are the changes from treatment period baseline in the following endpoints:

- The proportion of MitoPC "Sub-instrument Responders" over 8-week and 16-week treatment periods for each of the following: 6MWT; MFM-32; MFIS; FARS and PROMIS-GI.
- Mito PC sub-instrument score change (except 6MWT) from treatment baseline to Week 8 and Week 16.

- Change from treatment period baseline for each of the following MitoPC sub-instruments: 6MWT; MFM-32; MFIS; FARS and PROMIS-GI.
- Change from treatment period baseline for each of the following additional efficacy endpoints: 30CST; NMDAS; QIDS-SR; SF-36 PCS; CGI; PGIC; NIS-LL; MIDAS; MoCA; NYHAFC; DHP-18; MRD1; OMF-8 and DOS.

*Exploratory Endpoints*

In addition, exploratory objectives to evaluate the safety and efficacy of probucol, or pharmaceutically acceptable salts thereof, compared to placebo over a 16-week treatment period are included, as measured by the exploratory objectives listed above.

**E. Statistics and Analytics**

Data will be collected and processed according to the Standard Operating Procedures of the Sponsor's Clinical Research Organization, which are based on the principles of Good Clinical Practices.

*Statistical Methods for Evaluation of the Primary Assessment*

MitoPC Statistical Methods: the primary assessment is the comparison of treatment with Active (probucol, or pharmaceutically acceptable salts thereof) versus Placebo in the proportion of "MitoPC Responders" as previously defined.
Specifically, if:

$P_{Active}$ = Probability (MitoPC Responder, Active)
$P_{Placebo}$ = Probability (MitoPC Responder, Placebo)

then the primary hypothesis test of interest is:

$$H_0: P_{Active} = P_{Placebo}$$

$$H_A: P_{Active} \neq P_{Placebo}.$$

The "MitoPC Responder" definition allows the results of the study to be reduced to a dichotomous outcome: Responder vs. Non-Responder. These dichotomous outcomes can be summarized in the following contingency table (Table 7).

**Table 7: Contingency Table**

| | | Active Treatment | | |
|---|---|---|---|---|
| | | *Responder* | *Non-Responder* | |
| **Placebo Treatment** | *Responder* | **a** | **b** | **a + b** |
| | *Non-Responder* | **c** | **d** | **c + d** |
| | | **a + c** | **b + d** | **a + b + c + d = n** |

In the placebo-controlled randomized double-blinded crossover study of probucol, or pharmaceutically acceptable salts thereof, the proportion of subjects given Active treatment that are "MitoPC Responders" is (a + c) / n. Similarly, the proportion of subjects given Placebo treatment that are "MitoPC Responders" is (a + b) / n. Since a is common to both proportions, the b and c discordant pairs are critical to the test of the primary hypothesis.

The McNemar's test will be used to test the null hypothesis of whether the probability of a subject being a "MitoPC Responder" after treatment with probucol, or pharmaceutically acceptable salts thereof, is the same as the probability of being a "MitoPC Responder" after treatment with placebo. Specifically, the McNemar's exact binomial test will be used for these calculations, since the expected number of discordant pairs may be relatively small (< 25).

Sample Size: The sample size calculation for McNemar's exact test is based on the expected proportions of subjects allocated into the discordant pair categories described above (b/n and c/n). Assuming that 40% of the subjects

will be classified as an "Active Responder/Placebo Non-Responder", and 5% of the subjects will be classified as an "Active Non-Responder/Placebo Responder", then at least 30 evaluable subjects need to complete the study in order to ensure at least 85% power for testing the null hypothesis, given a Type I error rate of 0.05.

Assuming an attrition rate of no more than 32%, 44 subjects will need to be enrolled/randomized for a minimum of 30 evaluable subjects (i.e., subjects that complete the entire study).

Analysis Populations: All analyses will be performed for at least one of the following analysis populations. Details will be described in the SAP.

- Intent-to-Treat (ITT) Population: All subjects who receive at least one dose of the study drug and have at least one post-baseline efficacy data point will be included in the ITT population. Subject data will be included with the treatment allocation to which they were randomized.
- Per-Protocol (PP) Population: Subjects included in the ITT population who have no protocol deviations or missing data that are considered meaningful to the interpretation of the endpoint will be included in the PP population.
- Safety Population: All subjects who receive at least one dose of the study drug (including placebo) will be included in the safety evaluations.
- The frequencies of AEs by type, body system, severity, and relationship to study drug will be summarized. SAEs, if any, will be described in detail. AE incidence will be summarized with the corresponding exact binomial 95% two-sided confidence intervals.

*Statistical Methods for Evaluation of Key and Other Secondary Endpoints*

For the evaluation of the 6MWT, change from treatment period baseline will be calculated at 16 weeks. Probucol, or pharmaceutically acceptable salts thereof, will be compared to placebo using a linear mixed model including terms for sequence, treatment, and period as fixed effects and subjects nested within sequence as a random effect. Treatment period baseline 6MWT score will also be included in the model, but will be removed if not statistically significant at the 0.05 level. The model will be evaluated for normality and heterogeneity of variance assumptions and nonparametric methods will be considered, if appropriate.

Descriptive statistics (N, mean, standard deviation, minimum, median, maximum) will be presented for the 6MWT scores and other secondary endpoints at each time-point and for change from treatment period baseline.

*Statistical Methods for Evaluation of Exploratory Endpoints*

The proportion of MitoPC "Sub-instrument Responders" at 16 weeks will be determined and summarized for each Sub-instrument.

Responders and Non-responders will be summarized for each MitoPC sub-instrument. The proportions of "Sub-instrument Responders" in the probucol and placebo groups will be compared using McNemar’s test.

Change from treatment period baseline will be calculated for each of the MitoPC sub-instrument scores as well as the additional efficacy and exploratory endpoints. Descriptive statistics will be calculated for scores at each time-point as well as for change from treatment period baseline. Probucol, or pharmaceutically acceptable salts thereof, will be compared to placebo with respect to change from treatment period baseline scores using the same statistical methods described for the 6MWT.

Subject disposition and demographics will be summarized using frequency counts and percentages and descriptive statistics. AEs will be coded and summarized according to the number of subjects experiencing AEs and the number of AEs by treatment. Clinical laboratory results, vital signs, and ECGs will be summarized by time-point using descriptive statistics. QTc values will be calculated and summarized by treatment and will be correlated graphically with time-matched PK results.

### F. Pharmacokinetic Analysis

Blood samples for the determination of serum or plasma drug levels will be collected at the same approximate time as ECGs to assess any correlation with QTc measurements. The PK samples will allow for a population PK analysis, as well as an analysis of the correlation between PK results and any observed QTc prolongation.

### G. Safety Evaluations

Safety assessments will include the incidence, frequency, and severity of AEs and serious AEs (SAEs), including clinically significant changes from baseline to scheduled time points for: physical examination findings; vital signs; clinical laboratory results; ECG readings; and suicidality assessments.

Those skilled in the art will appreciate that numerous changes and modifications can be made to the preferred embodiments of the invention and that such changes and modifications can be made without departing from the spirit of the invention. It is, therefore, intended that the appended claims cover all such equivalent variations as fall within the true spirit and scope of the invention

**Table 8: Classes of genes associated with mitochondrial dysfunction**

| Class | Class description | Gene function | Examples of genes | Examples of associated diseases |
|---|---|---|---|---|
| **1** | **Genes in mitochondrial DNA** | | | |
| 1.1 | Respiratory chain protein genes | Complex I | MT-ND1, MT-ND2, MT-ND3, MT-ND4, MT-ND5, MT-ND6, MT-ND4L | LHON, Leigh syndrome |
| | | Complex III | MT-CYB | |
| | | Complex IV | MT-CO1, MT-CO2, MT-CO3 | |
| | | Complex V | MT-ATP6, MT-ATP8/6, MT-ATP8 | |
| 1.2 | Mitochondrial transfer RNA genes | MT-tRNAs | MT-TL1, MT-TK | MELAS, MERRF |
| 1.3 | Mitochondrial ribosomal RNA genes | 12SrRNA | MT-RNR1 | non-syndromic sensorineural hearing loss |
| | | 16S rRNA | MT-RNR2 | |
| **2** | **Genes in nuclear DNA** | | | |
| 2.1 | Respiratory chain protein genes | Complex I | ACAD9, FOXRED1, TIMMDC1, TMEM126B, NDUFA1, NDUFB11 | Leigh syndrome, cardiomyopathy, neurologic problems, renal failure |
| | | Complex II | SDHA, SDHB, SDHC, SDHD, SDHAF1, SDHAF2 | Leigh syndrome, leukoencephalopathy |
| | | Complex III | UQCRB, UQCRQ, UQCRC2, CYC1, BCS1L, LYRM7, UQCC2, UQCC3 | Hypoglycemia, hyperglycemia, hepatomegaly, Leigh syndrome, neurologic abnormalities, renal tubular acidosis, episodic metabolic decompensation |
| | | Complex IV | SURF1, SCO1, SCO2 | Leigh syndrome, cardiomyopathy |
| | | Complex V | ATP5A1, ATP5E, USMG5, TMEM70, ATPAF2 | |
| 2.2 | mtDNA genome maintenance genes | Polymerase gamma | POLG | CPEO, Leigh syndrome, Alpers-Huttenlocher syndrome |
| | | Twinkle helicase | TWNK | CPEO, spinocerebellar ataxia, epilepsy |
| | | Nucleotide conversion | TK2, DGUOK, SUCLG1, SUCLA2, ABAT, TYMP, RRM2B | MNGIE |
| | | Nucleotide importation | SLC25A4, AGK, MPV17 | |

(continued)

| 2 | Genes in nuclear DNA | | | |
|---|---|---|---|---|
| 2.3 | mtDNA translation genes | Mitochondrial transcription factor | TFAM | Liver failure |
| | | Mitochondrial aminoacyl-tRNA synthetases | DARS2, RARS2, EARS2, MARS2, FARS2, YARS2, SARS2, AARS2, HARS2 | Neurologic disease, liver disease, failure to thrive |
| | | Mitochondrial ribosomal subunits | MRPS, MRPL | Primary mitochondrial disease |
| | | Translation factors | TACO1, GFM1, C12ORF65 | Multiple RC deficiencies, neurologic disease, vision loss, liver problems |
| 2.4 | Mitochondrial fission and fusion genes | Mitochondrial membrane fusion regulation | OPA1, MFN1, MFN2 | Peripheral neuropathy, optic neuropathy |
| | | Mitochondrial fission regulation | DNM1L, MIEF2 | |

**Table 9: Mitochondrial Diseases and Associated Genes**

| Disease ID | | | | Mitochondrial disease (description) | Associated with genes |
|---|---|---|---|---|---|
| # | MSeqDR | OMIM | Abbrev. | | |
| 1 | 00434 | 201450 | ACADMD | ACYL-CoA DEHYDROGENASE, MEDIUM-CHAIN, DEFICIENCY OF; ACADMD | ACADM |
| 2 | 00435 | 201470 | ACADSD | ACYL-CoA DEHYDROGENASE, SHORT-CHAIN, DEFICIENCY OF; ACADSD | ACADS |
| 3 | 00436 | 201475 | ACADVLD | ACYL-CoA DEHYDROGENASE, VERY LONG-CHAIN, DEFICIENCY OF; ACADVLD | ACADVL |
| 4 | 00084 | 262000 | BJS | Bjornstad syndrome | BCS1L |
| 5 | 00399 | 302060 | BTHS | BARTH SYNDROME; BTHS | TAZ |
| 6 | 00437 | 212138 | CACTD | CARNITINE-ACYLCARNITINE TRANSLOCASE DEFICIENCY;CACTD | SLC25A20 |
| 7 | 00444 | 612718 | CCDS3 | CEREBRAL CREATINE DEFICIENCY SYNDROME 3; CCDS3 | 3ATM |
| 8 | 00473 | 212140 | CDSP | CARNITINE DEFICIENCY, SYSTEMIC PRIMARY; CDSP | SLC22A5 |
| 9 | 00482 | 604377 | CEMCOX1 | CARDIOENCEPHALOMYOPATHY, FATAL INFANTILE, DUE TO CYTOCHROME c OXIDASE DEFICIENCY 1; CEMCOX1 | SCO2 |
| 10 | 00086 | 613642 | CMD1GG | Cardiomyopathy, dilated, 1GG | SDHA |
| 11 | 00488 | 609260 | CMT2A2 | CHARCOT-MARIE-TOOTH DISEASE, AXONAL, TYPE 2A2; CMT2A2 | MFN2 |

(continued)

| Disease ID | | | | Mitochondrial disease (description) | Associated with genes |
|---|---|---|---|---|---|
| # | MSeqDR | OMIM | Abbrev. | | |
| 12 | 00090 | 607426 | COQ10D1 | Coenzyme Q10 deficiency, primary, 1 | COQ2 |
| 13 | 00446 | 614651 | COQ10D2 | COENZYME Q10 DEFICIENCY, PRIMARY, 2; COQ10D2 | PDSS1 |
| 14 | 00447 | 614652 | COQ10D3 | COENZYME Q10 DEFICIENCY, PRIMARY, 3; COQ10D3 | PDSS2 |
| 15 | 00443 | 612016 | COQ10D4 | COENZYME Q10 DEFICIENCY, PRIMARY, 4; COQ10D4 | ADCK3<br>COQ8A |
| 16 | 00448 | 614654 | COQ10D5 | COENZYME Q10 DEFICIENCY, PRIMARY, 5; COQ10D5 | COQ9 |
| 17 | 00480 | 614650 | COQ10D6 | COENZYME Q10 DEFICIENCY, PRIMARY, 6; COQ10D6 | COQ6 |
| 18 | 00639 | 616276 | COQ10D7 | COENZYME Q10 DEFICIENCY, PRIMARY, 7; COQ10D7 | COQ4 |
| 19 | 00640 | 616733 | COQ10D8 | COENZYME Q10 DEFICIENCY, PRIMARY, 8; COQ10D8 | COQ7 |
| 20 | 00092 | 609060 | COXPD1 | Combined oxidative phosphorylation deficiency 1 | GFM1 |
| 21 | 00093 | 614702 | COXPD10 | Combined oxidative phosphorylation deficiency 10 | MT01 |
| 22 | 00423 | 614922 | COXPD11 | COMBINED OXIDATIVE PHOSPHORYLATION DEFICIENCY 11; COXPD11 | RMND1 |
| 23 | 00094 | 614946 | COXPD14 | Combined oxidative phosphorylation deficiency 14 | FARS2 |
| 24 | 00095 | 614947 | COXPD15 | Combined oxidative phosphorylation deficiency 15 | MTFMT |
| 25 | 00080 | 615395 | COXPD16 | Combined oxidative phosphorylation deficiency 16 | MRPL44 |
| 26 | 00096 | 610498 | COXPD2 | Combined oxidative phosphorylation deficiency 2 | MRPS16 |
| 27 | 00097 | 610505 | COXPD3 | Combined oxidative phosphorylation deficiency 3 | TSFM |
| 28 | 00098 | 610678 | COXPD4 | Combined oxidative phosphorylation deficiency 4 | TUFM |
| 29 | 00099 | 611719 | COXPD5 | Combined oxidative phosphorylation deficiency 5 | MRPS22 |
| 30 | 00411 | 300816 | COXPD6 | COMBINED OXIDATIVE PHOSPHORYLATION DEFICIENCY 6; COXPD6 | AIFM1 |
| 31 | 00493 | 613559 | COXPD7 | COMBINED OXIDATIVE PHOSPHORYLATION DEFICIENCY 7; COXPD7 | C12orf65 |
| 32 | 00076 | 614096 | COXPD8 | COMBINED OXIDATIVE PHOSPHORYLATION DEFICIENCY 8; COXPD8 | AARS2 |
| 33 | 00100 | 614582 | COXPD9 | Combined oxidative phosphorylation deficiency 9 | MRPL3 |
| 34 | 00400 | 251900 | CPEO,CPEO Plus | MITOCHONDRIAL MYOPATHY: CPEO, CPEO "Plus" DOID:12558, Chronic progressive external ophthalmoplegia | MT-TA<br>MT-TL2<br>MT-TM<br>MT-TW |
| 35 | 00466 | 600649 | CPT II | CARNITINE PALMITOYLTRANSFERASE II DEFICIENCY, INFANTILE;CPT2 | CPT2 |
| 36 | 00088 | 603471 | CTLN2 | Citrullinemia, adult-onset type II | SLC25A13 |
| 37 | 00150 | 500007 | CVS | CYCLIC VOMITING SYNDROME; CVS | MT-TL1 |

(continued)

| Disease ID | | | | Mitochondrial disease (description) | Associated with genes |
|---|---|---|---|---|---|
| # | MSeqDR | OMIM | Abbrev. | | |
| 38 | 00091 | 615182 | D2L2AD | Combined D-2- and L-2-hydroxyglutaric aciduria | SLC25A1 |
| 39 | 00489 | 220290 | DFNB1A | DEAFNESS, AUTOSOMAL RECESSIVE 1A; DFNB1A | 3JB2<br>3JB3<br>3JB6 |
| 40 | 00573 | 602473 | EE 602473 | ENCEPHALOPATHY, ETHYLMALONIC; EE | ETHE1 |
| 41 | 00103 | 609304 | EIEE3 | Epileptic encephalopathy, early infantile, 3 | SLC25A22 |
| 42 | 00491 | 229300 | FRDA;FRDA 1;FA | FRIEDREICH ATAXIA 1; FRDA;FRIEDREICH ATAXIA WITH RETAINED REFLEXES, INCLUDED; FARR, INCLUDED | FXN |
| 43 | 00499 | 605899 | GCE | GLYCINE ENCEPHALOPATHY; GCE | AMT<br>GCSH<br>GLDC |
| 44 | 00467 | 231530 | HADH | 3-@HYDROXYACYL-CoA DEHYDROGENASE DEFICIENCY; HADH | HADH |
| 45 | 00421 | 612233 | HLD4 | LEUKODYSTROPHY, HYPOMYELINATING, 4; HLD4 | HSPD1 |
| 46 | 00476 | 255125 | HML | MYOPATHY WITH LACTIC ACIDOSIS, HEREDITARY; HML | ISCU |
| 47 | 00485 | 601152 | HSMN6 | NEUROPATHY, HEREDITARY MOTOR AND SENSORY, TYPE VI; HSMN6 | MFN2 |
| 48 | 00108 | 614559 | ICRD | Infantile cerebellar-retinal degeneration | ACO2 |
| 49 | 00143 | 530000 | KSS | KEARNS-SAYRE SYNDROME, caused by various mitochondrial deletions | MT-TL1 |
| 50 | 00474 | 609016 | LCHAD | LONG-CHAIN 3-HYDROXYACYL-CoA DEHYDROGENASE DEFICIENCY | HADHA |
| 51 | 00149 | 500001 | LDYT | LEBER OPTIC ATROPHY AND DYSTONIA, alt: LEBER HEREDITARY OPTIC NEUROPATHY WITH DYSTONIA; LDYT;; DYSTONIA, FAMILIAL, WITH VISUAL FAILURE AND STRIATAL LUCENCIES;; MARSDEN SYNDROME | MT-ND1<br>MT-ND3<br>MT-ND4<br>MT-ND6 |
| 52 | 00110 | 613070 | LFIT | Liver failure, transient infantile | TRMU<br>TRNT1 |
| 53 | 00072 | 535000 | LHON | LEBER OPTIC ATROPHY, LEBER HEREDITARY OPTIC NEUROPATHY; DOID:705 | MT-ATP6<br>MT-CO3<br>MT-CYB<br>MT-ND1<br>MT-ND2<br>MT-ND4<br>MT-ND4L<br>MT-ND5<br>MT-ND6 |
| 54 | 00172 | 551000 | LIMM | MITOCHONDRIAL MYOPATHY, LETHAL, INFANTILE | - |

(continued)

| Disease ID | | | | Mitochondrial disease (description) | Associated with genes |
|---|---|---|---|---|---|
| # | MSeqDR | OMIM | Abbrev. | | |
| 55 | 00638 | 615889 | LKENP | LEUKOENCEPHALOPATHY, PROGRESSIVE, WITH OVARIAN FAILURE; LKENP | AARS2 |
| 56 | 00492 | 308905 | LOAS | LEBER OPTIC ATROPHY, SUSCEPTIBILITY TO; LHON, MODIFIER OF;LOAS | - |
| 57 | 00015 | 256000 | LS | Leigh syndrome due to mitochondrial complex 1 deficiency | MT-ATP6<br>MT-TK<br>MT-TV<br>MT-TW<br>BCS1L<br>COX10<br>COX15<br>FOXRED1<br>NDUFA10<br>NDUFA12<br>NDUFA2<br>NDUFA9<br>NDUFAF2<br>NDUFAF6<br>NDUFS3<br>NDUFS4<br>NDUFS7<br>NDUFS8<br>SDHA<br>SURF1 |
| 58 | 00389 | 220111 | LSFC | Leigh syndrome, French-Canadian type | LRPPRC |
| 59 | 00406 | 238800 | LUFT | HYPERMETABOLISM DUE TO DEFECT IN MITOCHONDRIA;LUFT DISEASE | - |
| 60 | 00439 | 231680 | MADD | MULTIPLE ACYL-CoA DEHYDROGENASE DEFICIENCY; MADD | ETFA<br>ETFB<br>ETFDH |
| 61 | 00574 | 618233 | MC1DN10 | MITOCHONDRIAL COMPLEX I DEFICIENCY, NUCLEAR TYPE 10; MC1DN10 | NDUFAF2 |
| 62 | 00017 | 124000 | MC3DN1 | Mitochondrial complex III deficiency, nuclear type 1 | BCS1L<br>UQCRB |
| 63 | 00018 | 615157 | MC3DN2 | Mitochondrial complex III deficiency, nuclear type 2 | TTC19 |
| 64 | 00019 | 615158 | MC3DN3 | Mitochondrial complex III deficiency, nuclear type 3 | UQCRB |
| 65 | 00020 | 615159 | MC3DN4 | Mitochondrial complex III deficiency, nuclear type 4 | UQCRQ |
| 66 | 00021 | 615160 | MC3DN5 | Mitochondrial complex III deficiency, nuclear type 5 | UQCRC2 |
| 67 | 00022 | 615453 | MC3DN6 | Mitochondrial complex III deficiency, nuclear type 6 | CYC1 |
| 68 | 00112 | 607196 | MCPHA | Microcephaly, Amish type | SLC25A19 |
| 69 | 00415 | 602541 | MDCMC | MUSCULAR DYSTROPHY, CONGENITAL, MEGACONIAL TYPE; MDCMC | CHKB |

(continued)

| Disease ID | | | | Mitochondrial disease (description) | Associated with genes |
|---|---|---|---|---|---|
| # | MSeqDR | OMIM | Abbrev. | | |
| 70 | 00484 | 614739 | MEGDEL | 3-METHYLGLUTACONIC ACIDURIA WITH DEAFNESS, ENCEPHALOPATHY, AND LEIGH-LIKE SYNDROME; MEGDEL | SERAC1 |
| 71 | 00163 | 540000 | MELAS SYNDROME | MITOCHONDRIAL MYOPATHY, ENCEPHALOPATHY, LACTIC ACIDOSIS, AND STROKE- LIKE EPISODES;DOID:3687 | MT-ND1<br>MT-ND4<br>MT-ND5<br>MT-ND6<br>MT-TC<br>MT-TF<br>MT-TH<br>MT-TK<br>MT-TL1<br>MT-TQ<br>MT-TS1<br>MT-TS2 |
| 72 | 00162 | 545000 | MERRF | MYOCLONIC EPILEPSY ASSOCIATED WITH RAGGED-RED FIBERS; MERRF SYNDROME | MT-ND5<br>MT-TF<br>MT-TH<br>MT-TK<br>MT-TL1<br>MT-TS1<br>MT-TS2 |
| 73 | 00495 | 250950 | MGCA1 | 3-METHYLGLUTACONIC ACIDURIA, TYPE I; MGCA1 | AUH |
| 74 | 00496 | 258501 | MGCA3 | 3-METHYLGLUTACONIC ACIDURIA, TYPE III; MGCA3 | OPA3 |
| 75 | 00079 | 610198 | MGCA5 | 3-METHYLGLUTACONIC ACIDURIA, TYPE V; MGCA5 | DNAJC19 |
| 76 | 00165 | 520000 | MIDD | DIABETES AND DEAFNESS, MATERNALLY INHERITED;MIDD | MT-TE<br>MT-TK<br>MT-TL1 |
| 77 | 00039 | 600462 | MLASA1 | Mitochondrial myopathy and sideroblastic anemia 1 | PUS1 |
| 78 | 00472 | 613561 | M LASA2 | MYOPATHY, LACTIC ACIDOSIS, AND SIDEROBLASTIC ANEMIA 2; MLASA2 | YARS2 |
| 79 | 00572 | 500011 | M LASA3 | 500011 MYOPATHY, LACTIC ACIDOSIS, AND SIDEROBLASTIC ANEMIA 3; | MT-ATP6 |
| 80 | 00043 | 605711 | MMDS1 | Multiple mitochondrial dysfunctions syndrome 1 | NFU1 |
| 81 | 00044 | 614299 | MMDS2 | Multiple mitochondrial dysfunctions syndrome 2 | BOLA3 |
| 82 | 00013 | 615330 | MMDS3 | Multiple mitochondrial dysfunctions syndrome 3 | IBA57 |
| 83 | 00158 | 500009 | MMIT | MITOCHONDRIAL MYOPATHY, INFANTILE, TRANSIENT;MMIT | MT-TE |
| 84 | 00041 | 614741 | MPYCD | Mitochondrial pyruvate carrier deficiency | MPC1 |
| 85 | 00494 | 151800 | MSL | LIPOMATOSIS, MULTIPLE SYMMETRIC; MSL | MFN2 |

(continued)

| Disease ID | | | | Mitochondrial disease (description) | Associated with genes |
|---|---|---|---|---|---|
| # | MSeqDR | OMIM | Abbrev. | | |
| 86 | 00141 | 516060 | MTATP6 | ATP SYNTHASE 6;;MITOCHONDRIAL COMPLEX V (ATP SYNTHASE) DEFICIENCY, MITOCHONDRIAL TYPE 1, INCLUDED | MT-ATP6 |
| 87 | 00465 | 516020 | MTCYB | CYTOCHROME b OF COMPLEX III; MTCYB | MT-CYB |
| 88 | 00026 | 603041 | MTDPS1 | Mitochondrial DNA depletion syndrome 1 (MNGIE type) | MT-TK<br>POLG<br>TYMP |
| 89 | 0027 | 615084 | MTDPS11 | Mitochondrial DNA depletion syndrome 11 | MGME1 |
| 90 | 00028 | 615418 | MTDPS12 | Mitochondrial DNA depletion syndrome 12 (cardiomyopathic type) | SLC25A4 |
| 91 | 00029 | 615471 | MTDPS13 | Mitochondrial DNA depletion syndrome 13 (encephalomyopathic | FBXL4 |
| 92 | 00030 | 609560 | MTDPS2 | Mitochondrial DNA depletion syndrome 2 (myopathic type) | TK2 |
| 93 | 00031 | 251880 | MTDPS3 | Mitochondrial DNA depletion syndrome 3 (hepatocerebral type) | C10orf2<br>DGUOK |
| 94 | 00032 | 203700 | MTDPS4A | Mitochondrial DNA depletion syndrome 4A (Alpers type) | POLG |
| 95 | 00033 | 613662 | MTDPS4B | Mitochondrial DNA depletion syndrome 4B (MNGIE type) | POLG |
| 96 | 00035 | 256810 | MTDPS6 | Mitochondrial DNA depletion syndrome 6 (hepatocerebral type) | MPV17 |
| 97 | 00036 | 271245 | MTDPS7 | Mitochondrial DNA depletion syndrome 7 (hepatocerebral type) | C10orf2<br>TWNK |
| 98 | 00113 | 304700 | MTS | Mohr-Tranebjaerg syndrome | TIMM8A |
| 99 | 00168 | 551500 | NARP | NEUROPATHY, ATAXIA, AND RETINITIS PIGMENTOSA;NARP SYNDROME | MT-ATP6 |
| 100 | 00422 | 614298 | NBIA4 | NEURODEGENERATION WITH BRAIN IRON ACCUMULATION 4; NBIA4 | C19orf12 |
| 101 | 00392 | 101000 | NF2 | Neurofibromatosis, type 2 | NF2 |

(continued)

| Disease ID | | | | Mitochondrial disease (description) | Associated with genes |
|---|---|---|---|---|---|
| # | MSeqDR | OMIM | Abbrev. | | |
| 102 | 00102 | 125853 | NIDDM | Diabetes mellitus, noninsulin-dependent | ABCC8<br>AKT2<br>CAPN10<br>CDKAL1<br>ENPP1<br>GCGR<br>GCK<br>GPD2<br>HMGA1<br>HNF1A<br>HNF1B<br>HNF4A<br>IGF2BP2<br>IL6<br>INSR<br>IRS1<br>IRS2<br>KCNJ11<br>LIPC<br>MAPK8IP1<br>NEUROD1<br>PAX4<br>PDX1<br>PPARG<br>PPP1R3A<br>SLC2A2<br>SLC2A4<br>SLC30A8<br>TDF7L2<br>WFS1 |
| 103 | 00073 | 165500 | OPA1 | OPTIC ATROPHY 1; OPA1 | OPA1 |
| 104 | 00420 | 611523 | PCH6 | PONTOCEREBELLAR HYPOPLASIA, TYPE 6; PCH6 | RARS2 |
| 105 | 00442 | 312170 | PDHAD | PYRUVATE DEHYDROGENASE E1-ALPHA DEFICIENCY; PDHAD | PDHA1 |
| 106 | 00478 | 614111 | PDHBD | PYRUVATE DEHYDROGENASE E1-BETA DEFICIENCY; PDHBD | PDHB |
| 107 | 00475 | 245348 | PDHDD | PYRUVATE DEHYDROGENASE E2 DEFICIENCY; PDHDD | DLAT |
| 108 | 00479 | 614462 | PDHLD | PYRUVATE DEHYDROGENASE LIPOIC ACID SYNTHETASE DEFICIENCY; PDHLD | LIAS |
| 109 | 00477 | 608782 | PDHPD | PYRUVATE DEHYDROGENASE PHOSPHATASE DEFICIENCY; PDHPD | PDP1 |
| 110 | 00117 | 157640 | PEOA1 | Progressive external ophthalmoplegia, autosomal dominant | POLG |

(continued)

| Disease ID | | | | Mitochondrial disease (description) | Associated with genes |
|---|---|---|---|---|---|
| # | MSeqDR | OMIM | Abbrev. | | |
| 111 | 00074 | 609286 | PEOA3 | PROGRESSIVE EXTERNAL OPHTHALMOPLEGIA WITH MITOCHONDRIAL DNA DELETIONS, AUTOSOMAL DOMINANT, 3; | C10orf2 TWNK |
| 112 | 00049 | 613077 | PEOA5 | Progressive external ophthalmoplegia with mitochondrial DNA deletions, autosomal dominant, 5, PEOA5 | RRM2B |
| 113 | 00050 | 615156 | PEOA6 | Progressive external ophthalmoplegia with mitochondrial DNA deletions, autosomal dominant, 6, PEOA6 | DNA2 |
| 114 | 00125 | 615371 | PHN | (Pulmonary hypertension, neonatal, susceptibility to} | CPS1 |
| 115 | 00483 | 614926 | PRLTS2 | PERRAULT SYNDROME 2; PRLTS2 | HARS2 |
| 116 | 00116 | 615300 | PRLTS4 | Perrault syndrome 4, 615300 (3) | LARS2 |
| 117 | 00042 | 607459 | SANDO | Mitochondrial recessive ataxia syndrome (includes SANDO and SCAE) | C10orf2 POLG |
| 118 | 00498 | 610246 | SCA28 | SPINOCEREBELLAR ATAXIA 28; SCA28 | AFG3L2 |
| 119 | 00075 | 271930 | SNDI | STRIATONIGRAL DEGENERATION, INFANTILE; SNDI | NUP62 |
| 120 | 00083 | 613672 | SPAX4 | Ataxia, spastic, 4, 613672 (3) | MTPAP |
| 121 | 00497 | 607259 | SPG7 | SPASTIC PARAPLEGIA 7, AUTOSOMAL RECESSIVE; SPG7 | SPG7 |
| 122 | 00490 | 604928 | WFS2 | WOLFRAM SYNDROME 2; WFS2 | CISD2 |
| 123 | 00433 | 161700 | TBD | NECROTIZING ENCEPHALOMYELOPATHY, SUBACUTE, OF LEIGH, ADULT | - |
| 124 | 00464 | 201460 | TBD | MOVED TO 201475 | ACADVL |
| 125 | 00402 | 203750 | TBD | ALPHA-METHYLACETOACETIC ACIDURIA | ACAT1 |
| 126 | 00438 | 212160 | TBD | CARNITINE DEFICIENCY, MYOPATHIC | - |
| 127 | 00403 | 212350 | TBD | SENGERS SYNDROME | AGK |

(continued)

| Disease ID | | | | Mitochondrial disease (description) | Associated with genes |
|---|---|---|---|---|---|
| # | MSeqDR | OMIM | Abbrev. | | |
| 128 | 00012 | 220110 | TBD | Mitochondrial complex IV deficiency | MT-C01<br>MT-CO2<br>MT-CO3<br>MT-TL1<br>MT-TN<br>APO PT1<br>COA5<br>COA7<br>COX10<br>COX14<br>COX20<br>COX6B1<br>COX8A<br>FASTKD2<br>PET100<br>SC01<br>SURF1<br>TAC01 |
| 129 | 00404 | 221745 | TBD | DEAFNESS, SENSORINEURAL, AUTOSOMAL-MITOCHONDRIAL TYPE | - |
| 130 | 00085 | 237300 | TBD | Carbamoyl phosphate synthetase I deficiency;CPS I DEFICIENCY | CPS1 |
| 131 | 00405 | 238710 | TBD | HYPERLYSINEMIA DUE TO DEFECT IN LYSINE TRANSPORT INTO | - |
| 132 | 00106 | 238970 | TBD | Hyperornithinemia-hyperammonemia-homocitrullinemia syndrome | SLC25A15 |
| 133 | 00038 | 245400 | TBD | Mitochondrial DNA depletion syndrome 9 (encephalomyopathic type with methylmalonic aciduria) | SUCLG1 |
| 134 | 00407 | 251945 | TBD | MITOCHONDRIAL MYOPATHY WITH A DEFECT IN MITOCHONDRIAL-PROTEIN TRANSPORT | - |
| 135 | 00408 | 251950 | TBD | MITOCHONDRIAL MYOPATHY WITH LACTIC ACIDOSIS | PNPLA8 |

(continued)

| Disease ID | | | | Mitochondrial disease (description) | Associated with genes |
|---|---|---|---|---|---|
| # | MSeqDR | OMIM | Abbrev. | | |
| 136 | 00011 | 252010 | TBD | Mitochondrial complex I deficiency | MT-ND1<br>MT-ND2<br>MT-ND3<br>MT-ND4<br>MT-TN<br>FOXRED1<br>NDUFA1<br>NDUFA11<br>NDUFAF1<br>NDUFAF2<br>NDUFAF3<br>NDUFAF4<br>NDUFAF5<br>NDUFB3<br>NDUFB9<br>NDUFB11<br>NDUFS1<br>NDUFS2<br>NDUFS3<br>NDUFS4<br>NDUFS6<br>NDUFS8<br>NDUFV1<br>NDUFV2<br>NUBPL<br>TIMMDC1<br>TMEM126B |
| 137 | 00016 | 252011 | TBD | Mitochondrial complex II deficiency | SDHA<br>SDHAF1<br>SDHD |
| 138 | 00486 | 255110 | TBD | CARNITINE PALMITOYLTRANSFERASE II DEFICIENCY, LATE-ONSET | CPT2 |
| 139 | 00440 | 255120 | TBD | CARNITINE PALMITOYLTRANSFERASE I DEFICIENCY | CPT1A |
| 140 | 00409 | 255140 | TBD | MYOPATHY WITH GIANT ABNORMAL MITOCHONDRIA | - |
| 141 | 00118 | 258450 | TBD | Progressive external ophthalmoplegia, autosomal recessive | POLG |
| 142 | 00410 | 258470 | TBD | OPHTHALMOPLEGIC NEUROMUSCULAR DISORDER WITH ABNORMAL MITOCHONDRIA | - |
| 143 | 00046 | 261650 | TBD | PEPCK deficiency, mitochondrial | PCK2 |
| 144 | 00398 | 266150 | TBD | Pyruvate carboxylase deficiency | PC |
| 145 | 00119 | 267500 | TBD | Reticular dysgenesis | AK2 |
| 146 | 00526 | 300438 | TBD | HSD10 mitochondrial disease | HSD17B10 |

(continued)

| Disease ID | | | | Mitochondrial disease (description) | Associated with genes |
|---|---|---|---|---|---|
| # | MSeqDR | OMIM | Abbrev. | | |
| 147 | 00575 | 301020 | TBD | MITOCHONDRIAL COMPLEX I DEFICIENCY, NUCLEAR TYPE 12; MC1DN12 | NDUFA1 |
| 148 | 00576 | 301021 | TBD | MITOCHONDRIAL COMPLEX I DEFICIENCY, NUCLEAR TYPE 30; MC1DN30 | NDUFB11 |
| 149 | 00390 | 308930 | TBD | Leigh syndrome, X-linked | PDHA1 |
| 150 | 00151 | 500000 | TBD | CARDIOMYOPATHY, INFANTILE HISTIOCYTOID | MT-CYB |
| 151 | 00146 | 500002 | TBD | MITOCHONDRIAL MYOPATHY WITH DIABETES | MT-TE |
| 152 | 00157 | 500003 | TBD | STRIATONIGRAL DEGENERATION, INFANTILE, MITOCHONDRIAL | MT-ATP6 |
| 153 | 00160 | 500004 | TBD | RETINITIS PIGMENTOSA-DEAFNESS SYNDROME | MT-TS2<br>CDH23<br>CLRN1<br>DFNB31<br>GPR98<br>MYO7A<br>PCDH15<br>USH1C<br>USH1G<br>USH2A |
| 154 | 00148 | 500005 | TBD | HYPOMAGNESEMIA, HYPERTENSION, AND HYPERCHOLESTEROLEMIA, MITOCHONDRIAL, caused by a mutation | MT-TI |
| 155 | 00570 | 500006 | TBD | CARDIOMYOPATHY, INFANTILE HYPERTROPHIC | MT-ATP6<br>MT-ATP8 |
| 156 | 00159 | 500008 | TBD | DEAFNESS, NONSYNDROMIC SENSORINEURAL, MITOCHONDRIAL | MT-ND1<br>MT-RNR1<br>MT-TH<br>MT-TI<br>MT-TS1 |
| 157 | 00571 | 500010 | TBD | ATAXIA AND POLYNEUROPATHY, ADULT-ONSET | MT-ATP6 |
| 158 | 00577 | 500014 | TBD | MITOCHONDRIAL COMPLEX I DEFICIENCY, MITOCHONDRIAL TYPE 1; MC1DM1 | - |
| 159 | 00147 | 502000 | TBD | AGING aging represents a phenotype seemingly related to changes in mitochondrial | - |
| 160 | 00161 | 502500 | TBD | ALZHEIMER DISEASE, SUSCEPTIBILITY TO, MITOCHONDRIAL | - |
| 161 | 00145 | 515000 | TBD | CHLORAMPHENICOL TOXICITY;ANEMIA, CHLORAMPHENICOL- INDUCED; CHLORAMPHENICOL | MT-RNR2 |
| 162 | 00164 | 520100 | TBD | DIARRHEA, CHRONIC, WITH VILLOUS ATROPHY Links | - |
| 163 | 00166 | 550500 | TBD | MYOGLOBINURIA, RECURRENT | MT-C01 |

(continued)

| Disease ID | | | | Mitochondrial disease (description) | Associated with genes |
|---|---|---|---|---|---|
| # | MSeqDR | OMIM | Abbrev. | | |
| 164 | 00171 | 551200 | TBD | NEPHROPATHY, CHRONIC TUBULOINTERSTITIAL | - |
| 165 | 00167 | 553000 | TBD | ONCOCYTOMA | MT-ND6 |
| 166 | 00170 | 556500 | TBD | PARKINSON DISEASE, MITOCHONDRIAL | MT-TK<br>NDUFV2 |
| 167 | 00169 | 557000 | TBD | PEARSON MARROW-PANCREAS SYNDROME | - |
| 168 | 00144 | 560000 | TBD | RENAL TUBULOPATHY, DIABETES MELLITUS, AND CEREBELLAR ATAXIA, caused by duplication of mitochondrial DNA | - |
| 169 | 00051 | 580000 | TBD | DEAFNESS, AMINOGLYCOSIDE-INDUCED; STREPTOMYCIN-INDUCED | MT-C01<br>MT-RNR1<br>TRMU |
| 170 | 00156 | 598500 | TBD | WOLFRAM SYNDROME, MITOCHONDRIAL FORM | - |
| 171 | 00412 | 600706 | TBD | PROXIMAL MYOPATHY WITH FOCAL DEPLETION OF MITOCHONDRIA | - |
| 172 | 00566 | 600851 | TBD | Mitochondrial import-stimulating factor | - |
| 173 | 00114 | 601665 | TBD | Obesity, autosomal dominant | MT-CYB<br>ADRB2<br>ADRB3<br>AGRP<br>CARTPT<br>ENPP1<br>GHRL<br>KANK1<br>LEPR<br>MC4R<br>MRAP2<br>NR0B2<br>POMC<br>PPARG<br>PPARGC1B<br>PYY<br>SDC3<br>SIM1<br>UCP1<br>UCP3 |
| 174 | 00567 | 602252 | TBD | Mitochondrial intermembrane space protein tim12, yeast, homolog of | - |
| 175 | 00104 | 603358 | TBD | GRACILE syndrome | BCS1L |
| 176 | 00023 | 604273 | TBD | Mitochondrial complex V (ATP synthase) deficiency, nuclear type 1 | ATPAF2 |
| 177 | 00089 | 605814 | TBD | Citrullinemia, type II, neonatal-onset | SLC25A13 |

(continued)

| Disease ID | | | | Mitochondrial disease (description) | Associated with genes |
|---|---|---|---|---|---|
| # | MSeqDR | OMIM | Abbrev. | | |
| 178 | 00105 | 605911 | TBD | HMG-CoA synthase-2 deficiency; MITOCHONDRIAL HMG-CoA SYNTHASE DEFICIENCY | HMGCS2 |
| 179 | 00416 | 606407 | TBD | HYPOTONIA-CYSTINURIA SYNDROME | - |
| 180 | 00487 | 608836 | TBD | CARNITINE PALMITOYLTRANSFERASE II DEFICIENCY, LETHAL NEONATAL | CPT2 |
| 181 | 00417 | 609015 | TBD | TRIFUNCTIONAL PROTEIN DEFICIENCY | HADHA HADHB |
| 182 | 00047 | 609283 | TBD | Progressive external ophthalmoplegia with mitochondrial DNA deletions 3 | SLC25A4 |
| 183 | 00048 | 610131 | TBD | Progressive external ophthalmoplegia with mitochondrial DNA deletions, autosomal dominant 4 | POLG2 |
| 184 | 00081 | 610251 | TBD | Alcohol sensitivity, acute | ALDH2 |
| 185 | 00040 | 610773 | TBD | Mitochondrial phosphate carrier deficiency | SLC25A3 |
| 186 | 00418 | 611105 | TBD | LEUKOENCEPHALOPATHY WITH BRAINSTEM AND SPINAL CORD INVOLVEMENT | DARS2 |
| 187 | 00419 | 611126 | TBD | ACYL-CoA DEHYDROGENASE FAMILY, MEMBER 9, DEFICIENCY OF | ACAD9 |
| 188 | 00034 | 612073 | TBD | Mitochondrial DNA depletion syndrome 5 (encephalomyopathic with or without methylmalonic aciduria) | SUCLA2 |
| 189 | 00037 | 612075 | TBD | Mitochondrial DNA depletion syndrome 8A (encephalomyopathic type with renal tubulopathy) | RRM2B |
| 190 | 00107 | 612949 | TBD | Hypomyelination, global cerebral | SLC25A12 |
| 191 | 00045 | 613076 | TBD | Myopathy, mitochondrial progressive, with congenital cataract, hearing loss, and developmental delay | GFER |
| 192 | 00101 | 613657 | TBD | D-2-hydroxyglutaric aciduria 2 | IDH2 |
| 193 | 00024 | 614052 | TBD | Mitochondrial complex V (ATP synthase) deficiency, nuclear type 2 | TMEM70 |
| 194 | 00025 | 614053 | TBD | Mitochondrial complex V (ATP synthase) deficiency, nuclear type 3 | ATP5E ATP5F1E |
| 195 | 00014 | 614388 | TBD | Encephalopahty, lethal, due to defective mitochondrial peroxisomal fission | DNM1L |
| 196 | 00401 | 614520 | TBD | ENCEPHALOMYOPATHY, MITOCHONDRIAL, DUE TO VOLTAGE-DEPENDENT ANION CHANNEL DEFICIENCY | - |
| 197 | 00511 | 614924 | TBD | Combined oxidative phosphorylation deficiency 12 | EARS2 |
| 198 | 00512 | 614932 | TBD | Combined oxidative phosphorylation deficiency 13 | PNPT1 |
| 199 | 00010 | 615228 | TBD TBD | Mitochondrial complex (ATP synthase) deficiency, nuclear type 4 | ATP5A1 ATP5F1A |
| 200 | 00513 | 615440 | TBD | Combined oxidative phosphorylation deficiency 17 | ELAC2 |

(continued)

| Disease ID | | | | Mitochondrial disease (description) | Associated with genes |
|---|---|---|---|---|---|
| # | MSeqDR | OMIM | Abbrev. | | |
| 201 | 00514 | 615578 | TBD | Combined oxidative phosphorylation deficiency 18 | SFXN4 |
| 202 | 00500 | 615595 | TBD | Combined oxidative phosphorylation deficiency 19 | LYRM4 |
| 203 | 00507 | 615824 | TBD | Mitochondrial complex III deficiency, nuclear type 7 | UQCC2 |
| 204 | 00528 | 615838 | TBD | Mitochondrial complex III deficiency, nuclear type 8 | LYRM7 |
| 205 | 00515 | 615917 | TBD | Combined oxidative phosphorylation deficiency 20 | VARS2 |
| 206 | 00501 | 615918 | TBD | Combined oxidative phosphorylation deficiency 21 | TARS2 |
| 207 | 00502 | 616045 | TBD | Combined oxidative phosphorylation deficiency 22 | ATP5A1 ATP5F1A |
| 208 | 00508 | 616111 | TBD | Mitochondrial complex III deficiency, nuclear type 9 | UQCC3 |
| 209 | 00516 | 616198 | TBD | Combined oxidative phosphorylation deficiency 23 | GTPBP3 |
| 210 | 00509 | 616209 | TBD | Myopathy, isolated mitochondrial, autosomal dominant | CHCHD10 |
| 211 | 00517 | 616239 | TBD | Combined oxidative phosphorylation deficiency 24 | NARS2 |
| 212 | 00529 | 616277 | TBD | Mitochondrial short-chain enoyl-CoA hydratase 1 deficiency | ECHS1 |
| 213 | 00530 | 616370 | TBD | Multiple mitochondrial dysfunctions syndrome 4 | ISCA2 |
| 214 | 00503 | 616430 | TBD | Combined oxidative phosphorylation deficiency 25 | MARS2 |
| 215 | 00534 | 616479 | TBD | Progressive external ophthalmoplegia with mitochondrial DNA deletions, autosomal recessive 2 | RNASEH1 |
| 216 | 00518 | 616539 | TBD | Combined oxidative phosphorylation deficiency 26 | TRMT5 |
| 217 | 00519 | 616672 | TBD | Combined oxidative phosphorylation deficiency 27 | CARS2 |
| 218 | 00520 | 616794 | TBD | Combined oxidative phosphorylation deficiency 28 | SLC25A26 |
| 219 | 00504 | 616811 | TBD | Combined oxidative phosphorylation deficiency 29 | TXN2 |
| 220 | 00505 | 616896 | TBD | Mitochondrial DNA depletion syndrome 14 (encephalocardiomyopathic type) | OPA1 |
| 221 | 00521 | 616974 | TBD | Combined oxidative phosphorylation deficiency 30 | TRMT10C |
| 222 | 00510 | 617069 | TBD | Progressive external ophthalmoplegia with mitochondrial DNA deletions, autosomal recessive 3 | TK2 |
| 223 | 00535 | 617070 | TBD | Progressive external ophthalmoplegia with mitochondrial DNA deletions, autosomal recessive 4 | DGUOK |
| 224 | 00525 | 617086 | TBD | Encephalopathy due to defective mitochondrial and peroxisomal fission 2 | MFF |
| 225 | 00506 | 617156 | TBD | Mitochondrial DNA depletion syndrome 15 (hepatocerebral type) | TFAM |
| 226 | 00527 | 617184 | TBD | Mitochondrial DNA depletion syndrome 12A (cardiomyopathic type) AD | SLC25A4 |
| 227 | 00522 | 617228 | TBD | Combined oxidative phosphorylation deficiency 31 | MIPEP |
| 228 | 00531 | 617613 | TBD | Multiple mitochondrial dysfunctions syndrome 5 | ISCA1 |

(continued)

| Disease ID | | | | Mitochondrial disease (description) | Associated with genes |
|---|---|---|---|---|---|
| # | MSeqDR | OMIM | Abbrev. | | |
| 229 | 00523 | 617664 | TBD | Combined oxidative phosphorylation deficiency 32 | MRPS34 |
| 230 | 00532 | 617675 | TBD | Myopathy, mitochondrial, and ataxia | MST01 |
| 231 | 00533 | 617710 | TBD | Neurodevelopmental disorder, mitochondrial, with abnormal movements and lactic acidosis, with or without seizures | WARS2 |
| 232 | 00524 | 617713 | TBD | Combined oxidative phosphorylation deficiency 33 | C1QBP |
| 233 | 00563 | 617872 | TBD | COMBINED OXIDATIVE PHOSPHORYLATION DEFICIENCY 34 | MRPS7 |
| 234 | 00564 | 617873 | TBD | COMBINED OXIDATIVE PHOSPHORYLATION DEFICIENCY 35 | TRIT1 |
| 235 | 00565 | 617950 | TBD | COMBINED OXIDATIVE PHOSPHORYLATION DEFICIENCY 36 | MRPS2 |
| 236 | 00568 | 617954 | TBD | MULTIPLE MITOCHONDRIAL DYSFUNCTIONS SYNDROME 6 | PMPCB |
| 237 | 00578 | 618098 | TBD | PROGRESSIVE EXTERNAL OPHTHALMOPLEGIA WITH MITOCHONDRIAL DNA DELETIONS, AUTOSOMAL RECESSIVE 5; | TOP3A |
| 238 | 00579 | 618120 | TBD | MITOCHONDRIAL COMPLEX V (ATP SYNTHASE) DEFICIENCY, NUCLEAR TYPE 5; MC5DN5 | ATP5F1D |
| 239 | 00580 | 618222 | TBD | MITOCHONDRIAL COMPLEX I DEFICIENCY, NUCLEAR TYPE 2; MC1DN2 | NDUFS8 |
| 240 | 00581 | 618224 | TBD | MITOCHONDRIAL COMPLEX I DEFICIENCY, NUCLEAR TYPE 3; MC1DN3 | NDUFS7 |
| 241 | 00582 | 618225 | TBD | MITOCHONDRIAL COMPLEX I DEFICIENCY, NUCLEAR TYPE 4; MC1DN4 | NDUFV1 |
| 242 | 00583 | 618226 | TBD | MITOCHONDRIAL COMPLEX I DEFICIENCY, NUCLEAR TYPE 5; MC1DN5 | NDUFS1 |
| 243 | 00584 | 618228 | TBD | MITOCHONDRIAL COMPLEX I DEFICIENCY, NUCLEAR TYPE 6; MC1DN6 | NDUFS2 |
| 244 | 00585 | 618229 | TBD | MITOCHONDRIAL COMPLEX I DEFICIENCY, NUCLEAR TYPE 7; MC1DN7 | NDUFV2 |
| 245 | 00586 | 618230 | TBD | MITOCHONDRIAL COMPLEX I DEFICIENCY, NUCLEAR TYPE 8; MC1DN8 | NDUFS3 |
| 246 | 00587 | 618232 | TBD | MITOCHONDRIAL COMPLEX I DEFICIENCY, NUCLEAR TYPE 9; MC1DN9 | NDUFS6 |
| 247 | 00588 | 618234 | TBD | MITOCHONDRIAL COMPLEX I DEFICIENCY, NUCLEAR TYPE 11; MC1DN11 | NDUFAF1 |
| 248 | 00589 | 618235 | TBD | MITOCHONDRIAL COMPLEX I DEFICIENCY, NUCLEAR TYPE 13; MC1DN13 | NDUFA2 |
| 249 | 00590 | 618236 | TBD | MITOCHONDRIAL COMPLEX I DEFICIENCY, NUCLEAR TYPE 14; MC1DN14 | NDUFA11 |

(continued)

| Disease ID | | | | Mitochondrial disease (description) | Associated with genes |
|---|---|---|---|---|---|
| # | MSeqDR | OMIM | Abbrev. | | |
| 250 | 00591 | 618237 | TBD | MITOCHONDRIAL COMPLEX I DEFICIENCY, NUCLEAR TYPE 15; MC1DN15 | NDUFAF4 |
| 251 | 00592 | 618238 | TBD | MITOCHONDRIAL COMPLEX I DEFICIENCY, NUCLEAR TYPE 16; MC1DN16 | - |
| 252 | 00593 | 618239 | TBD | MITOCHONDRIAL COMPLEX I DEFICIENCY, NUCLEAR TYPE 17; MC1DN17 | - |
| 253 | 00594 | 618240 | TBD | MITOCHONDRIAL COMPLEX I DEFICIENCY, NUCLEAR TYPE 18; MC1DN18 | NDUFAF3 |
| 254 | 00595 | 618241 | TBD | MITOCHONDRIAL COMPLEX I DEFICIENCY, NUCLEAR TYPE 19; MC1DN19 | FOXRED1 |
| 255 | 00596 | 618242 | TBD | MITOCHONDRIAL COMPLEX I DEFICIENCY, NUCLEAR TYPE 21; MC1DN21 | NUBPL |
| 256 | 00597 | 618243 | TBD | MITOCHONDRIAL COMPLEX I DEFICIENCY, NUCLEAR TYPE 22; MC1DN22 | NDUFA10 |
| 257 | 00598 | 618244 | TBD | MITOCHONDRIAL COMPLEX I DEFICIENCY, NUCLEAR TYPE 23; MC1DN23 | NDUFA12 |
| 258 | 00599 | 618245 | TBD | MITOCHONDRIAL COMPLEX I DEFICIENCY, NUCLEAR TYPE 24; MC1DN24 | NDUFB9 |
| 259 | 00600 | 618246 | TBD | MITOCHONDRIAL COMPLEX I DEFICIENCY, NUCLEAR TYPE 25; MC1DN25 | NDUFB3 |
| 260 | 00601 | 618247 | TBD | MITOCHONDRIAL COMPLEX I DEFICIENCY, NUCLEAR TYPE 26; MC1DN26 | NDUFA9 |
| 261 | 00602 | 618248 | TBD | MITOCHONDRIAL COMPLEX I DEFICIENCY, NUCLEAR TYPE 27; MC1DN27 | MTFMT |
| 262 | 00603 | 618249 | TBD | MITOCHONDRIAL COMPLEX I DEFICIENCY, NUCLEAR TYPE 28; MC1DN28 | NDUFA13 |
| 263 | 00604 | 618250 | TBD | MITOCHONDRIAL COMPLEX I DEFICIENCY, NUCLEAR TYPE 29; MC1DN29 | TMEM126B |
| 264 | 00605 | 618251 | TBD | MITOCHONDRIAL COMPLEX I DEFICIENCY, NUCLEAR TYPE 31; MC1DN31 | TIMMDC1 |
| 265 | 00606 | 618252 | TBD | MITOCHONDRIAL COMPLEX I DEFICIENCY, NUCLEAR TYPE 32; MC1DN32 | NDUFB8 |
| 266 | 00607 | 618253 | TBD | MITOCHONDRIAL COMPLEX I DEFICIENCY, NUCLEAR TYPE 33; MC1DN33 | NDUFA6 |
| 267 | 00608 | 618256 | TBD | BIPARENTAL MITOCHONDRIAL DNA TRANSMISSION | - |

The present disclosure also relates to embodiments disclosed in the following numbered paragraphs:

1. A method of treating a human subject with a disease caused by mitochondrial dysfunction comprising administering an amount of probucol effective to maintain or improve mitochondrial function.

2. The method of paragraph 1 wherein the diagnosis of said mitochondrial dysfunction is based in whole or part on the presence of at least one mutation or variant in the subject's DNA.

3. A method of treating a human subject with a disease caused by mitochondrial dysfunction comprising:

(a) obtaining a biological sample from the human subject,
(b) detecting the presence in DNA from said sample of at least one mutation or variant associated with mitochondrial dysfunction, and
(c) administering to the human subject with said DNA mutation or variant associated with mitochondrial dysfunction probucol or a pharmaceutically acceptable salt thereof in an amount effective to maintain or improve mitochondrial function.

4. The method of any of the preceding paragraphs, wherein the subject has a mitochondrial disease selected from the group consisting of: autosomal dominant optic atrophy (ADOA); beta-oxidation defects; carnitine deficiency; carnitine-acyl-carnitine deficiency; chronic progressive external ophthalmoplegia syndrome (CPEO); coenzyme Q10 deficiency; complex I deficiency (NADH dehydrogenase deficiency); complex !! deficiency (succinate dehydrogenase deficiency); complex III deficiency (ubiquinone-cytochrome c oxidoreductase deficiency); complex IV deficiency (cytochrome c oxidase deficiency or COX deficiency'); complex V deficiency (ATP synthase deficiency); multiple respiratory chain complex deficiency; carnitine palmitoyltransferase (CPT) ! deficiency; CPT !! deficiency; diabetes mellitus and deafness (DAD); creatine deficiency syndrome; Friedreich's ataxia (FA); Kearns-Sayre syndrome (KSS); lactic acidosis; Leber's hereditary optic neuropathy (LHON); Leigh syndrome (Leigh disease); lethal infantile cardiomyopathy (LIC or Barth Syndrome); leukodystrophy; leukoencephalopathy with brain stem and spinal cord involvement and lactate elevation (LBSL); long-chain acyl-CoA dehydrogenase deficiency (LCAD); long-chain 3-hydroxyacyl-CoA dehydrogenase (LCHAD); Luft disease; medium-chain acyl-CoA dehydrogenase deficiency (MCAD); mitochondrial cytopathy; mitochondrial DNA depletion; mitochondrial DNA deletion(s); mitochondrial encephalopathy; mitochondrial myopathy (MM); mitochondrial myopathy, encephalomyopathy, lactic acidosis, stroke-like symptoms (MELAS); mitochondrial enoyl CoA reductase protein associated neurodegeneration (MEPAN); mitochondrial neurogastrointestinal encephalomyopathy (MNGIE); mitochondrial recessive ataxia syndrome (MIRAS); multiple acyl-CoA dehydrogenase deficiency (MAD or glutaric aciduria type II); myoclonic epilepsy with ragged red fibers (MERRF); neuropathy, ataxia, and retinitis pigmentosa (NARP); Pearson syndrome; POLG mutations; progressive infantile poliodystrophy (Alper's disease); ptosis; pyruvate carboxylase deficiency (PCD); pyruvate dehydrogenase complex deficiency (PDCD or PDH); short-chain acyl-CoA dehydrogenase deficiency (SCAD); short chain 3-hydroxyacyl-CoA dehydrogenase deficiency (SCHAD); and very long-chain acyl-CoA dehydrogenase deficiency (VLCAD).

5. The method of any of the preceding paragraphs wherein at least one mutation or variant is in a mitochondrial DNA encoded gene selected from the group consisting of MT-ATP6, MT-ATP8, MT-C01, MT-CO2, MT-CO3, MT-CYB, MT-ND1, MT-ND2, MT-ND3, MT-ND4, MT-ND4L, MT-ND5, MT-ND6, MT-RNR1, MT-RNR2, MT-TA, MT-TC, MT-TE, MT-TF, MT-TH, MT-TI, MT-TK, MT-TL1, MT-TL2, MT-TM, MT-TN, MT-TQ, MT-TS1, MT-TS2, MT-TV and MT-TW.

6. The method of any of the preceding paragraphs wherein at least one mutation or variant is in a nuclear DNA encoded gene selected from the group consisting of AARS2, ABCC8, ACAD9, ACADM, ACADS, ACADVL, ACAT1, ACO2, ADCK3, ADRB2, ADRB3, AFG3L2, AGK, AGRP, AIFM1, AK2, AKT2, ALDH2, AMT, APOPT1, ATP5A1, ATP5E, ATP5F1A, ATP5F1D, ATP5F1E, ATPAF2, AUH, BCS1L, BOLA3, C10orf2, C12orf65, C19orf12, C1QBP, CAPN10, CARS2, CARTPT, CDH23, CDKAL1, CHCHD10, CHKB, CISD2, CLRN1, COA5, COA7, COQ2, COQ4, COQ6, COQ7, COQ8A, COQ9, COX10, COX14, COX15, COX20, COX6B1, COX8A, CPS1, CPT1A, CPT2, CYC1, DARS2, DFNB31, DGUOK, DLAT, DNA2, DNAJC19, DNM1L, EARS2, ECHS1, ELAC2, ENPP1, ETFA, ETFB, ETFDH, ETHE1, FARS2, FASTKD2, FBXL4, FOXRED1, FXN, GATM, GCGR, GCK, GCSH, GFER, GFM1, GHRL, GJB2, GJB3, GJB6, GLDC, GPD2, GPR98, GTPBP3, HADH, HADHA, HADHB, HARS2, HMGA1, HMGCS2, HNF1A, HNF1B, HNF4A, HSD17B10, HSPD1, IBA57, IDH2, IGF2BP2, IL6, INSR, IRS1, IRS2, ISCA1, ISCA2, ISCU, KANK1, KCNJ11, LARS2, LEPR, LIAS, LIPC, LRPPRC, LYRM4, LYRM7, MAPK8IP1, MARS2, MC4R, MFF, MFN2, MGME1, MIPEP, MPC1, MPV17, MRAP2, MRPL3, MRPL44, MRPS16, MRPS2, MRPS22, MRPS34, MRPS7, MST01, MTFMT, MT01, MTPAP, MYO7A, NARS2, NDUFA1, NDUFA10, NDUFA11, NDUFA12, NDUFA13, NDUFA2, NDUFA6, NDUFA9, NDUFAF1, NDUFAF2, NDUFAF3, NDUFAF4, NDUFAF5, NDUFAF6, NDUFB11, NDUFB3, NDUFB8, NDUFB9, NDUFS1, NDUFS2, NDUFS3, NDUFS4, NDUFS6, NDUFS7, NDUFS8, NDUFV1, NDUFV2, NEUROD1, NF2, NFU1, NR0B2, NUBPL, NUP62, OPA1, OPA3, PAX4, PC, PCDH15, PCK2, PDHA1, PDHB, PDP1, PDSS1, PDSS2, PDX1, PET100, PMPCB, PNPLA8, PNPT1, POLG, POLG2, POMC, PPARG, PPARGC1B, PPP1R3A, PUS1, PYY, RARS2, RMND1, RNASEH1, RRM2B, SCO1, SCO2, SDC3, SDHA, SDHAF1, SDHD, SERAC1, SFXN4, SIM1, SLC22A5, SLC25A1, SLC25A12, SLC25A13, SLC25A15, SLC25A19, SLC25A20, SLC25A22, SLC25A26, SLC25A3, SLC25A4, SLC2A2, SLC2A4, SLC30A8, SPG7, SUCLA2, SUCLG1, SURF1, TAC01, TARS2, TAZ, TDF7L2, TFAM, TIMM8A, TIMMDC1, TK2, TMEM126B, TMEM70, TOP3A, TRIT1, TRMT10C,

TRMT5, TRMU, TRNT1, TSFM, TTC19, TUFM, TWNK, TXN2, TYMP, UCP1, UCP3, UQCC2, UQCC3, UQCRB, UQCRC2, UQCRQ, USH1C, USH1G, USH2A, VARS2, WARS2, WFS1 and YARS2.

7. The method of any of the preceding paragraphs wherein at least one mutation or variant is in a nuclear DNA encoded gene selected from the group consisting of ABCB7, ACADSB, AKAP10, ALAS2, ALDH4A1, ALDH6A1, AMACR, APTX, ARMS2, BAX, BCAT2, BCKDHA, BCKDHB, BCL2, C12orf62, C20orf7, C8orf38, COX4I2, CRAT, CYB5R3, CYCS, CYP11A1, CYP11B1, CYP11B2, CYP24A1, CYP27A1, CYP27B1, D2HGDH, DBT, DECR1, DHODH, DIABLO, DLD, DMGDH, FH, GDAP1, GK, GLRX5, GLUD1, HCCS, HIBCH, HK1, HLCS, HMGCL, HOGA1, HTRA2, IDH3B, IVD, KARS, KIF1B, L2HGDH, LRRK2, MAOA, MCCC1, MCCC2, MCEE, ME2, MLYCD, MMAA, MMAB, MMADHC, MUT, NAGS, NGLY1, OAT, OGDH, OTC, OXCT1, PANK2, PARK2, PARK7, PCCA, PCCB, PDHX, PINK1, PNKD, PPOX, PYCR1, REEP1, RMRP, SACS, SARDH, SARS2, SDHAF2, SDHB, SDHC, SOD2, SPG20, STAR, TMEM126, UCP2, USMG5, WWOX and XPNPEP3.

8. The method of paragraph 2 or 3 wherein the disease is Myoclonus Epilepsy with Ragged-Red Fibers (MERRF) and the DNA mutation or variant is in one or more genes selected from the group consisting of MT-ND5, MT-TF, MT-TH, MT-TS1, MT-TS2, MT-TL1 and MT-TK.

9. The method of paragraph 2 or 3 wherein the disease is mitochondrial encephalopathy, lactic acidosis, and stroke-like episodes syndrome (MELAS) and the DNA mutation or variant is in one or more genes selected from the group consisting of MT-ND1, MT-ND4, Mt-ND5, MT-ND6, MT-TC, MT-TF, MT-TH, MT-TQ, MT-TS1, MT-TS2, MT-TL1 and MT-TK.

10. The method of paragraph 2 or 3 wherein the disease is non-syndromic sensorineural hearing loss and the DNA mutation or variant is in one or more genes selected from the group consisting of MT-RNR1 and MT-RNR2.

11. The method of paragraph 2 or 3 wherein the disease is Leigh syndrome and the DNA mutation or variant is in one or more genes selected from the group consisting of MT-ATP6, MT-TK, MT-TV, MT-TW, ATP5A1, ATP5E, BCS1L, COX10, COX15, CYC1,FOXRED1, LRPPRC, LYRM7, NDUFA10, NDUFA12, NDUFA2, NDUFA9, NDUFAF2, NDUFAF6, NDUFS3, NDUFS4, NDUFS7, NDUFS8, POLG, SCO1, SCO2, SDHA, SDHC, SDHC, SDHAF1, SDHAF2, SURF1, TMEM70, ATPAF2, UQCRB, UQCRQ, UQCRC2, UQCC2, UQCC3 and USMGS.

12. The method of paragraph 2 or 3 wherein the disease is leukoencephalopathy and the DNA mutation or variant is in one or more genes selected from the group consisting of DARS2, SDHA, SDHB, SDHC, SDHD, SDHAF1 and SDHAF2.

13. The method of paragraph 2 or 3 wherein the disease is Alpers-Huttenlocher syndrome and the DNA mutation or variant is in the POLG gene.

14. The method of paragraph 2 or 3 wherein the disease is mitochondrial neurogastrointestinal encephalopathy (MNGIE) syndrome and the DNA mutation or variant is in one or more genes selected from the group consisting of TK2, DGUOK, SUCLG1, SUCLA2, ABAT, TYMP, RRM2B, MT-TK, POLG, SLC25A4, AGK and MPV17.

15. The method of paragraph 2 or 3 wherein the disease is liver failure and the DNA mutation or variant is in the TFAM gene.

16. The method of paragraph 2 or 3 wherein the disease is a neurologic disease or liver disease and the DNA mutation or variant is in one or more genes selected from the group consisting of DARS2, RARS2, EARS2, MARS2, FARS2, YARS2, SARS2, AARS2 and HARS2.

17. The method of paragraph 2 or 3 wherein the disease is primary mitochondrial disease and the DNA mutation or variant is in one or more genes selected from the group consisting of MRPS and MRPL.

18. The method of paragraph 2 or 3 wherein the disease is a respiratory chain deficiency, neurologic disease, vision loss or liver disease and the DNA mutation or variant is in one or more genes selected from the group consisting of TAC01, GFM1 and C120RF65.

19. The method of paragraph 2 or 3 wherein the disease is peripheral neuropathy or optic neuropathy and the DNA mutation or variant is in one or more genes selected from the group consisting of OPA1, MFN1, MFN2, DNM1L and

MIEF2.

20. The method of paragraph 2 or 3 wherein the disease is a mitochondrial complex deficiency and the DNA mutation or variant is in one or more genes selected from the group consisting of MT-ATP6, MT-CO1, MT-CO2, MT-CO3, MT-ND1, MT-ND2, MT-ND3, MT-ND4, MT-TL1, MT-TN, MT-TK, MT-TV, MT-TW, APOPT1, ATPAF2, ATP5A1, ATP5E, ATP5F1A, ATP5F1D, ATP5F1E, BCS1L, COA5, COA7, COX6B1, COX8A,COX10, COX14, COX15, COX20,CYC1, FASTKD2, FOXRED1, MTFMT, NDUFA1, NDUFA2, NDUFA6, NDUFA9, NDUFA10, NDUFA11, NDUFA12, NDUFA13, NDUFAF1, NDUFAF2, NDUFAF3, NDUFAF4, NDUFAF5, NDUFAF6, NDUFB3, NDUFB8, NDUFB9, NDUFB11, NDUFS1, NDUFS2, NDUFS3, NDUFS4, NDUFS6, NDUFS7, NDUFS8, NDUFV1, NDUFV2, NUBPL, PET100, SCO1, SDHA, SDHAF1, SDHD, SURF1, TAC01, TIMMDC1, TMEM70, TMEM126B, LYRM4, TTC19, UQCC2, UQCC3, UQCRB, UQCRC2 and UQCRQ.

21. The method of paragraph 2 or 3 wherein the disease is a co-enzyme Q10 deficiency and the DNA mutation or variant is in one or more genes selected from the group consisting of ADCK3, COQ2, COQ4, COQ6, COQ7, COQ8A, COQ9, PDSS1 and PDSS2.

22. The method of paragraph 2 or 3 wherein the disease is chronic progressive external ophthalmoplegia (CPEO) and the DNA mutation or variant is in one or more genes selected from the group consisting of MT-TA, MT-TL2, MT-TM, MT-TW, C10orf2, DGUOK, DNA2, POLG, RNASEH1, RRM2B, SLC25A4, TK2, TOP3A and TWNK.

23. The method of paragraph 2 or 3 wherein the disease is a carnitine deficiency and the DNA mutation or variant is in one or more genes selected from the group consisting of CPT1A, CPT2, SLC22A5 and SLC22A20.

24. The method of paragraph 2 or 3 wherein the disease is Friedreich's ataxia and the DNA mutation or variant is in the FXN gene.

25. The method of paragraph 2 or 3 wherein the disease is Kearns-Sayre syndrome and the DNA mutation or variant is in the MT-TL1 gene.

26. The method of paragraph 2 or 3 wherein the disease is lethal infantile cardiomyopathy (LIC) or Barth syndrome and the DNA mutation or variant is in the TAZ gene.

27. The method of paragraph 2 or 3 wherein the disease is Leber's hereditary optic neuropathy (LHON) and the mutation or variant is in one or more genes selected from the group consisting of MT-ND1, MT-ND2, MT-ND3, MT-ND4, MT-ND5, MT-ND6, MT-ND4L, MT-CYB, MT-C01, MT-CO2, MT-CO3, MT-ATP6, and MT-ATP8.

28. The method of any preceding paragraph wherein the subject's mitochondrial dysfunction or mitochondrial disease is assessed, monitored, or diagnosed by a method comprising:

(i) employing a panel of two or more sub-instruments to measure one or more clinical symptoms of mitochondrial dysfunction or mitochondrial disease in a subject,
(ii) combining the measurements obtained from said two or more sub-instruments into a single composite measurement, and
(iii) assessing the overall severity of, or change in, the mitochondrial dysfunction or mitochondrial disease in the subject by comparing the composite measurement to a reference value or another composite measurement in the same subject.

29. The method of paragraph 28, wherein:

(i) one or more composite measurements are employed to measure the clinical effect on the subject of a diagnostic, therapeutic or other type of medical intervention;
(ii) for each of the sub-instruments in said panel, the subject is classified as:

(a) a sub-instrument responder or sub-instrument non-responder,
(b) a member of a clinical category, or
(c) a member of a metric range, based on the change in said one or more clinical symptoms as measured using said sub-instrument; and

(iii) the measurements obtained from the sub-instruments in the panel are combined into a single composite measurement, by either:

(a) separately assessing the change in each measurement obtained from the panel sub-instruments prior to combining each measurement into a single composite measurement, or
(b) combining measurements obtained from the panel sub-instruments at a first time point and generating a single composite measurement for said first time point and then comparing the single composite measurement for said first time point to a single composite measurement generated from the same panel sub-instruments for a second time point.

30. The method of paragraph 28 or 29, wherein the panel sub-instruments comprise one or more of Motor Function Measure, Six Minute Walk Test, Two Minute Walk Test, Modified Fatigue Impact Scale, Friedreich's Ataxia Rating Scale, Patient-Reported Outcomes Measurement Information System Gastrointestinal Symptom Scales, 30-Second Chair Stand Test, Muscle Strength by Myometry Test, Newcastle Mitochondrial Disease Adult Scale, Newcastle Mitochondrial Disease Pediatric Scale, 36-Item Short Form Survey, Clinical Global Impression, Patient's Global Impression, Patient-Reported Initial MitoPC Symptoms, Patient-Reported MitoPC Symptom Changes, Columbia Suicide Severity Rating Scale, Neuropathy Impairment Score, Migraine Disability Assessment Test, Quick Inventory of Depressive Symptomatology - Self-Report, Montreal Cognitive Assessment, NY Heart Association Functional Classification, Diabetes Health Profile, Ocular Motility Assessments, Marginal Reflex Distance, Ocular Motility & Fixation in 8 Gaze Directions, Degree of Ocular Saccades, Visual Function Test, Logarithm of Minimum Angle of Resolution, Pelli-Robson Score, Humphrey Visual Field mean deviation, or a combination thereof.

31. The method of paragraph 28 or 29, wherein the panel sub-instruments comprise Motor Function Measure, Six Minute Walk Test, Modified Fatigue Impact Scale, Friedreich's Ataxia Rating Scale, and the Patient-Reported Outcomes Measurement Information System Gastrointestinal Symptom Scales.

32. The method of paragraph 28 or 29, wherein the panel sub-instruments comprises five sub-instruments.

33. The method of paragraph 32, wherein the five sub-instruments comprise Motor Function Measure, Six Minute Walk Test, Modified Fatigue Impact Scale, Friedreich's Ataxia Rating Scale, and the Patient-Reported Outcomes Measurement Information System Gastrointestinal Symptom Scales.

34. The method of paragraph 28 or 29, wherein the one or more clinical symptoms are selected from the group consisting of fatigue, muscle weakness, neuromuscular dysfunction, exercise intolerance, imbalance, dysautonomia, gastrointestinal dysfunction, vision loss, eye muscle dysfunction, retinal dysfunction, optic nerve dysfunction, ptosis, headache, dehydration, peripheral neuropathy, numbness, epilepsy, seizures, insomnia, mood disorder, depression, diabetes mellitus, obesity, kidney dysfunction, hyperlipidemia, liver disease, sleep apnea, autism spectrum behavior, delayed developmental milestones, arrhythmia, heart muscle dysfunction, cardiac disease, stroke, speech disorder, tinnitus, hearing impairment, learning disability, cognitive impairment, dementia, or a combination thereof.

35. The method of any one of the preceding paragraphs, wherein the probucol, or a pharmaceutically acceptable salt thereof, is administered at a dose of about 1 mg, about 10 mg, about 100 mg, about 250 mg, about 500 mg, about 1 g, about 2 g, about 5 g, or about 10 g.

36. The method of any one of the preceding paragraphs, wherein the probucol, or a pharmaceutically acceptable salt thereof, is administered at a dose of about 1 mg/day, about 10 mg/day, about 100 mg/day, about 250 mg/day, about 500 mg/day, about 1 g/day, about 2 g/day, about 5 g/day, or about 10 g/day.

37. The method of any preceding paragraph wherein the probucol is administered as a liquid formulation.

38. The method of paragraph 37 wherein the liquid formulation is administered by a route selected from the group consisting of oral, enteral, gastrostomy tube, jejunostomy tube, orogastric tube, nasogastric tube, parenteral, intravenous, subcutaneous, intramuscular, intraperitoneal, intracisternal, intraarticular, intracerebral, intraparenchymal, intracerebro-ventricular, nasal, vaginal, sublingual, intraocular, intravitreal, rectal, topical, transdermal and inhalation.

39. The method of any one of paragraphs 1 to 36, wherein the probucol, or a pharmaceutically acceptable salt thereof, is administered in the form of a tablet.

40. The method of any one of the preceding paragraphs, wherein the probucol, or a pharmaceutically acceptable salt thereof, is administered on a continuous dosing schedule.

41. The method of any one of the preceding paragraphs, wherein the probucol, or a pharmaceutically acceptable salt thereof, is administered twice per day, once per day, once every two days, once every three days, once every four days, once every five days, once every six days, once a week, once every two weeks or once per month.

42. The method of any one of the preceding paragraphs, wherein the probucol, or a pharmaceutically acceptable salt thereof, is administered initially in a loading dose that is higher than a subsequent dose.

43. The method of any one of the preceding paragraphs, further comprising administering one or more additional pharmaceutical agents.

44. The method of paragraph 43, wherein the one or more additional pharmaceutical agents are selected from niacin, cycloheximide, bezafibrate, vatiquinone, carnitine, coenzyme Q10, alpha-tocopherolquinone, coenzyme Q10 analogs, cytochrome C, dichloroacetate, 5-[(e)-2-(4-hydroxyphenyl)-ethenyl] benzene-1,3 diol, flavin mononucleotide, elamipretide, idebenone, latrepirdine, levocarnitine, 2',3',5'-tri-O-acetyluridine, olesoxime, omaveloxolone, thiamin diphosphate, ubiquinone, vitamin C, vitamin D, vitamin E, thiamine, riboflavin, magnesium, calcium, phosphate, membrane phospholipid, unsaturated fatty acid, creatine, pyruvate, $\alpha$-lipoic acid, NADH, PPAR delta modulator, PPAR delta agonist, cysteamine bitartrate, nicotinic acid, nicotinamide, nicotinamide riboside, acipimox, resveratrol, glucose, L-carnitine, glutathione, redox-modulating agent, dichloroacetate, curcumin, schisandrin, triheptanoin, viral or non-viral vector gene therapy targeting mitochondrial disease mutations, or a combination thereof.

## Claims

1. Probucol, or a pharmaceutically acceptable salt thereof, for use in a method of treating a human subject with a disease caused by mitochondrial dysfunction, wherein the method comprises administering an amount of probucol, or the pharmaceutically acceptable salt thereof, effective to maintain or improve mitochondrial function.

2. Probucol, or the pharmaceutically acceptable salt thereof, for use according to claim 1, wherein the diagnosis of said mitochondrial dysfunction is based in whole or part on the presence of at least one mutation or variant in the subject's DNA.

3. Probucol, or the pharmaceutically acceptable salt thereof, for use according to claim 1 or 2, wherein the subject has a mitochondrial disease selected from the group consisting of: autosomal dominant optic atrophy (ADOA); beta-oxidation defects; carnitine deficiency; carnitine-acyl-carnitine deficiency; chronic progressive external ophthalmoplegia syndrome (CPEO); co-enzyme Q10 deficiency; complex I deficiency (NADH dehydrogenase deficiency); complex II deficiency (succinate dehydrogenase deficiency); complex III deficiency (ubiquinone-cytochrome c oxidoreductase deficiency); complex IV deficiency (cytochrome c oxidase deficiency or COX deficiency'); complex V deficiency (ATP synthase deficiency); multiple respiratory chain complex deficiency; carnitine palmitoyltransferase (CPT) I deficiency; CPT II deficiency; diabetes mellitus and deafness (DAD); creatine deficiency syndrome; Friedreich's ataxia (FA); Kearns-Sayre syndrome (KSS); lactic acidosis; Leber's hereditary optic neuropathy (LHON); Leigh syndrome (Leigh disease); lethal infantile cardiomyopathy (LIC or Barth Syndrome); leukodystrophy; leukoencephalopathy with brain stem and spinal cord involvement and lactate elevation (LBSL); long-chain acyl-CoA dehydrogenase deficiency (LCAD); long-chain 3-hydroxyacyl-CoA dehydrogenase (LCHAD); Luft disease; medium-chain acyl-CoA dehydrogenase deficiency (MCAD); mitochondrial cytopathy; mitochondrial DNA depletion; mitochondrial DNA deletion(s); mitochondrial encephalopathy; mitochondrial myopathy (MM); mitochondrial myopathy, encephalomyopathy, lactic acidosis, stroke-like symptoms (MELAS); mitochondrial enoyl CoA reductase protein associated neurodegeneration (MEPAN); mitochondrial neurogastrointestinal encephalomyopathy (MNGIE); mitochondrial recessive ataxia syndrome (MIRAS); multiple acyl-CoA dehydrogenase deficiency (MAD or glutaric aciduria type II); myoclonic epilepsy with ragged red fibers (MERRF); neuropathy, ataxia, and retinitis pigmentosa (NARP); Pearson syndrome; POLG mutations; progressive infantile poliodystrophy (Alper's disease); ptosis; pyruvate carboxylase deficiency (PCD); pyruvate dehydrogenase complex deficiency (PDCD or PDH); short-chain acyl-CoA dehydrogenase deficiency (SCAD); short chain 3-hydroxyacyl-CoA dehydrogenase deficiency (SCHAD); and very long-chain acyl-CoA dehydrogenase deficiency (VLCAD).

4. Probucol, or the pharmaceutically acceptable salt thereof, for use according to claim 2 or 3, wherein at least one

mutation or variant is in:

(i) a mitochondrial DNA encoded gene selected from the group consisting of MT-ATP6, MT-ATP8, MT-CO1, MT-CO2, MT-CO3, MT-CYB, MT-ND1, MT-ND2, MT-ND3, MT-ND4, MT-ND4L, MT-ND5, MT-ND6, MT-RNR1, MT-RNR2, MT-TA, MT-TC, MT-TE, MT-TF, MT-TH, MT-TI, MT-TK, MT-TL1, MT-TL2, MT-TM, MT-TN, MT-TQ, MT-TS1, MT-TS2, MT-TV and MT-TW;
(ii) a nuclear DNA encoded gene selected from the group consisting of AARS2, ABCC8, ACAD9, ACADM, ACADS, ACADVL, ACAT1, ACO2, ADCK3, ADRB2, ADRB3, AFG3L2, AGK, AGRP, AIFM1, AK2, AKT2, ALDH2, AMT, APOPT1, ATP5A1, ATP5E, ATP5F1A, ATP5F1D, ATP5F1E, ATPAF2, AUH, BCS1L, BOLA3, C10orf2, C12orf65, C19orf12, C1QBP, CAPN10, CARS2, CARTPT, CDH23, CDKAL1, CHCHD10, CHKB, CISD2, CLRN1, COA5, COA7, COQ2, COQ4, COQ6, COQ7, COQ8A, COQ9, COX10, COX14, COX15, COX20, COX6B1, COX8A, CPS1, CPT1A, CPT2, CYC1, DARS2, DFNB31, DGUOK, DLAT, DNA2, DNAJC19, DNM1L, EARS2, ECHS1, ELAC2, ENPP1, ETFA, ETFB, ETFDH, ETHE1, FARS2, FASTKD2, FBXL4, FOXRED1, FXN, GATM, GCGR, GCK, GCSH, GFER, GFM1, GHRL, GJB2, GJB3, GJB6, GLDC, GPD2, GPR98, GTPBP3, HADH, HADHA, HADHB, HARS2, HMGA1, HMGCS2, HNF1A, HNF1B, HNF4A, HSD17B10, HSPD1, IBA57, IDH2, IGF2BP2, IL6, INSR, IRS1, IRS2, ISCA1, ISCA2, ISCU, KANK1, KCNJ11, LARS2, LEPR, LIAS, LIPC, LRPPRC, LYRM4, LYRM7, MAPK8IP1, MARS2, MC4R, MFF, MFN2, MGME1, MIPEP, MPC1, MPV17, MRAP2, MRPL3, MRPL44, MRPS16, MRPS2, MRPS22, MRPS34, MRPS7, MSTO1, MTFMT, MTO1, MTPAP, MYO7A, NARS2, NDUFA1, NDUFA10, NDUFA11, NDUFA12, NDUFA13, NDUFA2, NDUFA6, NDUFA9, NDUFAF1, NDUFAF2, NDUFAF3, NDUFAF4, NDUFAF5, NDUFAF6, NDUFB11, NDUFB3, NDUFB8, NDUFB9, NDUFS1, NDUFS2, NDUFS3, NDUFS4, NDUFS6, NDUFS7, NDUFS8, NDUFV1, NDUFV2, NEUROD1, NF2, NFU1, NR0B2, NUBPL, NUP62, OPA1, OPA3, PAX4, PC, PCDH15, PCK2, PDHA1, PDHB, PDP1, PDSS1, PDSS2, PDX1, PET100, PMPCB, PNPLA8, PNPT1, POLG, POLG2, POMC, PPARG, PPARGC1B, PPP1R3A, PUS1, PYY, RARS2, RMND1, RNASEH1, RRM2B, SCO1, SCO2, SDC3, SDHA, SDHAF1, SDHD, SERAC1, SFXN4, SIM1, SLC22A5, SLC25A1, SLC25A12, SLC25A13, SLC25A15, SLC25A19, SLC25A20, SLC25A22, SLC25A26, SLC25A3, SLC25A4, SLC2A2, SLC2A4, SLC30A8, SPG7, SUCLA2, SUCLG1, SURF1, TACO1, TARS2, TAZ, TDF7L2, TFAM, TIMM8A, TIMMDC1, TK2, TMEM126B, TMEM70, TOP3A, TRIT1, TRMT10C, TRMT5, TRMU, TRNT1, TSFM, TTC19, TUFM, TWNK, TXN2, TYMP, UCP1, UCP3, UQCC2, UQCC3, UQCRB, UQCRC2, UQCRQ, USH1C, USH1G, USH2A, VARS2, WARS2, WFS1 and YARS2; and/or
(iii) a nuclear DNA encoded gene selected from the group consisting of ABCB7, ACADSB, AKAP10, ALAS2, ALDH4A1, ALDH6A1, AMACR, APTX, ARMS2, BAX, BCAT2, BCKDHA, BCKDHB, BCL2, C12orf62, C20orf7, C8orf38, COX4I2, CRAT, CYB5R3, CYCS, CYP11A1, CYP11B1, CYP11B2, CYP24A1, CYP27A1, CYP27B1, D2HGDH, DBT, DECR1, DHODH, DIABLO, DLD, DMGDH, FH, GDAP1, GK, GLRX5, GLUD1, HCCS, HIBCH, HK1, HLCS, HMGCL, HOGA1, HTRA2, IDH3B, IVD, KARS, KIF1B, L2HGDH, LRRK2, MAOA, MCCC1, MCCC2, MCEE, ME2, MLYCD, MMAA, MMAB, MMADHC, MUT, NAGS, NGLY1, OAT, OGDH, OTC, OXCT1, PANK2, PARK2, PARK7, PCCA, PCCB, PDHX, PINK1, PNKD, PPOX, PYCR1, REEP1, RMRP, SACS, SARDH, SARS2, SDHAF2, SDHB, SDHC, SOD2, SPG20, STAR, TMEM126, UCP2, USMG5, WWOX and XPNPEP3.

**5.** Probucol, or the pharmaceutically acceptable salt thereof, for use according to claim 2, wherein:

(i) the disease is Myoclonus Epilepsy with Ragged-Red Fibers (MERRF) and the DNA mutation or variant is in one or more genes selected from the group consisting of MT-ND5, MT-TF, MT-TH, MT-TS1, MT-TS2, MT-TL1 and MT-TK;
(ii) the disease is mitochondrial encephalopathy, lactic acidosis, and stroke-like episodes syndrome (MELAS) and the DNA mutation or variant is in one or more genes selected from the group consisting of MT-ND1, MT-ND4, Mt-ND5, MT-ND6, MT-TC, MT-TF, MT-TH, MT-TQ, MT-TS1, MT-TS2, MT-TL1 and MT-TK;
(iii) the disease is non-syndromic sensorineural hearing loss and the DNA mutation or variant is in one or more genes selected from the group consisting of MT-RNR1 and MT-RNR2;
(iv) the disease is Leigh syndrome and the DNA mutation or variant is in one or more genes selected from the group consisting of MT-ATP6, MT-TK, MT-TV, MT-TW, ATP5A1, ATP5E, BCS1L, COX10, COX15, CYC1,FOXRED1, LRPPRC, LYRM7, NDUFA10, NDUFA12, NDUFA2, NDUFA9, NDUFAF2, NDUFAF6, NDUFS3, NDUFS4, NDUFS7, NDUFS8, POLG, SCO1, SCO2, SDHA, SDHC, SDHC, SDHAF1, SDHAF2, SURF1, TMEM70, ATPAF2, UQCRB, UQCRQ, UQCRC2, UQCC2, UQCC3 and USMG5;
(v) the disease is leukoencephalopathy and the DNA mutation or variant is in one or more genes selected from the group consisting of DARS2, SDHA, SDHB, SDHC, SDHD, SDHAF1 and SDHAF2;
(vi) the disease is Alpers-Huttenlocher syndrome and the DNA mutation or variant is in the POLG gene;
(vii) the disease is mitochondrial neurogastrointestinal encephalopathy (MNGIE) syndrome and the DNA mutation or variant is in one or more genes selected from the group consisting of TK2, DGUOK, SUCLG1, SUCLA2,

ABAT, TYMP, RRM2B, MT-TK, POLG, SLC25A4, AGK and MPV17;
(viii) the disease is liver failure and the DNA mutation or variant is in the TFAM gene;
(ix) the disease is a neurologic disease or liver disease and the DNA mutation or variant is in one or more genes selected from the group consisting of DARS2, RARS2, EARS2, MARS2, FARS2, YARS2, SARS2, AARS2 and HARS2;
(x) the disease is primary mitochondrial disease and the DNA mutation or variant is in one or more genes selected from the group consisting of MRPS and MRPL;
(xi) the disease is a respiratory chain deficiency, neurologic disease, vision loss or liver disease and the DNA mutation or variant is in one or more genes selected from the group consisting of TACO 1 , GFM1 and C12ORF65;
(xii) the disease is peripheral neuropathy or optic neuropathy and the DNA mutation or variant is in one or more genes selected from the group consisting of OPA1, MFN1, MFN2, DNM1L and MIEF2;
(xiii) the disease is a mitochondrial complex deficiency and the DNA mutation or variant is in one or more genes selected from the group consisting of MT-ATP6, MT-CO1, MT-CO2, MT-CO3, MT-ND1, MT-ND2, MT-ND3, MT-ND4, MT-TL1, MT-TN, MT-TK, MT-TV, MT-TW, APOPT1, ATPAF2, ATP5A1, ATP5E, ATP5F1A, ATP5F1D, ATP5F1E, BCS1L, COA5, COA7, COX6B1, COX8A,COX10, COX14, COX15, COX20,CYC1, FASTKD2, FOXRED1, MTFMT, NDUFA1, NDUFA2, NDUFA6, NDUFA9, NDUFA10, NDUFA11, NDUFA12, NDUFA13, NDUFAF1, NDUFAF2, NDUFAF3, NDUFAF4, NDUFAF5, NDUFAF6, NDUFB3, NDUFB8, NDUFB9, NDUFB11, NDUFS1, NDUFS2, NDUFS3, NDUFS4, NDUFS6, NDUFS7, NDUFS8, NDUFV1, NDUFV2, NUBPL, PET100, SCO1, SDHA, SDHAF1, SDHD, SURF1, TACO1, TIMMDC1, TMEM70, TMEM126B, LYRM4, TTC19, UQCC2, UQCC3, UQCRB, UQCRC2 and UQCRQ;
(xiv) the disease is a co-enzyme Q10 deficiency and the DNA mutation or variant is in one or more genes selected from the group consisting of ADCK3, COQ2, COQ4, COQ6, COQ7, COQ8A, COQ9, PDSS1 and PDSS2;
(xv) the disease is chronic progressive external ophthalmoplegia (CPEO) and the DNA mutation or variant is in one or more genes selected from the group consisting of MT-TA, MT-TL2, MT-TM, MT-TW, C10orf2, DGUOK, DNA2, POLG, RNASEH1, RRM2B, SLC25A4, TK2, TOP3A and TWNK;
(xvi) the disease is a carnitine deficiency and the DNA mutation or variant is in one or more genes selected from the group consisting of CPT1A, CPT2, SLC22A5 and SLC22A20;
(xvii) the disease is Friedreich's ataxia and the DNA mutation or variant is in the FXN gene;
(xviii) the disease is Kearns-Sayre syndrome and the DNA mutation or variant is in the MT-TL1 gene;
(xix) the disease is lethal infantile cardiomyopathy (LIC) or Barth syndrome and the DNA mutation or variant is in the TAZ gene; or
(xx) the disease is Leber's hereditary optic neuropathy (LHON) and the mutation or variant is in one or more genes selected from the group consisting of MT-ND1, MT-ND2, MT-ND3, MT-ND4, MT-ND5, MT-ND6, MT-ND4L, MT-CYB, MT-CO1, MT-CO2, MT-CO3, MT-ATP6, and MT-ATP8.

**6.** Probucol, or the pharmaceutically acceptable salt thereof, for use according to any preceding claim, wherein the subject's mitochondrial dysfunction or mitochondrial disease is assessed, monitored, or diagnosed by a method comprising:

(i) employing a panel of two or more sub-instruments to measure one or more clinical symptoms of mitochondrial dysfunction or mitochondrial disease in a subject,
(ii) combining the measurements obtained from said two or more sub-instruments into a single composite measurement, and
(iii) assessing the overall severity of, or change in, the mitochondrial dysfunction or mitochondrial disease in the subject by comparing the composite measurement to a reference value or another composite measurement in the same subject; optionally wherein:

(i) one or more composite measurements are employed to measure the clinical effect on the subject of a diagnostic, therapeutic or other type of medical intervention;
(ii) for each of the sub-instruments in said panel, the subject is classified as:

(a) a sub-instrument responder or sub-instrument non-responder,
(b) a member of a clinical category, or
(c) a member of a metric range, based on the change in said one or more clinical symptoms as measured using said sub-instrument; and

(iii) the measurements obtained from the sub-instruments in the panel are combined into a single composite

measurement, by either:

(a) separately assessing the change in each measurement obtained from the panel sub-instruments prior to combining each measurement into a single composite measurement, or
(b) combining measurements obtained from the panel sub-instruments at a first time point and generating a single composite measurement for said first time point and then comparing the single composite measurement for said first time point to a single composite measurement generated from the same panel sub-instruments for a second time point.

**7.** Probucol, or the pharmaceutically acceptable salt thereof, for use according to claim 6, wherein:

(i) the panel sub-instruments comprise one or more of Motor Function Measure, Six Minute Walk Test, Two Minute Walk Test, Modified Fatigue Impact Scale, Friedreich's Ataxia Rating Scale, Patient-Reported Outcomes Measurement Information System Gastrointestinal Symptom Scales, 30-Second Chair Stand Test, Muscle Strength by Myometry Test, Newcastle Mitochondrial Disease Adult Scale, Newcastle Mitochondrial Disease Pediatric Scale, 36-Item Short Form Survey, Clinical Global Impression, Patient's Global Impression, Patient-Reported Initial MitoPC Symptoms, Patient-Reported MitoPC Symptom Changes, Columbia Suicide Severity Rating Scale, Neuropathy Impairment Score, Migraine Disability Assessment Test, Quick Inventory of Depressive Symptomatology - Self-Report, Montreal Cognitive Assessment, NY Heart Association Functional Classification, Diabetes Health Profile, Ocular Motility Assessments, Marginal Reflex Distance, Ocular Motility & Fixation in 8 Gaze Directions, Degree of Ocular Saccades, Visual Function Test, Logarithm of Minimum Angle of Resolution, Pelli-Robson Score, Humphrey Visual Field mean deviation, or a combination thereof;
(ii) the panel sub-instruments comprise Motor Function Measure, Six Minute Walk Test, Modified Fatigue Impact Scale, Friedreich's Ataxia Rating Scale, and the Patient-Reported Outcomes Measurement Information System Gastrointestinal Symptom Scales;
(iii) the panel sub-instruments comprises five sub-instruments, optionally wherein the five sub-instruments comprise Motor Function Measure, Six Minute Walk Test, Modified Fatigue Impact Scale, Friedreich's Ataxia Rating Scale, and the Patient-Reported Outcomes Measurement Information System Gastrointestinal Symptom Scales; or
(iv) the one or more clinical symptoms are selected from the group consisting of fatigue, muscle weakness, neuromuscular dysfunction, exercise intolerance, imbalance, dysautonomia, gastrointestinal dysfunction, vision loss, eye muscle dysfunction, retinal dysfunction, optic nerve dysfunction, ptosis, headache, dehydration, peripheral neuropathy, numbness, epilepsy, seizures, insomnia, mood disorder, depression, diabetes mellitus, obesity, kidney dysfunction, hyperlipidemia, liver disease, sleep apnea, autism spectrum behavior, delayed developmental milestones, arrhythmia, heart muscle dysfunction, cardiac disease, stroke, speech disorder, tinnitus, hearing impairment, learning disability, cognitive impairment, dementia, or a combination thereof.

**8.** Probucol, or the pharmaceutically acceptable salt thereof, for use according to any one of the preceding claims, wherein the probucol, or a pharmaceutically acceptable salt thereof, is administered at a dose of about 1 mg, about 10 mg, about 100 mg, about 250 mg, about 500 mg, about 1 g, about 2 g, about 5 g, or about 10 g.

**9.** Probucol, or the pharmaceutically acceptable salt thereof, for use according to any one of the preceding claims, wherein the probucol, or a pharmaceutically acceptable salt thereof, is administered at a dose of about 1 mg/day, about 10 mg/day, about 100 mg/day, about 250 mg/day, about 500 mg/day, about 1 g/day, about 2 g/day, about 5 g/day, or about 10 g/day.

**10.** Probucol, or the pharmaceutically acceptable salt thereof, for use according to any preceding claim, wherein the probucol is administered as a liquid formulation, optionally wherein the liquid formulation is administered by a route selected from the group consisting of oral, enteral, gastrostomy tube, jejunostomy tube, orogastric tube, nasogastric tube, parenteral, intravenous, subcutaneous, intramuscular, intraperitoneal, intracisternal, intraarticular, intracerebral, intraparenchymal, intracerebro-ventricular, nasal, vaginal, sublingual, intraocular, intravitreal, rectal, topical, transdermal and inhalation.

**11.** Probucol, or the pharmaceutically acceptable salt thereof, for use according to any one of claims 1 to 9, wherein the probucol, or a pharmaceutically acceptable salt thereof, is administered in the form of a tablet.

**12.** Probucol, or the pharmaceutically acceptable salt thereof, for use according to any one of the preceding claims, wherein the probucol, or a pharmaceutically acceptable salt thereof, is administered on a continuous dosing schedule.

**13.** Probucol, or the pharmaceutically acceptable salt thereof, for use according to any one of the preceding claims, wherein the probucol, or a pharmaceutically acceptable salt thereof, is administered twice per day, once per day, once every two days, once every three days, once every four days, once every five days, once every six days, once a week, once every two weeks or once per month.

**14.** Probucol, or the pharmaceutically acceptable salt thereof, for use according to any one of the preceding claims, wherein the probucol, or a pharmaceutically acceptable salt thereof, is administered initially in a loading dose that is higher than a subsequent dose.

**15.** Probucol, or the pharmaceutically acceptable salt thereof, for use according to any one of the preceding claims, further comprising administering one or more additional pharmaceutical agents, optionally wherein the one or more additional pharmaceutical agents are selected from niacin, cycloheximide, bezafibrate, vatiquinone, carnitine, coenzyme Q10, alpha-tocopherolquinone, coenzyme Q10 analogs, cytochrome C, dichloroacetate, 5-[(e)-2-(4-hydroxyphenyl)-ethenyl] benzene-1,3 diol, flavin mononucleotide, elamipretide, idebenone, latrepirdine, levocarnitine, 2',3',5'-tri-O-acetyluridine, olesoxime, omaveloxolone, thiamin diphosphate, ubiquinone, vitamin C, vitamin D, vitamin E, thiamine, riboflavin, magnesium, calcium, phosphate, membrane phospholipid, unsaturated fatty acid, creatine, pyruvate, $\alpha$-lipoic acid, NADH, PPAR delta modulator, PPAR delta agonist, cysteamine bitartrate, nicotinic acid, nicotinamide, nicotinamide riboside, acipimox, resveratrol, glucose, L-carnitine, glutathione, redox-modulating agent, dichloroacetate, curcumin, schisandrin, triheptanoin, viral or non-viral vector gene therapy targeting mitochondrial disease mutations, or a combination thereof.

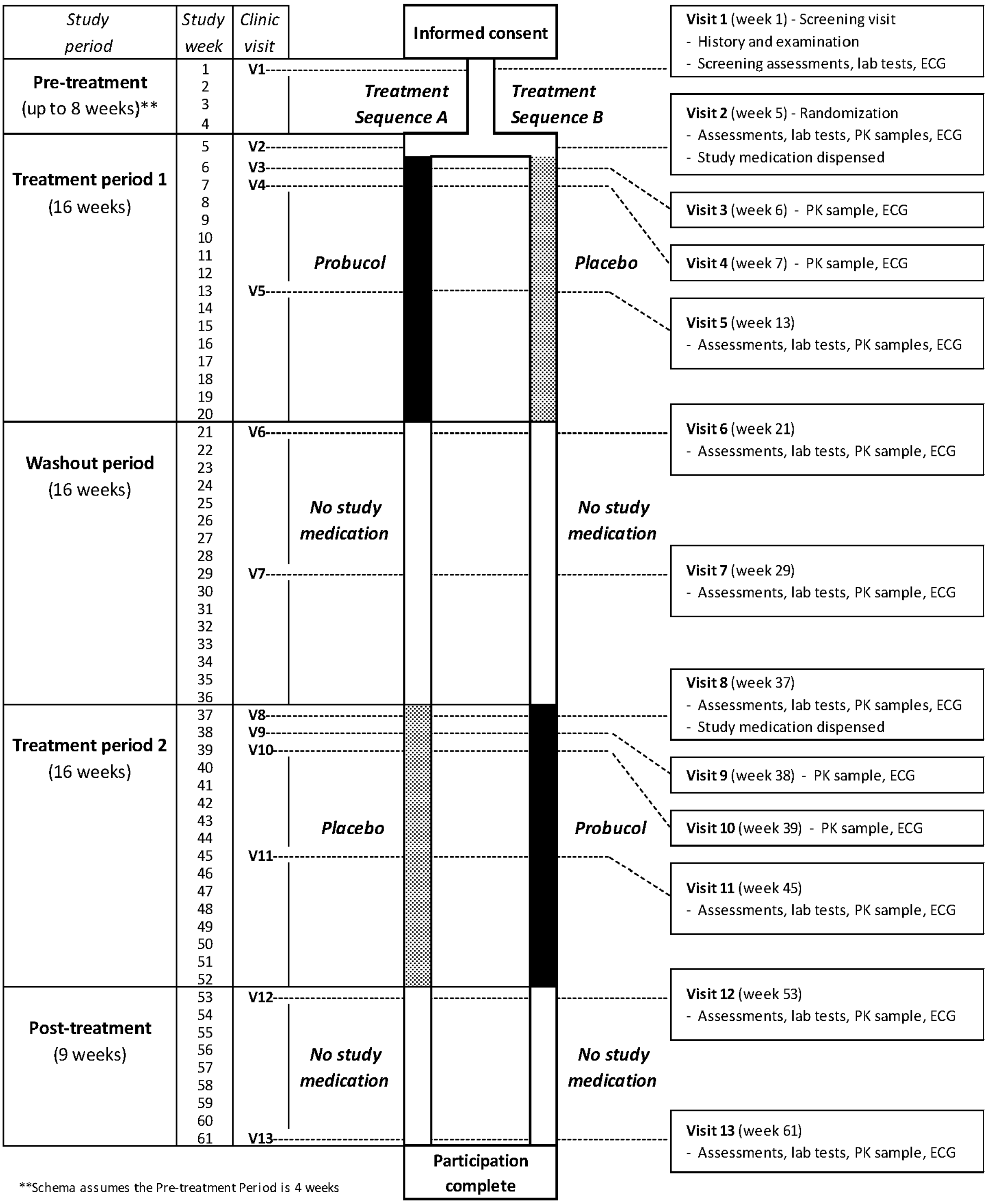
Study period
Study week
Clinic visit
Informed consent
Pre-treatment
(up to 8 weeks)**
Treatment period 1
(16 weeks)
Washout period
(16 weeks)
Treatment period 2
(16 weeks)
Post-treatment
(9 weeks)
V1
V2
V3
V4
V5
V6
V7
V8
V9
V10
V11
V12
V13
Treatment Sequence A
Treatment Sequence B
Probucol
Placebo
No study medication
No study medication
Placebo
Probucol
No study medication
No study medication
Participation complete
Visit 1 (week 1) - Screening visit
- History and examination
- Screening assessments, lab tests, ECG
Visit 2 (week 5) - Randomization
- Assessments, lab tests, PK samples, ECG
- Study medication dispensed
Visit 3 (week 6) - PK sample, ECG
Visit 4 (week 7) - PK sample, ECG
Visit 5 (week 13)
- Assessments, lab tests, PK samples, ECG
Visit 6 (week 21)
- Assessments, lab tests, PK sample, ECG
Visit 7 (week 29)
- Assessments, lab tests, PK sample, ECG
Visit 8 (week 37)
- Assessments, lab tests, PK samples, ECG
- Study medication dispensed
Visit 9 (week 38) - PK sample, ECG
Visit 10 (week 39) - PK sample, ECG
Visit 11 (week 45)
- Assessments, lab tests, PK sample, ECG
Visit 12 (week 53)
- Assessments, lab tests, PK sample, ECG
Visit 13 (week 61)
- Assessments, lab tests, PK sample, ECG
**Schema assumes the Pre-treatment Period is 4 weeks

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9210996 A
- WO 9924400 A
- JP 2000319441 A
- WO 2007114948 A
- WO 2019055988 A
- US 20020198231 A
- US 6916813 B
- US 20030013772 A
- US 20070259908 A

### Non-patent literature cited in the description


- **KREMER et al.** Mitochondrial Disease Genetics in Diagnosis and Management of Mitochondrial Disorders. Springer, 2019, 41-62
- **GORMAN et al.** *Ann. Neurol.,* 2015, vol. 77, 753-9
- **SCAGLIA et al.** *Pediatrics,* 2004, vol. 114, 925-31
- **VERITY et al.** *Dev. Med. Child Neurol.,* 2010, vol. 52, 434-40
- **MCCORMACK et al.** *Mol. Genet. Metab.,* 2017, vol. 121 (2), 119-126
- **PFEFFER et al.** *Nat. Rev. Neurol.,* 2013, vol. 9, 474-81
- **ZHANG et al.** *Mol. Genet. Metab.,* 2010, vol. 99 (3), 309-18
- **FALK et al.** *EMBO Mol. Med.,* 2011, vol. 3 (7), 410-27
- **PENG et al.** *Hum. Mol. Genet.,* 2015, vol. 24 (17), 4829-47
- **BYRNES et al.** *Neurochem. Int.,* 2018, vol. 117, 23-34
- **YAMASHITA et al.** *Curr. Opin. Lipidol.,* 2015, vol. 26 (4), 304-16
- **GORMAN et al.** *Nat. Rev. Dis. Prim.,* 2016, vol. 2, 16080
- **KIM et al.** *Catheterization and Cardiovascular Interventions,* 2002, vol. 57 (4), 424-8
- **DEBRAY et al.** *Pediatrics,* 2007, vol. 119 (4), 722-33
- **SKLADAL et al.** *Clin. Pediatr.,* 2003, vol. 42 (8), 703-10
- **KISLER et al.** *Dev. Med. Child. Neurol.,* 2010, vol. 52 (5), 422-33
- **READ et al.** *Int. J. Lan. Commun. Disord.,* 2012, vol. 47, 106-11
- **BRAEGGER et al.** *J Pediatr. Gastroenterol. Nutr.,* July 2010, vol. 51 (1), 110-22
- **CHOI ; LEE.** *Scientific Reports,* 2017, vol. 7, 16909
- **HAAS et al.** *Pediatrics.,* 2007, vol. 120 (6), 1326-33
- **FRASER et al.** *Surv. Ophthalmol.,* 2010, vol. 55 (4), 299-334
- **GRONLUND et al.** *Br. J. Ophthalmol.,* 2010, vol. 94 (1), 121-127
- **SCHOSER ; PONGRATZ.** *Strabismus,* 2006, vol. 14 (2), 107-13
- **SCHRIER ; FALK.** *Curr. Opin. Ophthalmol.,* 2011, vol. 22 (5), 325-331
- **JARRETT et al.** *Ophthalmic Res.,* 2010, vol. 44 (3), 179-90
- **MOHAMMAD et al.** *Lab. Invest.,* 2010, vol. 90 (9), 1365-1372
- **KENNEY et al.** *Invest. Ophthalmol. Vis. Sci.,* 2010, vol. 51 (8), 4289-4297
- *CHEMICAL ABSTRACTS,* 23288-49-5
- **PARIKH et al.** *Genet. Med.,* 2015, vol. 17, 689-701
- **KARAA et al.** *J. Inherit. Metab. Dis.,* 2017, vol. 40 (3), 403-14
- **POLYAK et al.** *Mol. Genet. Metab.,* 2018, vol. 123 (4), 449-462
- **PENG et al.** *Hum. Mol. Genet.,* 2015, vol. 24 (17), 4829-4847
- **ZHANG et al.** *PLoS One,* 2013, vol. 8 (7), e69282
- **MCCORMACK et al.** *Mitochondrion,* 2015, vol. 22, 45-59
- **FALK et al.** *Mol. Genet. Metab.,* 2015, vol. 114 (3), 388-396
- **SHEN et al.** *Hum. Mutat.,* 2016, vol. 37 (6), 540-548
- **KOOPMAN et al.** *N. Engl. J. Med.,* 2012, vol. 366, 1132-41
- **MCCORMICK et al.** *Neurotherapeutics,* 2013, vol. 10, 251-61

- **SHEN et al.** MSeqDR: a centralized knowledge repository and bioinformatics web resource to facilitate genomic investigations in mitochondrial disease. *Hum. Mutat.,* 2016, vol. 37 (6), 540-548
- **ZOLKIPLI-CUNNINGHAM et al.** *PLoS One,* 2018, vol. 13 (5), e0197513
- **BRENNAN et al.** *Am. J. Gastroenterol.,* 2014, vol. 109, 1804-1814
- **FLACHENECKER et al.** *Multiple Sclerosis,* 2002, vol. 8, 523-526
- **SUBRAMONY et al.** *Neurology,* 2005, vol. 64, 1261-1262
- **BERARD et al.** *Neuromuscular Disorders,* 2005, vol. 15, 463-470
- **ENRIGHT ; SCHERRILL.** *Am. J. Respir. Crit. Core Med.,* 1998, vol. 158, 1384-1387